# FASCICULE DE BREVET EUROPEEN

(11) **EP 2 553 453 B1**
(45) Date de publication et mention de la délivrance du brevet: **08.07.2015**
(21) Numéro de dépôt: 11718448.1
(22) Date de dépôt: 01.04.2011
(51) Int. Cl.: G01N 33/566, G01N 33/542, C07K 14/705, C12N 15/10, C12Q 1/68

(54) **Outils pour l'identification de ligands de Lingo-1, Lingo-2, Lingo-3 et Lingo-4, et leurs utilisations**
Werkzeuge zur Identifizierung von Lingo-1-, Lingo-2-, Lingo-3- und Lingo-4-Liganden und ihre Verwendung
Tools for the identification of Lingo-1-, Lingo-2-, Lingo-3- and Lingo-4- ligands, and uses thereof

(30) Priorité: 01.04.2010 FR 1001374
(43) Date de publication de la demande: 06.02.2013
(73) Titulaire: Centre National de la Recherche Scientifique (CNRS), 75794 Paris Cedex 16 (FR)
(72) Inventeur: MORISSET-LOPEZ, Séverine, F-45750 Saint-Pryve-Saint-Mesmin (FR); BENEDETTI, Hélène, F-45800 Saint-Jean-de-Braye (FR)
(74) Mandataire: Novagraaf Technologies
(86) Numéro de dépôt international: PCT/FR2011/050743
(87) Numéro de publication internationale: WO 2011/121257

(56) Documents cités:
- US-A1- 2007 026 463
- US-A1- 2009 246 189
- ZHANG Z. ET AL.: "LINGO-1 interacts with WNK1 to regulate nogo-induced inhibition of neurite extension.", J. BIOL. CHEM., vol. 284, no. 23, 5 juin 2009 (2009-06-05) , pages 15717-15728, XP002600129,
- anonymous: "GFP-tagged ORF clone of Homo sapiens leucine rich repeat and Ig domain containing 1 (LINGO1) as transfection-ready DNA", , 2010, XP002600130, Extrait de l'Internet: URL:http://www.origene.com/orf_clone/truec lone/NM_032808/RG203867/LINGO1.aspx [extrait le 2010-09-09]
- anonymous: "GFP-tagged ORF clone of Homo sapiens leucine rich repeat and Ig domain containing 2 (LINGO2) as transfection-ready DNA", , 2010, XP002600131, Extrait de l'Internet: URL:http://www.origene.com/orf_clone/truec lone/NM_152570/RG214465/LINGO2.aspx [extrait le 2010-09-09]
- anonymous: "GFP-tagged ORF clone of Homo sapiens leucine rich repeat and Ig domain containing 3 (LINGO3) as transfection-ready DNA", , 2010, XP002600132, Extrait de l'Internet: URL:http://www.origene.com/orf_clone/truec lone/NM_001101391/RG225998/LINGO3.aspx [extrait le 2010-09-09]
- anonymous: "GFP-tagged ORF clone of Homo sapiens leucine rich repeat and Ig domain containing 4 (LINGO4) as transfection-ready DNA", , 2010, XP002600133, Extrait de l'Internet: URL:http://www.origene.com/orf_clone/truec lone/NM_001004432/RG215621/LINGO4.aspx [extrait le 2010-09-09]

### SEQUENCE LISTING

<110> Centre National de la Recherche Scientifique (CNRS)
<120> Outils pour l'identification de ligands de Lingo-1, Lingo-2, Lingo-3 et Lingo-4, et utilisations
<130> BIP205699PC00
<150> FR 10/01374
   <151> 2010-04-01
<160> 19
<170> PatentIn version 3.5
<210> 1
   <211> 620
   <212> PRT
   <213> Homo sapiens
<400> 1 
Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175
 
Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
    370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430
 
Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile
    610                 615                 620
<210> 2
   <211> 606
   <212> PRT
   <213> Homo sapiens
<400> 2 
Met Leu His Thr Ala Ile Ser Cys Trp Gln Pro Phe Leu Gly Leu Ala
1               5                   10                  15

Val Val Leu Ile Phe Met Gly Ser Thr Ile Gly Cys Pro Ala Arg Cys
            20                  25                  30

Glu Cys Ser Ala Gln Asn Lys Ser Val Ser Cys His Arg Arg Arg Leu
 
        35                  40                  45

Ile Ala Ile Pro Glu Gly Ile Pro Ile Glu Thr Lys Ile Leu Asp Leu
    50                  55                  60

Ser Lys Asn Arg Leu Lys Ser Val Asn Pro Glu Glu Phe Ile Ser Tyr
65                  70                  75                  80

Pro Leu Leu Glu Glu Ile Asp Leu Ser Asp Asn Ile Ile Ala Asn Val
                85                  90                  95

Glu Pro Gly Ala Phe Asn Asn Leu Phe Asn Leu Arg Ser Leu Arg Leu
            100                 105                 110

Lys Gly Asn Arg Leu Lys Leu Val Pro Leu Gly Val Phe Thr Gly Leu
        115                 120                 125

Ser Asn Leu Thr Lys Leu Asp Ile Ser Glu Asn Lys Ile Val Ile Leu
    130                 135                 140

Leu Asp Tyr Met Phe Gln Asp Leu His Asn Leu Lys Ser Leu Glu Val
145                 150                 155                 160

Gly Asp Asn Asp Leu Val Tyr Ile Ser His Arg Ala Phe Ser Gly Leu
                165                 170                 175

Leu Ser Leu Glu Gln Leu Thr Leu Glu Lys Cys Asn Leu Thr Ala Val
            180                 185                 190

Pro Thr Glu Ala Leu Ser His Leu Arg Ser Leu Ile Ser Leu His Leu
        195                 200                 205

Lys His Leu Asn Ile Asn Asn Met Pro Val Tyr Ala Phe Lys Arg Leu
    210                 215                 220

Phe His Leu Lys His Leu Glu Ile Asp Tyr Trp Pro Leu Leu Asp Met
225                 230                 235                 240

Met Pro Ala Asn Ser Leu Tyr Gly Leu Asn Leu Thr Ser Leu Ser Val
                245                 250                 255

Thr Asn Thr Asn Leu Ser Thr Val Pro Phe Leu Ala Phe Lys His Leu
            260                 265                 270

Val Tyr Leu Thr His Leu Asn Leu Ser Tyr Asn Pro Ile Ser Thr Ile
        275                 280                 285

Glu Ala Gly Met Phe Ser Asp Leu Ile Arg Leu Gln Glu Leu His Ile
    290                 295                 300
 
Val Gly Ala Gln Leu Arg Thr Ile Glu Pro His Ser Phe Gln Gly Leu
305                 310                 315                 320

Arg Phe Leu Arg Val Leu Asn Val Ser Gln Asn Leu Leu Glu Thr Leu
                325                 330                 335

Glu Glu Asn Val Phe Ser Ser Pro Arg Ala Leu Glu Val Leu Ser Ile
            340                 345                 350

Asn Asn Asn Pro Leu Ala Cys Asp Cys Arg Leu Leu Trp Ile Leu Gln
        355                 360                 365

Arg Gln Pro Thr Leu Gln Phe Gly Gly Gln Gln Pro Met Cys Ala Gly
    370                 375                 380

Pro Asp Thr Ile Arg Glu Arg Ser Phe Lys Asp Phe His Ser Thr Ala
385                 390                 395                 400

Leu Ser Phe Tyr Phe Thr Cys Lys Lys Pro Lys Ile Arg Glu Lys Lys
                405                 410                 415

Leu Gln His Leu Leu Val Asp Glu Gly Gln Thr Val Gln Leu Glu Cys
            420                 425                 430

Ser Ala Asp Gly Asp Pro Gln Pro Val Ile Ser Trp Val Thr Pro Arg
        435                 440                 445

Arg Arg Phe Ile Thr Thr Lys Ser Asn Gly Arg Ala Thr Val Leu Gly
    450                 455                 460

Asp Gly Thr Leu Glu Ile Arg Phe Ala Gln Asp Gln Asp Ser Gly Met
465                 470                 475                 480

Tyr Val Cys Ile Ala Ser Asn Ala Ala Gly Asn Asp Thr Phe Thr Ala
                485                 490                 495

Ser Leu Thr Val Lys Gly Phe Ala Ser Asp Arg Phe Leu Tyr Ala Asn
            500                 505                 510

Arg Thr Pro Met Tyr Met Thr Asp Ser Asn Asp Thr Ile Ser Asn Gly
        515                 520                 525

Thr Asn Ala Asn Thr Phe Ser Leu Asp Leu Lys Thr Ile Leu Val Ser
    530                 535                 540

Thr Ala Met Gly Cys Phe Thr Phe Leu Gly Val Val Leu Phe Cys Phe
545                 550                 555                 560
 
Leu Leu Leu Phe Val Trp Ser Arg Gly Lys Gly Lys His Lys Asn Ser
                565                 570                 575

Ile Asp Leu Glu Tyr Val Pro Arg Lys Asn Asn Gly Ala Val Val Glu
            580                 585                 590

Gly Glu Val Ala Gly Pro Arg Arg Phe Asn Met Lys Met Ile
        595                 600                 605
<210> 3
   <211> 592
   <212> PRT
   <213> Homo sapiens
<400> 3 
   Met Thr Cys Trp Leu Cys Val Leu Ser Leu Pro Leu Leu Leu Leu Pro
   1               5                   10                  15
   Ala Ala Pro Pro Pro Ala Gly Gly Cys Pro Ala Arg Cys Glu Cys Thr
               20                  25                  30
   Val Gln Thr Arg Ala Val Ala Cys Thr Arg Arg Arg Leu Thr Ala Val
           35                  40                  45
   Pro Asp Gly Ile Pro Ala Glu Thr Arg Leu Leu Glu Leu Ser Arg Asn
       50                  55                  60
   Arg Ile Arg Cys Leu Asn Pro Gly Asp Leu Ala Ala Leu Pro Ala Leu
   65                  70                  75                  80
   Glu Glu Leu Asp Leu Ser Glu Asn Ala Ile Ala His Val Glu Pro Gly
                   85                  90                  95
   Ala Phe Ala Asn Leu Pro Arg Leu Arg Val Leu Arg Leu Arg Gly Asn
               100                 105                 110
   Gln Leu Lys Leu Ile Pro Pro Gly Val Phe Thr Arg Leu Asp Asn Leu
           115                 120                 125
   Thr Leu Leu Asp Leu Ser Glu Asn Lys Leu Val Ile Leu Leu Asp Tyr
       130                 135                 140
   Thr Phe Gln Asp Leu His Ser Leu Arg Arg Leu Glu Val Gly Asp Asn
   145                 150                 155                 160
   Asp Leu Val Phe Val Ser Arg Arg Ala Phe Ala Gly Leu Leu Ala Leu
                   165                 170                 175
   Glu Glu Leu Thr Leu Glu Arg Cys Asn Leu Thr Ala Leu Ser Gly Glu
   180 185 190
   Ser Leu Gly His Leu Arg Ser Leu Gly Ala Leu Arg Leu Arg His Leu
   195 200 205
   Ala Ile Ala Ser Leu Glu Asp Gln Asn Phe Arg Arg Leu Pro Gly Leu
   210 215 220
   Leu His Leu Glu Ile Asp Asn Trp Pro Leu Leu Glu Glu Val Ala Ala
   225 230 235 240
   Gly Ser Leu Arg Gly Leu Asn Leu Thr Ser Leu Ser Val Thr His Thr
   245 250 255
   Asn Ile Thr Ala Val Pro Ala Ala Ala Leu Arg His Gln Ala His Leu
   260 265 270
   Thr Cys Leu Asn Leu Ser His Asn Pro Ile Ser Thr Val Pro Arg Gly
   275 280 285
   Ser Phe Arg Asp Leu Val Arg Leu Arg Glu Leu His Leu Ala Gly Ala
   290 295 300
   Leu Leu Ala Val Val Glu Pro Gln Ala Phe Leu Gly Leu Arg Gln Ile
   305 310 315 320
   Arg Leu Leu Asn Leu Ser Asn Asn Leu Leu Ser Thr Leu Glu Glu Ser
   325 330 335
   Thr Phe His Ser Val Asn Thr Leu Glu Thr Leu Arg Val Asp Gly Asn
   340 345 350
   Pro Leu Ala Cys Asp Cys Arg Leu Leu Trp Ile Val Gln Arg Arg Lys
   355 360 365
   Thr Leu Asn Phe Asp Gly Arg Leu Pro Ala Cys Ala Thr Pro Ala Glu
   370 375 380
   Val Arg Gly Asp Ala Leu Arg Asn Leu Pro Asp Ser Val Leu Phe Glu
   385 390 395 400
   Tyr Phe Val Cys Arg Lys Pro Lys Ile Arg Glu Arg Arg Leu Gln Arg
   405 410 415
   Val Thr Ala Thr Ala Gly Glu Asp Val Arg Phe Leu Cys Arg Ala Glu
   420 425 430
   Gly Glu Pro Ala Pro Thr Val Ala Trp Val Thr Pro Gln His Arg Pro
   435 440 445
   Val Thr Ala Thr Ser Ala Gly Arg Ala Arg Val Leu Pro Gly Gly Thr
       450                 455                 460
   Leu Glu Ile Gln Asp Ala Arg Pro Gln Asp Ser Gly Thr Tyr Thr Cys
   465                 470                 475                 480
   Val Ala Ser Asn Ala Gly Gly Asn Asp Thr Tyr Phe Ala Thr Leu Thr
                   485                 490                 495
   Val Arg Pro Glu Pro Ala Ala Asn Arg Thr Pro Gly Glu Ala His Asn
               500                 505                 510
   Glu Thr Leu Ala Ala Leu Arg Ala Pro Leu Asp Leu Thr Thr Ile Leu
           515                 520                 525
   Val Ser Thr Ala Met Gly Cys Ile Thr Phe Leu Gly Val Val Leu Phe
       530                 535                 540
   Cys Phe Val Leu Leu Phe Val Trp Ser Arg Gly Arg Gly Gln His Lys
   545                 550                 555                 560
   Asn Asn Phe Ser Val Glu Tyr Ser Phe Arg Lys Val Asp Gly Pro Ala
                   565                 570                 575
   Ala Ala Ala Gly Gln Gly Gly Ala Arg Lys Phe Asn Met Lys Met Ile
               580                 585                 590
<210> 4
   <211> 593
   <212> PRT
   <213> Homo sapiens
<400> 4 
Met Asp Ala Ala Thr Ala Pro Lys Gln Ala Trp Pro Pro Trp Pro Pro
1               5                   10                  15

Leu Leu Phe Leu Leu Leu Leu Pro Gly Gly Ser Gly Gly Ser Cys Pro
            20                  25                  30

Ala Val Cys Asp Cys Thr Ser Gln Pro Gln Ala Val Leu Cys Gly His
        35                  40                  45

Arg Gln Leu Glu Ala Val Pro Gly Gly Leu Pro Leu Asp Thr Glu Leu
    50                  55                  60

Leu Asp Leu Ser Gly Asn Arg Leu Trp Gly Leu Gln Gln Gly Met Leu
65                  70                  75                  80
 
Ser Arg Leu Ser Leu Leu Gln Glu Leu Asp Leu Ser Tyr Asn Gln Leu
                85                  90                  95

Ser Thr Leu Glu Pro Gly Ala Phe His Gly Leu Gln Ser Leu Leu Thr
            100                 105                 110

Leu Arg Leu Gln Gly Asn Arg Leu Arg Ile Met Gly Pro Gly Val Phe
        115                 120                 125

Ser Gly Leu Ser Ala Leu Thr Leu Leu Asp Leu Arg Leu Asn Gln Ile
    130                 135                 140

Val Leu Phe Leu Asp Gly Ala Phe Gly Glu Leu Gly Ser Leu Gln Lys
145                 150                 155                 160

Leu Glu Val Gly Asp Asn His Leu Val Phe Val Ala Pro Gly Ala Phe
                165                 170                 175

Ala Gly Leu Ala Lys Leu Ser Thr Leu Thr Leu Glu Arg Cys Asn Leu
            180                 185                 190

Ser Thr Val Pro Gly Leu Ala Leu Ala Arg Leu Pro Ala Leu Val Ala
        195                 200                 205

Leu Arg Leu Arg Glu Leu Asp Ile Gly Arg Leu Pro Ala Gly Ala Leu
    210                 215                 220

Arg Gly Leu Gly Gln Leu Lys Glu Leu Glu Ile His Leu Trp Pro Ser
225                 230                 235                 240

Leu Glu Ala Leu Asp Pro Gly Ser Leu Val Gly Leu Asn Leu Ser Ser
                245                 250                 255

Leu Ala Ile Thr Arg Cys Asn Leu Ser Ser Val Pro Phe Gln Ala Leu
            260                 265                 270

Tyr His Leu Ser Phe Leu Arg Val Leu Asp Leu Ser Gln Asn Pro Ile
        275                 280                 285

Ser Ala Ile Pro Ala Arg Arg Leu Ser Pro Leu Val Arg Leu Gln Glu
    290                 295                 300

Leu Arg Leu Ser Gly Ala Cys Leu Thr Ser Ile Ala Ala His Ala Phe
305                 310                 315                 320

His Gly Leu Thr Ala Phe His Leu Leu Asp Val Ala Asp Asn Ala Leu
                325                 330                 335

Gln Thr Leu Glu Glu Thr Ala Phe Pro Ser Pro Asp Lys Leu Val Thr
 
            340                 345                 350

Leu Arg Leu Ser Gly Asn Pro Leu Thr Cys Asp Cys Arg Leu Leu Trp
        355                 360                 365

Leu Leu Arg Leu Arg Arg His Leu Asp Phe Gly Met Ser Pro Pro Ala
    370                 375                 380

Cys Ala Gly Pro His His Val Gln Gly Lys Ser Leu Lys Glu Phe Ser
385                 390                 395                 400

Asp Ile Leu Pro Pro Gly His Phe Thr Cys Lys Pro Ala Leu Ile Arg
                405                 410                 415

Lys Ser Gly Pro Arg Trp Val Ile Ala Glu Glu Gly Gly His Ala Val
            420                 425                 430

Phe Ser Cys Ser Gly Asp Gly Asp Pro Ala Pro Thr Val Ser Trp Met
        435                 440                 445

Arg Pro His Gly Ala Trp Leu Gly Arg Ala Gly Arg Val Arg Val Leu
    450                 455                 460

Glu Asp Gly Thr Leu Glu Ile Arg Ser Val Gln Leu Arg Asp Arg Gly
465                 470                 475                 480

Ala Tyr Val Cys Val Val Ser Asn Val Ala Gly Asn Asp Ser Leu Arg
                485                 490                 495

Thr Trp Leu Glu Val Ile Gln Val Glu Pro Pro Asn Gly Thr Leu Ser
            500                 505                 510

Asp Pro Asn Ile Thr Val Pro Gly Ile Pro Gly Pro Phe Phe Leu Asp
        515                 520                 525

Ser Arg Gly Val Ala Met Val Leu Ala Val Gly Phe Leu Pro Phe Leu
    530                 535                 540

Thr Ser Val Thr Leu Cys Phe Gly Leu Ile Ala Leu Trp Ser Lys Gly
545                 550                 555                 560

Lys Gly Arg Val Lys His His Met Thr Phe Asp Phe Val Ala Pro Arg
                565                 570                 575

Pro Ser Gly Asp Lys Asn Ser Gly Gly Asn Arg Val Thr Ala Lys Leu
            580                 585                 590

Phe
<210> 5
   <211> 2932
   <212> DNA
   <213> Homo sapiens
<400> 5 
ggagagacat gcgattggtg accgagccga gcggaccgaa ggcgcgcccg agatgcaggt 60

gagcaagagg atgctggcgg ggggcgtgag gagcatgccc agccccctcc tggcctgctg 120

gcagcccatc ctcctgctgg tgctgggctc agtgctgtca ggctcggcca cgggctgccc 180

gccccgctgc gagtgctccg cccaggaccg cgctgtgctg tgccaccgca agcgctttgt 240

ggcagtcccc gagggcatcc ccaccgagac gcgcctgctg gacctaggca agaaccgcat 300

caaaacgctc aaccaggacg agttcgccag cttcccgcac ctggaggagc tggagctcaa 360

cgagaacatc gtgagcgccg tggagcccgg cgccttcaac aacctcttca acctccggac 420

gctgggtctc cgcagcaacc gcctgaagct catcccgcta ggcgtcttca ctggcctcag 480

caacctgacc aagctggaca tcagcgagaa caagatcgtt atcctactgg actacatgtt 540

tcaggacctg tacaacctca agtcactgga ggttggcgac aatgacctcg tctacatctc 600

tcaccgcgcc ttcagcggcc tcaacagcct ggagcagctg acgctggaga aatgcaacct 660

gacctccatc cccaccgagg cgctgtccca cctgcacggc ctcatcgtcc tgaggctccg 720

gcacctcaac atcaatgcca tccgggacta ctccttcaag aggctgtacc gactcaaggt 780

cttggagatc tcccactggc cctacttgga caccatgaca cccaactgcc tctacggcct 840

caacctgacg tccctgtcca tcacacactg caatctgacc gctgtgccct acctggccgt 900

ccgccaccta gtctatctcc gcttcctcaa cctctcctac aaccccatca gcaccattga 960

gggctccatg ttgcatgagc tgctccggct gcaggagatc cagctggtgg gcgggcagct 1020

ggccgtggtg gagccctatg ccttccgcgg cctcaactac ctgcgcgtgc tcaatgtctc 1080

tggcaaccag ctgaccacac tggaggaatc agtcttccac tcggtgggca acctggagac 1140

actcatcctg gactccaacc cgctggcctg cgactgtcgg ctcctgtggg tgttccggcg 1200

ccgctggcgg ctcaacttca accggcagca gcccacgtgc gccacgcccg agtttgtcca 1260

gggcaaggag ttcaaggact tccctgatgt gctactgccc aactacttca cctgccgccg 1320

cgcccgcatc cgggaccgca aggcccagca ggtgtttgtg gacgagggcc acacggtgca 1380

gtttgtgtgc cgggccgatg gcgacccgcc gcccgccatc ctctggctct caccccgaaa 1440

gcacctggtc tcagccaaga gcaatgggcg gctcacagtc ttccctgatg gcacgctgga 1500

ggtgcgctac gcccaggtac aggacaacgg cacgtacctg tgcatcgcgg ccaacgcggg 1560

cggcaacgac tccatgcccg cccacctgca tgtgcgcagc tactcgcccg actggcccca 1620

tcagcccaac aagaccttcg ctttcatctc caaccagccg ggcgagggag aggccaacag 1680

cacccgcgcc actgtgcctt tccccttcga catcaagacc ctcatcatcg ccaccaccat 1740
 
gggcttcatc tctttcctgg gcgtcgtcct cttctgcctg gtgctgctgt ttctctggag 1800

ccggggcaag ggcaacacaa agcacaacat cgagatcgag tatgtgcccc gaaagtcgga 1860

cgcaggcatc agctccgccg acgcgccccg caagttcaac atgaagatga tatgaggccg 1920

gggcgggggg caggaccccc cgggcggccg ggcaggggaa ggggcctggc cgccacctgc 1980

tcactctcca gtccttccca cctcctccct acccttctac acacgttctc tttctccctc 2040

ccgcctccgt cccctgctgc cccccgccag ccctcaccac ctgccctcct tctaccagga 2100

cctcagaagc ccagacctgg ggaccccacc tacacagggg cattgacaga ctggagttga 2160

aagccgacga accgacacgc ggcagagtca ataattcaat aaaaaagtta cgaactttct 2220

ctgtaacttg ggtttcaata attatggatt tttatgaaaa cttgaaataa taaaaagaga 2280

aaaaaactat ttcctatagc tagtcggaat gcaaactttt gacgtcctga ttgctccagg 2340

gccctcttcc aactcagttt cttgtttttc tcttcctcct cctcctcttc ttcctccttt 2400

ctcttctctt ccccagtggg gagggatcac tcaggaaaac aggaaaggag gttccagccc 2460

cacccacctg cccaccccgc cccaggcacc atcaggagca ggctaggggg caggcctggg 2520

cccagctccg ggctggcttt ttgcagggcg caggtggagg ggacaggtct gccgatgggg 2580

gtgggagcct gtctgctggg ctgccaggcg gcaccactgc aaggggtggg agcctggctt 2640

gggtgtggct gagactctgg acagaggctg gggtcctcct gggggacagc acagtcagtg 2700

gagagagcca ggggctggag gtggggccca ccccagcctc tggtcccagc tctgctgctc 2760

acttgctgtg tggcctcaag caggtcactg gcctctctgg gcctcagtct ccacatctgt 2820

acaaatggga acattacccc ctgccctgcc tacctcacag ggctgttgtg aggaattgat 2880

gagatgatgt atgtgaaaca ctttgtaacc tgtaaagcgc tgtgcacacg tg 2932
<210> 6
   <211> 2792
   <212> DNA
   <213> Homo sapiens
<400> 6 
aatttagaga agatgtaggg agtgttcaac atgttcgttg tggaagagaa agagctaaga 60

gagaggagct taaagacaca aacgggtaga atcaaggagt gtgctctcaa atgagaggaa 120

caggagtgac attaaccttg aaatgctcgg agactctact ccttcatgac agtaggagga 180

taattaacaa tagatacaaa tgcaggaatt gatgagtgcc atcagaaagc tgtatcatga 240

gctgcctgca cttctaaagt gtccagtgga tttttaatca catgagcctg gaaatagggt 300

tatgaaaaga agctcagagc agagcaccga aagtggccac taccagcatg aagagcccaa 360

caattcaaac tggtgaagtg agaaaaacag aatgcagctt tcaaggttcg tttcaagcag 420

ttggcttgtg ggactctgag agatgctgct gcccatgaca tgcgggaatt atcatgatca 480

actacccagc ttggatttca tccagtggcc aagagctttg tgtgggagac ggcaagggtt 540
 
ggatttttca aaagagtaaa ccaggataaa tcatgaggaa cctataaccc ttttggccac 600
atgcaaaaaa gcaagacccg tgaccaaggt gtagactaag aagtggagtc atgcttcaca 660
cggccatatc atgctggcag ccattcctgg gtctggctgt ggtgttaatc ttcatgggat 720
ccaccattgg ctgccccgct cgctgtgagt gctctgccca gaacaaatct gttagctgtc 780
acagaaggcg attgatcgcc atcccagagg gcattcccat cgaaaccaaa atcttggacc 840
tcagtaaaaa caggctaaaa agcgtcaacc ctgaagaatt catatcatat cctctgctgg 900
aagagataga cttgagtgac aacatcattg ccaatgtgga accaggagca ttcaacaatc 960
tctttaacct gcgttccctc cgcctaaaag gcaatcgtct aaagctggtc cctttgggag 1020
tattcacggg gctgtccaat ctcactaagc ttgacattag tgagaataag attgtcattt 1080
tactagacta catgttccaa gatctacata acctgaagtc tctagaagtg ggggacaatg 1140
atttggttta tatatcacac agggcattca gtgggcttct tagcttggag cagctcaccc 1200
tggagaaatg caacttaaca gcagtaccaa cagaagccct ctcccacctc cgcagcctca 1260
tcagcctgca tctgaagcat ctcaatatca acaatatgcc tgtgtatgcc tttaaaagat 1320
tgttccacct gaaacaccta gagattgact attggccttt actggatatg atgcctgcca 1380
atagcctcta cggtctcaac ctcacatccc tttcagtcac caacaccaat ctgtctactg 1440
tacccttcct tgcctttaaa cacctggtat acctgactca ccttaacctc tcctacaatc 1500
ccatcagcac tattgaagca ggcatgttct ctgacctgat ccgccttcag gagcttcata 1560
tagtgggggc ccagcttcgc accattgagc ctcactcctt ccaagggctc cgcttcctac 1620
gcgtgctcaa tgtgtctcag aacctgctgg aaactttgga agagaatgtc ttctcctccc 1680
ctagggctct ggaggtcttg agcattaaca acaaccctct ggcctgtgac tgccgccttc 1740
tctggatctt gcagcgacag cccaccctgc agtttggtgg ccagcaacct atgtgtgctg 1800
gcccagacac catccgtgag aggtctttca aggatttcca tagcactgcc ctttcttttt 1860
actttacctg caaaaaaccc aaaatccgtg aaaagaagtt gcagcatctg ctagtagatg 1920
aagggcagac agtccagcta gaatgcagtg cagatggaga cccgcagcct gtgatttcct 1980
gggtgacacc ccgaaggcgt ttcatcacca ccaagtccaa tggaagagcc accgtgttgg 2040
gtgatggcac cttggaaatc cgctttgccc aggatcaaga cagcgggatg tatgtttgca 2100
tcgctagcaa tgctgctggg aatgatacct tcacagcctc cttaactgtg aaaggattcg 2160
cttcagatcg ttttctttat gcgaacagga cccctatgta catgaccgac tccaatgaca 2220
ccatttccaa tggcaccaat gccaatactt tttccctgga ccttaaaaca atactggtgt 2280
ctacagctat gggctgcttc acattcctgg gagtggtttt attttgtttt cttctccttt 2340
ttgtgtggag ccgagggaaa ggcaagcaca aaaacagcat tgaccttgag tatgtgccca 2400
gaaaaaacaa tggtgctgtt gtggaaggag aggtagctgg acccaggagg ttcaacatga 2460
 
aaatgatttg aaggcccacc cctcacatta ctgtctcttt gtcaatgtgg gtaatcagta 2520

agacagtatg gcacagtaaa ttactagatt aagaggcagc catgtgcagc tgcccctgta 2580

tcaaaagcag ggtctatgga agcaggagga cttccaatgg agactctcca tcgaaaggca 2640

ggcaggcagg catgtgtcag agcccttcac acagtgggat actaagtgtt tgcgttgcaa 2700

atattggcgt tctggggatc tcagtaatga acctgaatat ttggctcaca ctcacggaca 2760

attattcagc attttctacc actgcaaaaa ac 2792
<210> 7
   <211> 2130
   <212> DNA
   <213> Homo sapiens
<400> 7 
ggcggcggcg gcggctgctc gtgtcggctc cgggcggccc ccgcgccgca gcccgcgccg 60

tgtccgcggc ggagccgccc aggtgcgcag gaggatggtg gcgcggccct aggcccacgc 120

tccgcaccat gacctgctgg ctgtgcgtcc tgagcctgcc cctgctcctg ctgcccgcgg 180

cgccgccccc ggctggaggc tgcccggccc gctgcgagtg caccgtgcag acccgcgcgg 240

tggcctgcac gcgccgccgc ctgaccgccg tgcccgacgg catcccggcc gagacccgcc 300

tgctggagct cagccgcaac cgcatccgct gcctgaaccc gggcgacctg gccgcgctgc 360

ccgcgctgga ggagctggac ctgagcgaga acgccatcgc gcacgtggag cccggcgcct 420

tcgccaacct gccgcgcctg cgcgtcctgc gtctccgtgg caaccagctg aagctcatcc 480

cgcccggggt cttcacgcgc ctggacaacc tcacgctgct ggacctgagc gagaacaagc 540

tggtaatcct gctggactac actttccagg acctgcacag cctgcgccgg ctggaagtgg 600

gcgacaacga cctggtattc gtctcgcgcc gcgccttcgc ggggctgctg gccctggagg 660

agctgaccct ggagcgctgc aacctcacgg ctctgtccgg ggagtcgctg ggccatctgc 720

gcagcctggg cgccctgcgg ctgcgccacc tggccatcgc ctccctggag gaccagaact 780

tccgcaggct gcccgggctg ctgcacctgg agattgacaa ctggccgctg ctggaggagg 840

tggcggcggg cagcctgcgg ggcctgaacc tgacctcgct gtcggtcacc cacaccaaca 900

tcaccgccgt gccggccgcc gcgctgcggc accaggcgca cctcacctgc ctcaatctgt 960

cgcacaaccc catcagcacg gtgccgcggg ggtcgttccg ggacctggtc cgcctgcgcg 1020

agctgcacct ggccggggcc ctgctggctg tggtggagcc gcaggccttc ctgggcctgc 1080

gccagatccg cctgctcaac ctctccaaca acctgctctc cacgttggag gagagcacct 1140

tccactcggt gaacacgcta gagacgctgc gcgtggacgg gaacccgctg gcctgcgact 1200

gtcgcctgct gtggatcgtg cagcgtcgca agaccctcaa cttcgacggg cggctgccgg 1260

cctgcgccac cccggccgag gtgcgcggcg acgcgctgcg aaacctgccg gactccgtgc 1320

tgttcgagta cttcgtgtgc cgcaaaccca agatccggga gcggcggctg cagcgcgtca 1380
 
cggccaccgc gggcgaagac gtccgcttcc tctgccgcgc cgagggcgag ccggcgccca 1440

ccgtggcctg ggtgaccccc cagcaccggc cggtgacggc caccagcgcg ggccgggcgc 1500

gcgtgctccc cggggggacg ctggagatcc aggacgcgcg gccgcaggac agcggcacct 1560

acacgtgcgt ggccagcaac gcgggcggca acgacaccta cttcgccacg ctgaccgtgc 1620

gccccgagcc ggccgccaac cggaccccgg gcgaggccca caacgagacg ctggcggccc 1680

tgcgcgcgcc gctcgacctc accaccatcc tggtgtccac cgccatgggc tgcatcacct 1740

tcctgggcgt ggtcctcttc tgcttcgtgc tgctgttcgt gtggagccgc ggccgcgggc 1800

agcacaaaaa caacttctcg gtggagtact ccttccgcaa ggtggatggg ccggccgccg 1860

cggcgggcca gggaggcgcg cgcaagttca acatgaagat gatctgaggg gtccccaggg 1920

cggaccctcc cctcccctcc cccgcgggcc ggccgctcgc gtgtccacct atgcatttcc 1980

cggaggggaa ggggacggct gcacggcgct ccccaggcag aacttcccct ttttttgtag 2040

acgcccaacc gcaggactgt ttttcatcag catgcgtctt ttttgcagtt tctcaagcgt 2100

tttctaaaga tttcaacccg tcttcctgtc 2130
<210> 8
   <211> 2606
   <212> DNA
   <213> Homo sapiens
<400> 8 
gcggccgcag cagcaacagc agcagcagca gcggcaggca gcagccgggc agccaggcag 60

cgggggttga ggcacacagg gaaggtgcag gggcctgagg tgcagctcga atgggacagg 120

gcccccagcg ctggacagat gcagtgccaa acttgatgcc accttccagc ttctccggac 180

tgaagaggga atggatgcag ccacagctcc aaagcaagcc tggcccccat ggcccccgct 240

ccttttcctc ctcctcctac ctggagggag cggtggcagc tgccctgctg tgtgtgactg 300

cacctcccag ccccaggctg tgctctgtgg ccacaggcaa ctggaggctg tacctggagg 360

actcccactg gacactgagc tcctggacct gagtgggaac cgcctgtggg ggctccagca 420

gggaatgctc tcccgcctga gcctgctcca ggaattggac ctcagctaca accagctctc 480

aacccttgag cctggggcct tccatggcct acaaagccta ctcaccctga ggctgcaggg 540

caatcggctc agaatcatgg ggcctggggt cttctcaggc ctctctgctc tgaccctgct 600

ggacctccgc ctcaaccaga ttgttctctt cctagatgga gcttttgggg agctaggcag 660

cctccagaag ctggaggttg gggacaacca cctggtattt gtggctccgg gggcctttgc 720

agggctagcc aagttgagca ccctcaccct ggagcgctgc aacctcagca cagtgcctgg 780

cctagccctt gcccgtctcc cggcactagt ggccctaagg cttagagaac tggatattgg 840

gaggctgcca gctgggcccc tgcgggggct ggggcagctc aaggagctgg agatccacct 900

ctggccatct ctggaggctc tggaccctgg gagcctggtt gggctcaatc tcagcagcct 960
 
ggccatcact cgctgcaatc tgagctcggt gcccttccaa gcactgtacc acctcagctt 1020

cctcagggtc ctggatctgt cccagaatcc catctcagcc atcccagccc gaaggctcag 1080

ccccctggtg cggctccagg agctacgcct gtcaggggca tgcctcacct ccattgctgc 1140

ccatgccttc catggcttga ctgccttcca cctcctggat gtggcagata acgcccttca 1200

gacactagag gaaacagctt tcccttctcc agacaaactg gtcaccttga ggctgtctgg 1260

caacccccta acctgtgact gccgcctcct ctggctgctc cggctccgcc gccacctgga 1320

ctttggcatg tccccccctg cctgtgctgg cccccatcat gtccagggga agagcctgaa 1380

ggagttttca gacatcctgc ctccagggca cttcacctgc aaaccagccc tgatccgaaa 1440

gtcggggcct cgatgggtca ttgcagagga gggcgggcat gcggttttct cctgctctgg 1500

agatggagac ccagccccca ctgtctcctg gatgaggcct catggggctt ggctgggcag 1560

ggctgggaga gtaagggtcc tagaggatgg gacactggag atccgctcag tgcagctacg 1620

ggacagaggg gcctatgtct gtgtggttag caatgtcgct gggaatgact ccctgaggac 1680

ctggctggaa gtcatccagg tggaaccacc aaacggcaca ctttctgacc ccaacatcac 1740

cgtgccaggg atcccagggc ctttttttct ggatagcaga ggtgtggcca tggtgctggc 1800

agtcggcttc ctccccttcc tcacctcagt gaccctctgc tttggcctga ttgccctttg 1860

gagcaagggc aaaggtcggg tcaaacatca catgaccttt gactttgtgg cacctcggcc 1920

ctctggggat aaaaactctg gggtaaccgg ggtcactgcc aagctcttct gacctttcct 1980

tccccagtgg ggaacccacc aagtccgctt cagataccaa aggggaagac agaaccaagg 2040

ctgcttgaac cagaacctag tcccgagcag caccgctctc ctgcacctcc cgcctgcgtt 2100

gtgcctcctg ccggagagtc tgcttcctga gcttttccgg tctgaggata gcattgtcat 2160

ttcttctctg agggtcccag ggagctgcag atgcagaccc cgttgttagt ccagcccccg 2220

cttcaccccc tccacacaca aaacaggaaa cataatcaaa gcgctagtca gctagtctaa 2280

ccactaggct ttcttcacac atgcttatat cctttaataa ccaattgcca accacggcta 2340

taagattatt tcagaggtgg ggctgggaag tgccacttgc tccttagagt ctgtttgtca 2400

accaggcaga gtccctttct tttctgctcc ccaccccaac cctgccccta tgtacaggaa 2460

taagagcaaa ggacccacag gctacagaga agaggatggg gacagagtgt gggatggaga 2520

ggacagacca tatactgcac tgtgtttgca tgagcctcta ccaccttcct ctatctacca 2580

gatcattaaa cctgctgtca aagggc 2606
<210> 9
   <211> 720
   <212> DNA
   <213> synthetic
<400> 9 atggtgagca agggcgagga gctgttcacc ggggtggtgc ccatcctggt cgagctggac 60 
ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcggcta cggcctgcag tgcttcgccc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagct accagtccgc cctgagcaaa gaccccaacg agaagcgcga tcacatggtc 660

ctgctggagt tcgtgaccgc cgccgggatc actctcggca tggacgagct gtacaagtaa 720
<210> 10
   <211> 2604
   <212> DNA
   <213> synthetic
<400> 10 
atgcaggtga gcaagaggat gctggcgggg ggcgtgagga gcatgcccag ccccctcctg 60

gcctgctggc agcccatcct cctgctggtg ctgggctcag tgctgtcagg ctcggccacg 120

ggctgcccgc cccgctgcga gtgctccgcc caggaccgcg ctgtgctgtg ccaccgcaag 180

cgctttgtgg cagtccccga gggcatcccc accgagacgc gcctgctgga cctaggcaag 240

aaccgcatca aaacgctcaa ccaggacgag ttcgccagct tcccgcacct ggaggagctg 300

gagctcaacg agaacatcgt gagcgccgtg gagcccggcg ccttcaacaa cctcttcaac 360

ctccggacgc tgggtctccg cagcaaccgc ctgaagctca tcccgctagg cgtcttcact 420

ggcctcagca acctgaccaa gctggacatc agcgagaaca agatcgttat cctactggac 480

tacatgtttc aggacctgta caacctcaag tcactggagg ttggcgacaa tgacctcgtc 540

tacatctctc accgcgcctt cagcggcctc aacagcctgg agcagctgac gctggagaaa 600

tgcaacctga cctccatccc caccgaggcg ctgtcccacc tgcacggcct catcgtcctg 660

aggctccggc acctcaacat caatgccatc cgggactact ccttcaagag gctgtaccga 720

ctcaaggtct tggagatctc ccactggccc tacttggaca ccatgacacc caactgcctc 780

tacggcctca acctgacgtc cctgtccatc acacactgca atctgaccgc tgtgccctac 840

ctggccgtcc gccacctagt ctatctccgc ttcctcaacc tctcctacaa ccccatcagc 900

accattgagg gctccatgtt gcatgagctg ctccggctgc aggagatcca gctggtgggc 960

gggcagctgg ccgtggtgga gccctatgcc ttccgcggcc tcaactacct gcgcgtgctc 1020

aatgtctctg gcaaccagct gaccacactg gaggaatcag tcttccactc ggtgggcaac 1080
 
ctggagacac tcatcctgga ctccaacccg ctggcctgcg actgtcggct cctgtgggtg 1140

ttccggcgcc gctggcggct caacttcaac cggcagcagc ccacgtgcgc cacgcccgag 1200

tttgtccagg gcaaggagtt caaggacttc cctgatgtgc tactgcccaa ctacttcacc 1260

tgccgccgcg cccgcatccg ggaccgcaag gcccagcagg tgtttgtgga cgagggccac 1320

acggtgcagt ttgtgtgccg ggccgatggc gacccgccgc ccgccatcct ctggctctca 1380

ccccgaaagc acctggtctc agccaagagc aatgggcggc tcacagtctt ccctgatggc 1440

acgctggagg tgcgctacgc ccaggtacag gacaacggca cgtacctgtg catcgcggcc 1500

aacgcgggcg gcaacgactc catgcccgcc cacctgcatg tgcgcagcta ctcgcccgac 1560

tggccccatc agcccaacaa gaccttcgct ttcatctcca accagccggg cgagggagag 1620

gccaacagca cccgcgccac tgtgcctttc cccttcgaca tcaagaccct catcatcgcc 1680

accaccatgg gcttcatctc tttcctgggc gtcgtcctct tctgcctggt gctgctgttt 1740

ctctggagcc ggggcaaggg caacacaaag cacaacatcg agatcgagta tgtgccccga 1800

aagtcggacg caggcatcag ctccgccgac gcgccccgca agttcaacat gaagatgata 1860

tcacgggatc caccggtcgc caccatggtg agcaagggcg aggagctgtt caccggggtg 1920

gtgcccatcc tggtcgagct ggacggcgac gtaaacggcc acaagttcag cgtgtccggc 1980

gagggcgagg gcgatgccac ctacggcaag ctgaccctga agttcatctg caccaccggc 2040

aagctgcccg tgccctggcc caccctcgtg accaccttcg gctacggcct gcagtgcttc 2100

gcccgctacc ccgaccacat gaagcagcac gacttcttca agtccgccat gcccgaaggc 2160

tacgtccagg agcgcaccat cttcttcaag gacgacggca actacaagac ccgcgccgag 2220

gtgaagttcg agggcgacac cctggtgaac cgcatcgagc tgaagggcat cgacttcaag 2280

gaggacggca acatcctggg gcacaagctg gagtacaact acaacagcca caacgtctat 2340

atcatggccg acaagcagaa gaacggcatc aaggtgaact tcaagatccg ccacaacatc 2400

gaggacggca gcgtgcagct cgccgaccac taccagcaga acacccccat cggcgacggc 2460

cccgtgctgc tgcccgacaa ccactacctg agctaccagt ccgccctgag caaagacccc 2520

aacgagaagc gcgatcacat ggtcctgctg gagttcgtga ccgccgccgg gatcactctc 2580

ggcatggacg agctgtacaa gtaa 2604
<210> 11
   <211> 867
   <212> PRT
   <213> synthetic
<400> 11 
Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
 
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285
 
Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
    370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540
 
Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile Ser Arg Asp Pro
    610                 615                 620

Pro Val Ala Thr Met Val Ser Lys Gly Glu Glu Leu Phe Thr Gly Val
625                 630                 635                 640

Val Pro Ile Leu Val Glu Leu Asp Gly Asp Val Asn Gly His Lys Phe
                645                 650                 655

Ser Val Ser Gly Glu Gly Glu Gly Asp Ala Thr Tyr Gly Lys Leu Thr
            660                 665                 670

Leu Lys Phe Ile Cys Thr Thr Gly Lys Leu Pro Val Pro Trp Pro Thr
        675                 680                 685

Leu Val Thr Thr Phe Gly Tyr Gly Leu Gln Cys Phe Ala Arg Tyr Pro
    690                 695                 700

Asp His Met Lys Gln His Asp Phe Phe Lys Ser Ala Met Pro Glu Gly
705                 710                 715                 720

Tyr Val Gln Glu Arg Thr Ile Phe Phe Lys Asp Asp Gly Asn Tyr Lys
                725                 730                 735

Thr Arg Ala Glu Val Lys Phe Glu Gly Asp Thr Leu Val Asn Arg Ile
            740                 745                 750

Glu Leu Lys Gly Ile Asp Phe Lys Glu Asp Gly Asn Ile Leu Gly His
        755                 760                 765

Lys Leu Glu Tyr Asn Tyr Asn Ser His Asn Val Tyr Ile Met Ala Asp
    770                 775                 780

Lys Gln Lys Asn Gly Ile Lys Val Asn Phe Lys Ile Arg His Asn Ile
785                 790                 795                 800
 
Glu Asp Gly Ser Val Gln Leu Ala Asp His Tyr Gln Gln Asn Thr Pro
                805                 810                 815

Ile Gly Asp Gly Pro Val Leu Leu Pro Asp Asn His Tyr Leu Ser Tyr
            820                 825                 830

Gln Ser Ala Leu Ser Lys Asp Pro Asn Glu Lys Arg Asp His Met Val
        835                 840                 845

Leu Leu Glu Phe Val Thr Ala Ala Gly Ile Thr Leu Gly Met Asp Glu
    850                 855                 860

Leu Tyr Lys
865
<210> 12
   <211> 936
   <212> DNA
   <213> synthetic
<400> 12 
atgaccagca aggtgtacga ccccgagcag aggaagagga tgatcaccgg cccccagtgg 60

tgggccaggt gcaagcagat gaacgtgctg gacagcttca tcaactacta cgacagcgag 120

aagcacgccg agaacgccgt gatcttcctg cacggcaacg ccgctagtag ctacctgtgg 180

aggcacgtgg tgccccacat cgagcccgtg gccaggtgca tcatccccga tctgatcggc 240

atgggcaaga gcggcaagag cggcaacggc agctacaggc tgctggacca ctacaagtac 300

ctgaccgcct ggttcgagct cctgaacctg cccaagaaga tcatcttcgt gggccacgac 360

tggggcgcct gcctggcctt ccactacagc tacgagcacc aggacaagat caaggccatc 420

gtgcacgccg agagcgtggt ggacgtgatc gagagctggg acgagtggcc agacatcgag 480

gaggacatcg ccctgatcaa gagcgaggag ggcgagaaga tggtgctgga gaacaacttc 540

ttcgtggaga ccatgctgcc cagcaagatc atgagaaagc tggagcccga ggagttcgcc 600

gcctacctgg agcccttcaa ggagaagggc gaggtgagaa gacccaccct gagctggccc 660

agagagatcc ccctggtgaa gggcggcaag cccgacgtgg tgcagatcgt gagaaactac 720

aacgcctacc tgagagccag cgacgacctg cccaagatgt tcatcgagag cgaccccggc 780

ttcttcagca acgccatcgt ggagggcgcc aagaagttcc ccaacaccga gttcgtgaag 840

gtgaagggcc tgcacttcag ccaggaggac gcccccgacg agatgggcaa gtacatcaag 900

agcttcgtgg agagagtgct gaagaacgag cagtaa 936
<210> 13
   <211> 2820
   <212> DNA
   <213> synthetic
<400> 13 
atgcaggtga gcaagaggat gctggcgggg ggcgtgagga gcatgcccag ccccctcctg 60

gcctgctggc agcccatcct cctgctggtg ctgggctcag tgctgtcagg ctcggccacg 120

ggctgcccgc cccgctgcga gtgctccgcc caggaccgcg ctgtgctgtg ccaccgcaag 180

cgctttgtgg cagtccccga gggcatcccc accgagacgc gcctgctgga cctaggcaag 240

aaccgcatca aaacgctcaa ccaggacgag ttcgccagct tcccgcacct ggaggagctg 300

gagctcaacg agaacatcgt gagcgccgtg gagcccggcg ccttcaacaa cctcttcaac 360

ctccggacgc tgggtctccg cagcaaccgc ctgaagctca tcccgctagg cgtcttcact 420

ggcctcagca acctgaccaa gctggacatc agcgagaaca agatcgttat cctactggac 480

tacatgtttc aggacctgta caacctcaag tcactggagg ttggcgacaa tgacctcgtc 540

tacatctctc accgcgcctt cagcggcctc aacagcctgg agcagctgac gctggagaaa 600

tgcaacctga cctccatccc caccgaggcg ctgtcccacc tgcacggcct catcgtcctg 660

aggctccggc acctcaacat caatgccatc cgggactact ccttcaagag gctgtaccga 720

ctcaaggtct tggagatctc ccactggccc tacttggaca ccatgacacc caactgcctc 780

tacggcctca acctgacgtc cctgtccatc acacactgca atctgaccgc tgtgccctac 840

ctggccgtcc gccacctagt ctatctccgc ttcctcaacc tctcctacaa ccccatcagc 900

accattgagg gctccatgtt gcatgagctg ctccggctgc aggagatcca gctggtgggc 960

gggcagctgg ccgtggtgga gccctatgcc ttccgcggcc tcaactacct gcgcgtgctc 1020

aatgtctctg gcaaccagct gaccacactg gaggaatcag tcttccactc ggtgggcaac 1080

ctggagacac tcatcctgga ctccaacccg ctggcctgcg actgtcggct cctgtgggtg 1140

ttccggcgcc gctggcggct caacttcaac cggcagcagc ccacgtgcgc cacgcccgag 1200

tttgtccagg gcaaggagtt caaggacttc cctgatgtgc tactgcccaa ctacttcacc 1260

tgccgccgcg cccgcatccg ggaccgcaag gcccagcagg tgtttgtgga cgagggccac 1320

acggtgcagt ttgtgtgccg ggccgatggc gacccgccgc ccgccatcct ctggctctca 1380

ccccgaaagc acctggtctc agccaagagc aatgggcggc tcacagtctt ccctgatggc 1440

acgctggagg tgcgctacgc ccaggtacag gacaacggca cgtacctgtg catcgcggcc 1500

aacgcgggcg gcaacgactc catgcccgcc cacctgcatg tgcgcagcta ctcgcccgac 1560

tggccccatc agcccaacaa gaccttcgct ttcatctcca accagccggg cgagggagag 1620

gccaacagca cccgcgccac tgtgcctttc cccttcgaca tcaagaccct catcatcgcc 1680

accaccatgg gcttcatctc tttcctgggc gtcgtcctct tctgcctggt gctgctgttt 1740

ctctggagcc ggggcaaggg caacacaaag cacaacatcg agatcgagta tgtgccccga 1800

aagtcggacg caggcatcag ctccgccgac gcgccccgca agttcaacat gaagatgata 1860

tcacgggatc caccggtagc aaccatgacc agcaaggtgt acgaccccga gcagaggaag 1920

aggatgatca ccggccccca gtggtgggcc aggtgcaagc agatgaacgt gctggacagc 1980
 
ttcatcaact actacgacag cgagaagcac gccgagaacg ccgtgatctt cctgcacggc 2040
aacgccgcta gtagctacct gtggaggcac gtggtgcccc acatcgagcc cgtggccagg 2100
tgcatcatcc ccgatctgat cggcatgggc aagagcggca agagcggcaa cggcagctac 2160
aggctgctgg accactacaa gtacctgacc gcctggttcg agctcctgaa cctgcccaag 2220
aagatcatct tcgtgggcca cgactggggc gcctgcctgg ccttccacta cagctacgag 2280
caccaggaca agatcaaggc catcgtgcac gccgagagcg tggtggacgt gatcgagagc 2340
tgggacgagt ggccagacat cgaggaggac atcgccctga tcaagagcga ggagggcgag 2400
aagatggtgc tggagaacaa cttcttcgtg gagaccatgc tgcccagcaa gatcatgaga 2460
aagctggagc ccgaggagtt cgccgcctac ctggagccct tcaaggagaa gggcgaggtg 2520
agaagaccca ccctgagctg gcccagagag atccccctgg tgaagggcgg caagcccgac 2580
gtggtgcaga tcgtgagaaa ctacaacgcc tacctgagag ccagcgacga cctgcccaag 2640
atgttcatcg agagcgaccc cggcttcttc agcaacgcca tcgtggaggg cgccaagaag 2700
ttccccaaca ccgagttcgt gaaggtgaag ggcctgcact tcagccagga ggacgccccc 2760
gacgagatgg gcaagtacat caagagcttc gtggagagag tgctgaagaa cgagcagtaa 2820
<210> 14
   <211> 939
   <212> PRT
   <213> synthetic
<400> 14 
Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110
 
Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
 
370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile Ser Arg Asp Pro
    610                 615                 620

Pro Val Ala Thr Met Thr Ser Lys Val Tyr Asp Pro Glu Gln Arg Lys
625                 630                 635                 640
 
Arg Met Ile Thr Gly Pro Gln Trp Trp Ala Arg Cys Lys Gln Met Asn
                645                 650                 655

Val Leu Asp Ser Phe Ile Asn Tyr Tyr Asp Ser Glu Lys His Ala Glu
            660                 665                 670

Asn Ala Val Ile Phe Leu His Gly Asn Ala Ala Ser Ser Tyr Leu Trp
        675                 680                 685

Arg His Val Val Pro His Ile Glu Pro Val Ala Arg Cys Ile Ile Pro
    690                 695                 700

Asp Leu Ile Gly Met Gly Lys Ser Gly Lys Ser Gly Asn Gly Ser Tyr
705                 710                 715                 720

Arg Leu Leu Asp His Tyr Lys Tyr Leu Thr Ala Trp Phe Glu Leu Leu
                725                 730                 735

Asn Leu Pro Lys Lys Ile Ile Phe Val Gly His Asp Trp Gly Ala Cys
            740                 745                 750

Leu Ala Phe His Tyr Ser Tyr Glu His Gln Asp Lys Ile Lys Ala Ile
        755                 760                 765

Val His Ala Glu Ser Val Val Asp Val Ile Glu Ser Trp Asp Glu Trp
    770                 775                 780

Pro Asp Ile Glu Glu Asp Ile Ala Leu Ile Lys Ser Glu Glu Gly Glu
785                 790                 795                 800

Lys Met Val Leu Glu Asn Asn Phe Phe Val Glu Thr Met Leu Pro Ser
                805                 810                 815

Lys Ile Met Arg Lys Leu Glu Pro Glu Glu Phe Ala Ala Tyr Leu Glu
            820                 825                 830

Pro Phe Lys Glu Lys Gly Glu Val Arg Arg Pro Thr Leu Ser Trp Pro
        835                 840                 845

Arg Glu Ile Pro Leu Val Lys Gly Gly Lys Pro Asp Val Val Gln Ile
    850                 855                 860

Val Arg Asn Tyr Asn Ala Tyr Leu Arg Ala Ser Asp Asp Leu Pro Lys
865                 870                 875                 880

Met Phe Ile Glu Ser Asp Pro Gly Phe Phe Ser Asn Ala Ile Val Glu
                885                 890                 895
 
Gly Ala Lys Lys Phe Pro Asn Thr Glu Phe Val Lys Val Lys Gly Leu
            900                 905                 910

His Phe Ser Gln Glu Asp Ala Pro Asp Glu Met Gly Lys Tyr Ile Lys
        915                 920                 925

Ser Phe Val Glu Arg Val Leu Lys Asn Glu Gln
    930                 935
<210> 15
   <211> 4960
   <212> DNA
   <213> synthetic
<400> 15 
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660

gatccaccgg tagcaaccat gaccagcaag gtgtacgacc ccgagcagag gaagaggatg 720

atcaccggcc cccagtggtg ggccaggtgc aagcagatga acgtgctgga cagcttcatc 780

aactactacg acagcgagaa gcacgccgag aacgccgtga tcttcctgca cggcaacgcc 840

gctagtagct acctgtggag gcacgtggtg ccccacatcg agcccgtggc caggtgcatc 900

atccccgatc tgatcggcat gggcaagagc ggcaagagcg gcaacggcag ctacaggctg 960

ctggaccact acaagtacct gaccgcctgg ttcgagctcc tgaacctgcc caagaagatc 1020

atcttcgtgg gccacgactg gggcgcctgc ctggccttcc actacagcta cgagcaccag 1080

gacaagatca aggccatcgt gcacgccgag agcgtggtgg acgtgatcga gagctgggac 1140

gagtggccag acatcgagga ggacatcgcc ctgatcaaga gcgaggaggg cgagaagatg 1200

gtgctggaga acaacttctt cgtggagacc atgctgccca gcaagatcat gagaaagctg 1260

gagcccgagg agttcgccgc ctacctggag cccttcaagg agaagggcga ggtgagaaga 1320

cccaccctga gctggcccag agagatcccc ctggtgaagg gcggcaagcc cgacgtggtg 1380
 
cagatcgtga gaaactacaa cgcctacctg agagccagcg acgacctgcc caagatgttc 1440
atcgagagcg accccggctt cttcagcaac gccatcgtgg agggcgccaa gaagttcccc 1500
aacaccgagt tcgtgaaggt gaagggcctg cacttcagcc aggaggacgc ccccgacgag 1560
atgggcaagt acatcaagag cttcgtggag agagtgctga agaacgagca gtaatgtaca 1620
agtaaagcgg ccgcgactct agatcataat cagccatacc acatttgtag aggttttact 1680
tgctttaaaa aacctcccac acctccccct gaacctgaaa cataaaatga atgcaattgt 1740
tgttgttaac ttgtttattg cagcttataa tggttacaaa taaagcaata gcatcacaaa 1800
tttcacaaat aaagcatttt tttcactgca ttctagttgt ggtttgtcca aactcatcaa 1860
tgtatcttaa ggcgtaaatt gtaagcgtta atattttgtt aaaattcgcg ttaaattttt 1920
gttaaatcag ctcatttttt aaccaatagg ccgaaatcgg caaaatccct tataaatcaa 1980
aagaatagac cgagataggg ttgagtgttg ttccagtttg gaacaagagt ccactattaa 2040
agaacgtgga ctccaacgtc aaagggcgaa aaaccgtcta tcagggcgat ggcccactac 2100
gtgaaccatc accctaatca agttttttgg ggtcgaggtg ccgtaaagca ctaaatcgga 2160
accctaaagg gagcccccga tttagagctt gacggggaaa gccggcgaac gtggcgagaa 2220
aggaagggaa gaaagcgaaa ggagcgggcg ctagggcgct ggcaagtgta gcggtcacgc 2280
tgcgcgtaac caccacaccc gccgcgctta atgcgccgct acagggcgcg tcaggtggca 2340
cttttcgggg aaatgtgcgc ggaaccccta tttgtttatt tttctaaata cattcaaata 2400
tgtatccgct catgagacaa taaccctgat aaatgcttca ataatattga aaaaggaaga 2460
gtcctgaggc ggaaagaacc agctgtggaa tgtgtgtcag ttagggtgtg gaaagtcccc 2520
aggctcccca gcaggcagaa gtatgcaaag catgcatctc aattagtcag caaccaggtg 2580
tggaaagtcc ccaggctccc cagcaggcag aagtatgcaa agcatgcatc tcaattagtc 2640
agcaaccata gtcccgcccc taactccgcc catcccgccc ctaactccgc ccagttccgc 2700
ccattctccg ccccatggct gactaatttt ttttatttat gcagaggccg aggccgcctc 2760
ggcctctgag ctattccaga agtagtgagg aggctttttt ggaggcctag gcttttgcaa 2820
agatcgatca agagacagga tgaggatcgt ttcgcatgat tgaacaagat ggattgcacg 2880
caggttctcc ggccgcttgg gtggagaggc tattcggcta tgactgggca caacagacaa 2940
tcggctgctc tgatgccgcc gtgttccggc tgtcagcgca ggggcgcccg gttctttttg 3000
tcaagaccga cctgtccggt gccctgaatg aactgcaaga cgaggcagcg cggctatcgt 3060
ggctggccac gacgggcgtt ccttgcgcag ctgtgctcga cgttgtcact gaagcgggaa 3120
gggactggct gctattgggc gaagtgccgg ggcaggatct cctgtcatct caccttgctc 3180
ctgccgagaa agtatccatc atggctgatg caatgcggcg gctgcatacg cttgatccgg 3240
ctacctgccc attcgaccac caagcgaaac atcgcatcga gcgagcacgt actcggatgg 3300
aagccggtct tgtcgatcag gatgatctgg acgaagagca tcaggggctc gcgccagccg 3360
 
aactgttcgc caggctcaag gcgagcatgc ccgacggcga ggatctcgtc gtgacccatg 3420

gcgatgcctg cttgccgaat atcatggtgg aaaatggccg cttttctgga ttcatcgact 3480

gtggccggct gggtgtggcg gaccgctatc aggacatagc gttggctacc cgtgatattg 3540

ctgaagagct tggcggcgaa tgggctgacc gcttcctcgt gctttacggt atcgccgctc 3600

ccgattcgca gcgcatcgcc ttctatcgcc ttcttgacga gttcttctga gcgggactct 3660

ggggttcgaa atgaccgacc aagcgacgcc caacctgcca tcacgagatt tcgattccac 3720

cgccgccttc tatgaaaggt tgggcttcgg aatcgttttc cgggacgccg gctggatgat 3780

cctccagcgc ggggatctca tgctggagtt cttcgcccac cctaggggga ggctaactga 3840

aacacggaag gagacaatac cggaaggaac ccgcgctatg acggcaataa aaagacagaa 3900

taaaacgcac ggtgttgggt cgtttgttca taaacgcggg gttcggtccc agggctggca 3960

ctctgtcgat accccaccga gaccccattg gggccaatac gcccgcgttt cttccttttc 4020

cccaccccac cccccaagtt cgggtgaagg cccagggctc gcagccaacg tcggggcggc 4080

aggccctgcc atagcctcag gttactcata tatactttag attgatttaa aacttcattt 4140

ttaatttaaa aggatctagg tgaagatcct ttttgataat ctcatgacca aaatccctta 4200

acgtgagttt tcgttccact gagcgtcaga ccccgtagaa aagatcaaag gatcttcttg 4260

agatcctttt tttctgcgcg taatctgctg cttgcaaaca aaaaaaccac cgctaccagc 4320

ggtggtttgt ttgccggatc aagagctacc aactcttttt ccgaaggtaa ctggcttcag 4380

cagagcgcag ataccaaata ctgtccttct agtgtagccg tagttaggcc accacttcaa 4440

gaactctgta gcaccgccta catacctcgc tctgctaatc ctgttaccag tggctgctgc 4500

cagtggcgat aagtcgtgtc ttaccgggtt ggactcaaga cgatagttac cggataaggc 4560

gcagcggtcg ggctgaacgg ggggttcgtg cacacagccc agcttggagc gaacgaccta 4620

caccgaactg agatacctac agcgtgagct atgagaaagc gccacgcttc ccgaagggag 4680

aaaggcggac aggtatccgg taagcggcag ggtcggaaca ggagagcgca cgagggagct 4740

tccaggggga aacgcctggt atctttatag tcctgtcggg tttcgccacc tctgacttga 4800

gcgtcgattt ttgtgatgct cgtcaggggg gcggagccta tggaaaaacg ccagcaacgc 4860

ggccttttta cggttcctgg ccttttgctg gccttttgct cacatgttct ttcctgcgtt 4920

atcccctgat tctgtggata accgtattac cgccatgcat 4960
<210> 16
   <211> 4733
   <212> DNA
   <213> synthetic
<400> 16 
tagttattaa tagtaatcaa ttacggggtc attagttcat agcccatata tggagttccg 60

cgttacataa cttacggtaa atggcccgcc tggctgaccg cccaacgacc cccgcccatt 120
 
gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660

gatccaccgg tcgccaccat ggtgagcaag ggcgaggagc tgttcaccgg ggtggtgccc 720

atcctggtcg agctggacgg cgacgtaaac ggccacaagt tcagcgtgtc cggcgagggc 780

gagggcgatg ccacctacgg caagctgacc ctgaagttca tctgcaccac cggcaagctg 840

cccgtgccct ggcccaccct cgtgaccacc ttcggctacg gcctgcagtg cttcgcccgc 900

taccccgacc acatgaagca gcacgacttc ttcaagtccg ccatgcccga aggctacgtc 960

caggagcgca ccatcttctt caaggacgac ggcaactaca agacccgcgc cgaggtgaag 1020

ttcgagggcg acaccctggt gaaccgcatc gagctgaagg gcatcgactt caaggaggac 1080

ggcaacatcc tggggcacaa gctggagtac aactacaaca gccacaacgt ctatatcatg 1140

gccgacaagc agaagaacgg catcaaggtg aacttcaaga tccgccacaa catcgaggac 1200

ggcagcgtgc agctcgccga ccactaccag cagaacaccc ccatcggcga cggccccgtg 1260

ctgctgcccg acaaccacta cctgagctac cagtccgccc tgagcaaaga ccccaacgag 1320

aagcgcgatc acatggtcct gctggagttc gtgaccgccg ccgggatcac tctcggcatg 1380

gacgagctgt acaagtaaag cggccgcgac tctagatcat aatcagccat accacatttg 1440

tagaggtttt acttgcttta aaaaacctcc cacacctccc cctgaacctg aaacataaaa 1500

tgaatgcaat tgttgttgtt aacttgttta ttgcagctta taatggttac aaataaagca 1560

atagcatcac aaatttcaca aataaagcat ttttttcact gcattctagt tgtggtttgt 1620

ccaaactcat caatgtatct taaggcgtaa attgtaagcg ttaatatttt gttaaaattc 1680

gcgttaaatt tttgttaaat cagctcattt tttaaccaat aggccgaaat cggcaaaatc 1740

ccttataaat caaaagaata gaccgagata gggttgagtg ttgttccagt ttggaacaag 1800

agtccactat taaagaacgt ggactccaac gtcaaagggc gaaaaaccgt ctatcagggc 1860

gatggcccac tacgtgaacc atcaccctaa tcaagttttt tggggtcgag gtgccgtaaa 1920

gcactaaatc ggaaccctaa agggagcccc cgatttagag cttgacgggg aaagccggcg 1980

aacgtggcga gaaaggaagg gaagaaagcg aaaggagcgg gcgctagggc gctggcaagt 2040
 
gtagcggtca cgctgcgcgt aaccaccaca cccgccgcgc ttaatgcgcc gctacagggc 2100

gcgtcaggtg gcacttttcg gggaaatgtg cgcggaaccc ctatttgttt atttttctaa 2160

atacattcaa atatgtatcc gctcatgaga caataaccct gataaatgct tcaataatat 2220

tgaaaaagga agagtcctga ggcggaaaga accagctgtg gaatgtgtgt cagttagggt 2280

gtggaaagtc cccaggctcc ccagcaggca gaagtatgca aagcatgcat ctcaattagt 2340

cagcaaccag gtgtggaaag tccccaggct ccccagcagg cagaagtatg caaagcatgc 2400

atctcaatta gtcagcaacc atagtcccgc ccctaactcc gcccatcccg cccctaactc 2460

cgcccagttc cgcccattct ccgccccatg gctgactaat tttttttatt tatgcagagg 2520

ccgaggccgc ctcggcctct gagctattcc agaagtagtg aggaggcttt tttggaggcc 2580

taggcttttg caaagatcga tcaagagaca ggatgaggat cgtttcgcat gattgaacaa 2640

gatggattgc acgcaggttc tccggccgct tgggtggaga ggctattcgg ctatgactgg 2700

gcacaacaga caatcggctg ctctgatgcc gccgtgttcc ggctgtcagc gcaggggcgc 2760

ccggttcttt ttgtcaagac cgacctgtcc ggtgccctga atgaactgca agacgaggca 2820

gcgcggctat cgtggctggc cacgacgggc gttccttgcg cagctgtgct cgacgttgtc 2880

actgaagcgg gaagggactg gctgctattg ggcgaagtgc cggggcagga tctcctgtca 2940

tctcaccttg ctcctgccga gaaagtatcc atcatggctg atgcaatgcg gcggctgcat 3000

acgcttgatc cggctacctg cccattcgac caccaagcga aacatcgcat cgagcgagca 3060

cgtactcgga tggaagccgg tcttgtcgat caggatgatc tggacgaaga gcatcagggg 3120

ctcgcgccag ccgaactgtt cgccaggctc aaggcgagca tgcccgacgg cgaggatctc 3180

gtcgtgaccc atggcgatgc ctgcttgccg aatatcatgg tggaaaatgg ccgcttttct 3240

ggattcatcg actgtggccg gctgggtgtg gcggaccgct atcaggacat agcgttggct 3300

acccgtgata ttgctgaaga gcttggcggc gaatgggctg accgcttcct cgtgctttac 3360

ggtatcgccg ctcccgattc gcagcgcatc gccttctatc gccttcttga cgagttcttc 3420

tgagcgggac tctggggttc gaaatgaccg accaagcgac gcccaacctg ccatcacgag 3480

atttcgattc caccgccgcc ttctatgaaa ggttgggctt cggaatcgtt ttccgggacg 3540

ccggctggat gatcctccag cgcggggatc tcatgctgga gttcttcgcc caccctaggg 3600

ggaggctaac tgaaacacgg aaggagacaa taccggaagg aacccgcgct atgacggcaa 3660

taaaaagaca gaataaaacg cacggtgttg ggtcgtttgt tcataaacgc ggggttcggt 3720

cccagggctg gcactctgtc gataccccac cgagacccca ttggggccaa tacgcccgcg 3780

tttcttcctt ttccccaccc caccccccaa gttcgggtga aggcccaggg ctcgcagcca 3840

acgtcggggc ggcaggccct gccatagcct caggttactc atatatactt tagattgatt 3900

taaaacttca tttttaattt aaaaggatct aggtgaagat cctttttgat aatctcatga 3960

ccaaaatccc ttaacgtgag ttttcgttcc actgagcgtc agaccccgta gaaaagatca 4020
 
aaggatcttc ttgagatcct ttttttctgc gcgtaatctg ctgcttgcaa acaaaaaaac 4080

caccgctacc agcggtggtt tgtttgccgg atcaagagct accaactctt tttccgaagg 4140

taactggctt cagcagagcg cagataccaa atactgtcct tctagtgtag ccgtagttag 4200

gccaccactt caagaactct gtagcaccgc ctacatacct cgctctgcta atcctgttac 4260

cagtggctgc tgccagtggc gataagtcgt gtcttaccgg gttggactca agacgatagt 4320

taccggataa ggcgcagcgg tcgggctgaa cggggggttc gtgcacacag cccagcttgg 4380

agcgaacgac ctacaccgaa ctgagatacc tacagcgtga gctatgagaa agcgccacgc 4440

ttcccgaagg gagaaaggcg gacaggtatc cggtaagcgg cagggtcgga acaggagagc 4500

gcacgaggga gcttccaggg ggaaacgcct ggtatcttta tagtcctgtc gggtttcgcc 4560

acctctgact tgagcgtcga tttttgtgat gctcgtcagg ggggcggagc ctatggaaaa 4620

acgccagcaa cgcggccttt ttacggttcc tggccttttg ctggcctttt gctcacatgt 4680

tctttcctgc gttatcccct gattctgtgg ataaccgtat taccgccatg cat 4733
<210> 17
   <211> 6816
   <212> DNA
   <213> synthetic
<400> 17 
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacgccacc atgcaggtga 660

gcaagaggat gctggcgggg ggcgtgagga gcatgcccag ccccctcctg gcctgctggc 720

agcccatcct cctgctggtg ctgggctcag tgctgtcagg ctcggccacg ggctgcccgc 780

cccgctgcga gtgctccgcc caggaccgcg ctgtgctgtg ccaccgcaag cgctttgtgg 840

cagtccccga gggcatcccc accgagacgc gcctgctgga cctaggcaag aaccgcatca 900

aaacgctcaa ccaggacgag ttcgccagct tcccgcacct ggaggagctg gagctcaacg 960

agaacatcgt gagcgccgtg gagcccggcg ccttcaacaa cctcttcaac ctccggacgc 1020
 
tgggtctccg cagcaaccgc ctgaagctca tcccgctagg cgtcttcact ggcctcagca 1080

acctgaccaa gctggacatc agcgagaaca agatcgttat cctactggac tacatgtttc 1140

aggacctgta caacctcaag tcactggagg ttggcgacaa tgacctcgtc tacatctctc 1200

accgcgcctt cagcggcctc aacagcctgg agcagctgac gctggagaaa tgcaacctga 1260

cctccatccc caccgaggcg ctgtcccacc tgcacggcct catcgtcctg aggctccggc 1320

acctcaacat caatgccatc cgggactact ccttcaagag gctgtaccga ctcaaggtct 1380

tggagatctc ccactggccc tacttggaca ccatgacacc caactgcctc tacggcctca 1440

acctgacgtc cctgtccatc acacactgca atctgaccgc tgtgccctac ctggccgtcc 1500

gccacctagt ctatctccgc ttcctcaacc tctcctacaa ccccatcagc accattgagg 1560

gctccatgtt gcatgagctg ctccggctgc aggagatcca gctggtgggc gggcagctgg 1620

ccgtggtgga gccctatgcc ttccgcggcc tcaactacct gcgcgtgctc aatgtctctg 1680

gcaaccagct gaccacactg gaggaatcag tcttccactc ggtgggcaac ctggagacac 1740

tcatcctgga ctccaacccg ctggcctgcg actgtcggct cctgtgggtg ttccggcgcc 1800

gctggcggct caacttcaac cggcagcagc ccacgtgcgc cacgcccgag tttgtccagg 1860

gcaaggagtt caaggacttc cctgatgtgc tactgcccaa ctacttcacc tgccgccgcg 1920

cccgcatccg ggaccgcaag gcccagcagg tgtttgtgga cgagggccac acggtgcagt 1980

ttgtgtgccg ggccgatggc gacccgccgc ccgccatcct ctggctctca ccccgaaagc 2040

acctggtctc agccaagagc aatgggcggc tcacagtctt ccctgatggc acgctggagg 2100

tgcgctacgc ccaggtacag gacaacggca cgtacctgtg catcgcggcc aacgcgggcg 2160

gcaacgactc catgcccgcc cacctgcatg tgcgcagcta ctcgcccgac tggccccatc 2220

agcccaacaa gaccttcgct ttcatctcca accagccggg cgagggagag gccaacagca 2280

cccgcgccac tgtgcctttc cccttcgaca tcaagaccct catcatcgcc accaccatgg 2340

gcttcatctc tttcctgggc gtcgtcctct tctgcctggt gctgctgttt ctctggagcc 2400

ggggcaaggg caacacaaag cacaacatcg agatcgagta tgtgccccga aagtcggacg 2460

caggcatcag ctccgccgac gcgccccgca agttcaacat gaagatgata tcacgggatc 2520

caccggtagc aaccatgacc agcaaggtgt acgaccccga gcagaggaag aggatgatca 2580

ccggccccca gtggtgggcc aggtgcaagc agatgaacgt gctggacagc ttcatcaact 2640

actacgacag cgagaagcac gccgagaacg ccgtgatctt cctgcacggc aacgccgcta 2700

gtagctacct gtggaggcac gtggtgcccc acatcgagcc cgtggccagg tgcatcatcc 2760

ccgatctgat cggcatgggc aagagcggca agagcggcaa cggcagctac aggctgctgg 2820

accactacaa gtacctgacc gcctggttcg agctcctgaa cctgcccaag aagatcatct 2880

tcgtgggcca cgactggggc gcctgcctgg ccttccacta cagctacgag caccaggaca 2940
 
agatcaaggc catcgtgcac gccgagagcg tggtggacgt gatcgagagc tgggacgagt 3000

ggccagacat cgaggaggac atcgccctga tcaagagcga ggagggcgag aagatggtgc 3060

tggagaacaa cttcttcgtg gagaccatgc tgcccagcaa gatcatgaga aagctggagc 3120

ccgaggagtt cgccgcctac ctggagccct tcaaggagaa gggcgaggtg agaagaccca 3180

ccctgagctg gcccagagag atccccctgg tgaagggcgg caagcccgac gtggtgcaga 3240

tcgtgagaaa ctacaacgcc tacctgagag ccagcgacga cctgcccaag atgttcatcg 3300

agagcgaccc cggcttcttc agcaacgcca tcgtggaggg cgccaagaag ttccccaaca 3360

ccgagttcgt gaaggtgaag ggcctgcact tcagccagga ggacgccccc gacgagatgg 3420

gcaagtacat caagagcttc gtggagagag tgctgaagaa cgagcagtaa tgtacaagta 3480

aagcggccgc gactctagat cataatcagc cataccacat ttgtagaggt tttacttgct 3540

ttaaaaaacc tcccacacct ccccctgaac ctgaaacata aaatgaatgc aattgttgtt 3600

gttaacttgt ttattgcagc ttataatggt tacaaataaa gcaatagcat cacaaatttc 3660

acaaataaag catttttttc actgcattct agttgtggtt tgtccaaact catcaatgta 3720

tcttaaggcg taaattgtaa gcgttaatat tttgttaaaa ttcgcgttaa atttttgtta 3780

aatcagctca ttttttaacc aataggccga aatcggcaaa atcccttata aatcaaaaga 3840

atagaccgag atagggttga gtgttgttcc agtttggaac aagagtccac tattaaagaa 3900

cgtggactcc aacgtcaaag ggcgaaaaac cgtctatcag ggcgatggcc cactacgtga 3960

accatcaccc taatcaagtt ttttggggtc gaggtgccgt aaagcactaa atcggaaccc 4020

taaagggagc ccccgattta gagcttgacg gggaaagccg gcgaacgtgg cgagaaagga 4080

agggaagaaa gcgaaaggag cgggcgctag ggcgctggca agtgtagcgg tcacgctgcg 4140

cgtaaccacc acacccgccg cgcttaatgc gccgctacag ggcgcgtcag gtggcacttt 4200

tcggggaaat gtgcgcggaa cccctatttg tttatttttc taaatacatt caaatatgta 4260

tccgctcatg agacaataac cctgataaat gcttcaataa tattgaaaaa ggaagagtcc 4320

tgaggcggaa agaaccagct gtggaatgtg tgtcagttag ggtgtggaaa gtccccaggc 4380

tccccagcag gcagaagtat gcaaagcatg catctcaatt agtcagcaac caggtgtgga 4440

aagtccccag gctccccagc aggcagaagt atgcaaagca tgcatctcaa ttagtcagca 4500

accatagtcc cgcccctaac tccgcccatc ccgcccctaa ctccgcccag ttccgcccat 4560

tctccgcccc atggctgact aatttttttt atttatgcag aggccgaggc cgcctcggcc 4620

tctgagctat tccagaagta gtgaggaggc ttttttggag gcctaggctt ttgcaaagat 4680

cgatcaagag acaggatgag gatcgtttcg catgattgaa caagatggat tgcacgcagg 4740

ttctccggcc gcttgggtgg agaggctatt cggctatgac tgggcacaac agacaatcgg 4800

ctgctctgat gccgccgtgt tccggctgtc agcgcagggg cgcccggttc tttttgtcaa 4860

gaccgacctg tccggtgccc tgaatgaact gcaagacgag gcagcgcggc tatcgtggct 4920
 
ggccacgacg ggcgttcctt gcgcagctgt gctcgacgtt gtcactgaag cgggaaggga 4980

ctggctgcta ttgggcgaag tgccggggca ggatctcctg tcatctcacc ttgctcctgc 5040

cgagaaagta tccatcatgg ctgatgcaat gcggcggctg catacgcttg atccggctac 5100

ctgcccattc gaccaccaag cgaaacatcg catcgagcga gcacgtactc ggatggaagc 5160

cggtcttgtc gatcaggatg atctggacga agagcatcag gggctcgcgc cagccgaact 5220

gttcgccagg ctcaaggcga gcatgcccga cggcgaggat ctcgtcgtga cccatggcga 5280

tgcctgcttg ccgaatatca tggtggaaaa tggccgcttt tctggattca tcgactgtgg 5340

ccggctgggt gtggcggacc gctatcagga catagcgttg gctacccgtg atattgctga 5400

agagcttggc ggcgaatggg ctgaccgctt cctcgtgctt tacggtatcg ccgctcccga 5460

ttcgcagcgc atcgccttct atcgccttct tgacgagttc ttctgagcgg gactctgggg 5520

ttcgaaatga ccgaccaagc gacgcccaac ctgccatcac gagatttcga ttccaccgcc 5580

gccttctatg aaaggttggg cttcggaatc gttttccggg acgccggctg gatgatcctc 5640

cagcgcgggg atctcatgct ggagttcttc gcccacccta gggggaggct aactgaaaca 5700

cggaaggaga caataccgga aggaacccgc gctatgacgg caataaaaag acagaataaa 5760

acgcacggtg ttgggtcgtt tgttcataaa cgcggggttc ggtcccaggg ctggcactct 5820

gtcgataccc caccgagacc ccattggggc caatacgccc gcgtttcttc cttttcccca 5880

ccccaccccc caagttcggg tgaaggccca gggctcgcag ccaacgtcgg ggcggcaggc 5940

cctgccatag cctcaggtta ctcatatata ctttagattg atttaaaact tcatttttaa 6000

tttaaaagga tctaggtgaa gatccttttt gataatctca tgaccaaaat cccttaacgt 6060

gagttttcgt tccactgagc gtcagacccc gtagaaaaga tcaaaggatc ttcttgagat 6120

cctttttttc tgcgcgtaat ctgctgcttg caaacaaaaa aaccaccgct accagcggtg 6180

gtttgtttgc cggatcaaga gctaccaact ctttttccga aggtaactgg cttcagcaga 6240

gcgcagatac caaatactgt ccttctagtg tagccgtagt taggccacca cttcaagaac 6300

tctgtagcac cgcctacata cctcgctctg ctaatcctgt taccagtggc tgctgccagt 6360

ggcgataagt cgtgtcttac cgggttggac tcaagacgat agttaccgga taaggcgcag 6420

cggtcgggct gaacgggggg ttcgtgcaca cagcccagct tggagcgaac gacctacacc 6480

gaactgagat acctacagcg tgagctatga gaaagcgcca cgcttcccga agggagaaag 6540

gcggacaggt atccggtaag cggcagggtc ggaacaggag agcgcacgag ggagcttcca 6600

gggggaaacg cctggtatct ttatagtcct gtcgggtttc gccacctctg acttgagcgt 6660

cgatttttgt gatgctcgtc aggggggcgg agcctatgga aaaacgccag caacgcggcc 6720

tttttacggt tcctggcctt ttgctggcct tttgctcaca tgttctttcc tgcgttatcc 6780

cctgattctg tggataaccg tattaccgcc atgcat 6816
<210> 18
   <211> 6589
   <212> DNA
   <213> synthetic
<400> 18 
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacgccacc atgcaggtga 660

gcaagaggat gctggcgggg ggcgtgagga gcatgcccag ccccctcctg gcctgctggc 720

agcccatcct cctgctggtg ctgggctcag tgctgtcagg ctcggccacg ggctgcccgc 780

cccgctgcga gtgctccgcc caggaccgcg ctgtgctgtg ccaccgcaag cgctttgtgg 840

cagtccccga gggcatcccc accgagacgc gcctgctgga cctaggcaag aaccgcatca 900

aaacgctcaa ccaggacgag ttcgccagct tcccgcacct ggaggagctg gagctcaacg 960

agaacatcgt gagcgccgtg gagcccggcg ccttcaacaa cctcttcaac ctccggacgc 1020

tgggtctccg cagcaaccgc ctgaagctca tcccgctagg cgtcttcact ggcctcagca 1080

acctgaccaa gctggacatc agcgagaaca agatcgttat cctactggac tacatgtttc 1140

aggacctgta caacctcaag tcactggagg ttggcgacaa tgacctcgtc tacatctctc 1200

accgcgcctt cagcggcctc aacagcctgg agcagctgac gctggagaaa tgcaacctga 1260

cctccatccc caccgaggcg ctgtcccacc tgcacggcct catcgtcctg aggctccggc 1320

acctcaacat caatgccatc cgggactact ccttcaagag gctgtaccga ctcaaggtct 1380

tggagatctc ccactggccc tacttggaca ccatgacacc caactgcctc tacggcctca 1440

acctgacgtc cctgtccatc acacactgca atctgaccgc tgtgccctac ctggccgtcc 1500

gccacctagt ctatctccgc ttcctcaacc tctcctacaa ccccatcagc accattgagg 1560

gctccatgtt gcatgagctg ctccggctgc aggagatcca gctggtgggc gggcagctgg 1620

ccgtggtgga gccctatgcc ttccgcggcc tcaactacct gcgcgtgctc aatgtctctg 1680

gcaaccagct gaccacactg gaggaatcag tcttccactc ggtgggcaac ctggagacac 1740
 
tcatcctgga ctccaacccg ctggcctgcg actgtcggct cctgtgggtg ttccggcgcc 1800

gctggcggct caacttcaac cggcagcagc ccacgtgcgc cacgcccgag tttgtccagg 1860

gcaaggagtt caaggacttc cctgatgtgc tactgcccaa ctacttcacc tgccgccgcg 1920

cccgcatccg ggaccgcaag gcccagcagg tgtttgtgga cgagggccac acggtgcagt 1980

ttgtgtgccg ggccgatggc gacccgccgc ccgccatcct ctggctctca ccccgaaagc 2040

acctggtctc agccaagagc aatgggcggc tcacagtctt ccctgatggc acgctggagg 2100

tgcgctacgc ccaggtacag gacaacggca cgtacctgtg catcgcggcc aacgcgggcg 2160

gcaacgactc catgcccgcc cacctgcatg tgcgcagcta ctcgcccgac tggccccatc 2220

agcccaacaa gaccttcgct ttcatctcca accagccggg cgagggagag gccaacagca 2280

cccgcgccac tgtgcctttc cccttcgaca tcaagaccct catcatcgcc accaccatgg 2340

gcttcatctc tttcctgggc gtcgtcctct tctgcctggt gctgctgttt ctctggagcc 2400

ggggcaaggg caacacaaag cacaacatcg agatcgagta tgtgccccga aagtcggacg 2460

caggcatcag ctccgccgac gcgccccgca agttcaacat gaagatgata tcacgggatc 2520

caccggtcgc caccatggtg agcaagggcg aggagctgtt caccggggtg gtgcccatcc 2580

tggtcgagct ggacggcgac gtaaacggcc acaagttcag cgtgtccggc gagggcgagg 2640

gcgatgccac ctacggcaag ctgaccctga agttcatctg caccaccggc aagctgcccg 2700

tgccctggcc caccctcgtg accaccttcg gctacggcct gcagtgcttc gcccgctacc 2760

ccgaccacat gaagcagcac gacttcttca agtccgccat gcccgaaggc tacgtccagg 2820

agcgcaccat cttcttcaag gacgacggca actacaagac ccgcgccgag gtgaagttcg 2880

agggcgacac cctggtgaac cgcatcgagc tgaagggcat cgacttcaag gaggacggca 2940

acatcctggg gcacaagctg gagtacaact acaacagcca caacgtctat atcatggccg 3000

acaagcagaa gaacggcatc aaggtgaact tcaagatccg ccacaacatc gaggacggca 3060

gcgtgcagct cgccgaccac taccagcaga acacccccat cggcgacggc cccgtgctgc 3120

tgcccgacaa ccactacctg agctaccagt ccgccctgag caaagacccc aacgagaagc 3180

gcgatcacat ggtcctgctg gagttcgtga ccgccgccgg gatcactctc ggcatggacg 3240

agctgtacaa gtaaagcggc cgcgactcta gatcataatc agccatacca catttgtaga 3300

ggttttactt gctttaaaaa acctcccaca cctccccctg aacctgaaac ataaaatgaa 3360

tgcaattgtt gttgttaact tgtttattgc agcttataat ggttacaaat aaagcaatag 3420

catcacaaat ttcacaaata aagcattttt ttcactgcat tctagttgtg gtttgtccaa 3480

actcatcaat gtatcttaag gcgtaaattg taagcgttaa tattttgtta aaattcgcgt 3540

taaatttttg ttaaatcagc tcatttttta accaataggc cgaaatcggc aaaatccctt 3600

ataaatcaaa agaatagacc gagatagggt tgagtgttgt tccagtttgg aacaagagtc 3660

cactattaaa gaacgtggac tccaacgtca aagggcgaaa aaccgtctat cagggcgatg 3720
 
gcccactacg tgaaccatca ccctaatcaa gttttttggg gtcgaggtgc cgtaaagcac 3780

taaatcggaa ccctaaaggg agcccccgat ttagagcttg acggggaaag ccggcgaacg 3840

tggcgagaaa ggaagggaag aaagcgaaag gagcgggcgc tagggcgctg gcaagtgtag 3900

cggtcacgct gcgcgtaacc accacacccg ccgcgcttaa tgcgccgcta cagggcgcgt 3960

caggtggcac ttttcgggga aatgtgcgcg gaacccctat ttgtttattt ttctaaatac 4020

attcaaatat gtatccgctc atgagacaat aaccctgata aatgcttcaa taatattgaa 4080

aaaggaagag tcctgaggcg gaaagaacca gctgtggaat gtgtgtcagt tagggtgtgg 4140

aaagtcccca ggctccccag caggcagaag tatgcaaagc atgcatctca attagtcagc 4200

aaccaggtgt ggaaagtccc caggctcccc agcaggcaga agtatgcaaa gcatgcatct 4260

caattagtca gcaaccatag tcccgcccct aactccgccc atcccgcccc taactccgcc 4320

cagttccgcc cattctccgc cccatggctg actaattttt tttatttatg cagaggccga 4380

ggccgcctcg gcctctgagc tattccagaa gtagtgagga ggcttttttg gaggcctagg 4440

cttttgcaaa gatcgatcaa gagacaggat gaggatcgtt tcgcatgatt gaacaagatg 4500

gattgcacgc aggttctccg gccgcttggg tggagaggct attcggctat gactgggcac 4560

aacagacaat cggctgctct gatgccgccg tgttccggct gtcagcgcag gggcgcccgg 4620

ttctttttgt caagaccgac ctgtccggtg ccctgaatga actgcaagac gaggcagcgc 4680

ggctatcgtg gctggccacg acgggcgttc cttgcgcagc tgtgctcgac gttgtcactg 4740

aagcgggaag ggactggctg ctattgggcg aagtgccggg gcaggatctc ctgtcatctc 4800

accttgctcc tgccgagaaa gtatccatca tggctgatgc aatgcggcgg ctgcatacgc 4860

ttgatccggc tacctgccca ttcgaccacc aagcgaaaca tcgcatcgag cgagcacgta 4920

ctcggatgga agccggtctt gtcgatcagg atgatctgga cgaagagcat caggggctcg 4980

cgccagccga actgttcgcc aggctcaagg cgagcatgcc cgacggcgag gatctcgtcg 5040

tgacccatgg cgatgcctgc ttgccgaata tcatggtgga aaatggccgc ttttctggat 5100

tcatcgactg tggccggctg ggtgtggcgg accgctatca ggacatagcg ttggctaccc 5160

gtgatattgc tgaagagctt ggcggcgaat gggctgaccg cttcctcgtg ctttacggta 5220

tcgccgctcc cgattcgcag cgcatcgcct tctatcgcct tcttgacgag ttcttctgag 5280

cgggactctg gggttcgaaa tgaccgacca agcgacgccc aacctgccat cacgagattt 5340

cgattccacc gccgccttct atgaaaggtt gggcttcgga atcgttttcc gggacgccgg 5400

ctggatgatc ctccagcgcg gggatctcat gctggagttc ttcgcccacc ctagggggag 5460

gctaactgaa acacggaagg agacaatacc ggaaggaacc cgcgctatga cggcaataaa 5520

aagacagaat aaaacgcacg gtgttgggtc gtttgttcat aaacgcgggg ttcggtccca 5580

gggctggcac tctgtcgata ccccaccgag accccattgg ggccaatacg cccgcgtttc 5640
 
ttccttttcc ccaccccacc ccccaagttc gggtgaaggc ccagggctcg cagccaacgt 5700

cggggcggca ggccctgcca tagcctcagg ttactcatat atactttaga ttgatttaaa 5760

acttcatttt taatttaaaa ggatctaggt gaagatcctt tttgataatc tcatgaccaa 5820

aatcccttaa cgtgagtttt cgttccactg agcgtcagac cccgtagaaa agatcaaagg 5880

atcttcttga gatccttttt ttctgcgcgt aatctgctgc ttgcaaacaa aaaaaccacc 5940

gctaccagcg gtggtttgtt tgccggatca agagctacca actctttttc cgaaggtaac 6000

tggcttcagc agagcgcaga taccaaatac tgtccttcta gtgtagccgt agttaggcca 6060

ccacttcaag aactctgtag caccgcctac atacctcgct ctgctaatcc tgttaccagt 6120

ggctgctgcc agtggcgata agtcgtgtct taccgggttg gactcaagac gatagttacc 6180

ggataaggcg cagcggtcgg gctgaacggg gggttcgtgc acacagccca gcttggagcg 6240

aacgacctac accgaactga gatacctaca gcgtgagcta tgagaaagcg ccacgcttcc 6300

cgaagggaga aaggcggaca ggtatccggt aagcggcagg gtcggaacag gagagcgcac 6360

gagggagctt ccagggggaa acgcctggta tctttatagt cctgtcgggt ttcgccacct 6420

ctgacttgag cgtcgatttt tgtgatgctc gtcagggggg cggagcctat ggaaaaacgc 6480

cagcaacgcg gcctttttac ggttcctggc cttttgctgg ccttttgctc acatgttctt 6540

tcctgcgtta tcccctgatt ctgtggataa ccgtattacc gccatgcat 6589
<210> 19
   <211> 7311
   <212> DNA
   <213> synthetic
<400> 19

gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840
ctgcttactg gcttatcgaa attaatacga ctcactatag ggagacccaa gcttgccacc 900
atgcaggtga gcaagaggat gctggcgggg ggcgtgagga gcatgcccag ccccctcctg 960
gcctgctggc agcccatcct cctgctggtg ctgggctcag tgctgtcagg ctcggccacg 1020
ggctgcccgc cccgctgcga gtgctccgcc caggaccgcg ctgtgctgtg ccaccgcaag 1080
cgctttgtgg cagtccccga gggcatcccc accgagacgc gcctgctgga cctaggcaag 1140
aaccgcatca aaacgctcaa ccaggacgag ttcgccagct tcccgcacct ggaggagctg 1200
gagctcaacg agaacatcgt gagcgccgtg gagcccggcg ccttcaacaa cctcttcaac 1260
ctccggacgc tgggtctccg cagcaaccgc ctgaagctca tcccgctagg cgtcttcact 1320
ggcctcagca acctgaccaa gctggacatc agcgagaaca agatcgttat cctactggac 1380
tacatgtttc aggacctgta caacctcaag tcactggagg ttggcgacaa tgacctcgtc 1440
tacatctctc accgcgcctt cagcggcctc aacagcctgg agcagctgac gctggagaaa 1500
tgcaacctga cctccatccc caccgaggcg ctgtcccacc tgcacggcct catcgtcctg 1560
aggctccggc acctcaacat caatgccatc cgggactact ccttcaagag gctgtaccga 1620
ctcaaggtct tggagatctc ccactggccc tacttggaca ccatgacacc caactgcctc 1680
tacggcctca acctgacgtc cctgtccatc acacactgca atctgaccgc tgtgccctac 1740
ctggccgtcc gccacctagt ctatctccgc ttcctcaacc tctcctacaa ccccatcagc 1800
accattgagg gctccatgtt gcatgagctg ctccggctgc aggagatcca gctggtgggc 1860
gggcagctgg ccgtggtgga gccctatgcc ttccgcggcc tcaactacct gcgcgtgctc 1920
aatgtctctg gcaaccagct gaccacactg gaggaatcag tcttccactc ggtgggcaac 1980
ctggagacac tcatcctgga ctccaacccg ctggcctgcg actgtcggct cctgtgggtg 2040
ttccggcgcc gctggcggct caacttcaac cggcagcagc ccacgtgcgc cacgcccgag 2100
tttgtccagg gcaaggagtt caaggacttc cctgatgtgc tactgcccaa ctacttcacc 2160
tgccgccgcg cccgcatccg ggaccgcaag gcccagcagg tgtttgtgga cgagggccac 2220
acggtgcagt ttgtgtgccg ggccgatggc gacccgccgc ccgccatcct ctggctctca 2280
ccccgaaagc acctggtctc agccaagagc aatgggcggc tcacagtctt ccctgatggc 2340
acgctggagg tgcgctacgc ccaggtacag gacaacggca cgtacctgtg catcgcggcc 2400
aacgcgggcg gcaacgactc catgcccgcc cacctgcatg tgcgcagcta ctcgcccgac 2460
tggccccatc agcccaacaa gaccttcgct ttcatctcca accagccggg cgagggagag 2520
gccaacagca cccgcgccac tgtgcctttc cccttcgaca tcaagaccct catcatcgcc 2580
accaccatgg gcttcatctc tttcctgggc gtcgtcctct tctgcctggt gctgctgttt 2640
ctctggagcc ggggcaaggg caacacaaag cacaacatcg agatcgagta tgtgccccga 2700
aagtcggacg caggcatcag ctccgccgac gcgccccgca agttcaacat gaagatgata 2760

ggatccgcta gctatccata tgatgttcct gattatgctt gagaattctg cagatatcca 2820

tcacactggc ggccgctcga gcatgcatct agagggccct attctatagt gtcacctaaa 2880

tgctagagct cgctgatcag cctcgactgt gccttctagt tgccagccat ctgttgtttg 2940

cccctccccc gtgccttcct tgaccctgga aggtgccact cccactgtcc tttcctaata 3000

aaatgaggaa attgcatcgc attgtctgag taggtgtcat tctattctgg ggggtggggt 3060

ggggcaggac agcaaggggg aggattggga agacaatagc aggcatgctg gggatgcggt 3120

gggctctatg gcttctgagg cggaaagaac cagctggggc tctagggggt atccccacgc 3180

gccctgtagc ggcgcattaa gcgcggcggg tgtggtggtt acgcgcagcg tgaccgctac 3240

acttgccagc gccctagcgc ccgctccttt cgctttcttc ccttcctttc tcgccacgtt 3300

cgccggcttt ccccgtcaag ctctaaatcg ggcatccctt tagggttccg atttagtgct 3360

ttacggcacc tcgaccccaa aaaacttgat tagggtgatg gttcacgtag tgggccatcg 3420

ccctgataga cggtttttcg ccctttgacg ttggagtcca cgttctttaa tagtggactc 3480

ttgttccaaa ctggaacaac actcaaccct atctcggtct attcttttga tttataaggg 3540

attttgggga tttcggccta ttggttaaaa aatgagctga tttaacaaaa atttaacgcg 3600

aattaattct gtggaatgtg tgtcagttag ggtgtggaaa gtccccaggc tccccaggca 3660

ggcagaagta tgcaaagcat gcatctcaat tagtcagcaa ccaggtgtgg aaagtcccca 3720

ggctccccag caggcagaag tatgcaaagc atgcatctca attagtcagc aaccatagtc 3780

ccgcccctaa ctccgcccat cccgccccta actccgccca gttccgccca ttctccgccc 3840

catggctgac taattttttt tatttatgca gaggccgagg ccgcctctgc ctctgagcta 3900

ttccagaagt agtgaggagg cttttttgga ggcctaggct tttgcaaaaa gctcccggga 3960

gcttgtatat ccattttcgg atctgatcaa gagacaggat gaggatcgtt tcgcatgatt 4020

gaacaagatg gattgcacgc aggttctccg gccgcttggg tggagaggct attcggctat 4080

gactgggcac aacagacaat cggctgctct gatgccgccg tgttccggct gtcagcgcag 4140

gggcgcccgg ttctttttgt caagaccgac ctgtccggtg ccctgaatga actgcaggac 4200

gaggcagcgc ggctatcgtg gctggccacg acgggcgttc cttgcgcagc tgtgctcgac 4260

gttgtcactg aagcgggaag ggactggctg ctattgggcg aagtgccggg gcaggatctc 4320

ctgtcatctc accttgctcc tgccgagaaa gtatccatca tggctgatgc aatgcggcgg 4380

ctgcatacgc ttgatccggc tacctgccca ttcgaccacc aagcgaaaca tcgcatcgag 4440

cgagcacgta ctcggatgga agccggtctt gtcgatcagg atgatctgga cgaagagcat 4500

caggggctcg cgccagccga actgttcgcc aggctcaagg cgcgcatgcc cgacggcgag 4560

gatctcgtcg tgacccatgg cgatgcctgc ttgccgaata tcatggtgga aaatggccgc 4620

ttttctggat tcatcgactg tggccggctg ggtgtggcgg accgctatca ggacatagcg 4680

gttggctacc cgtgatattg ctgaagagct tggcggcgaa tgggctgacc gcttcctcgt 4740

gctttacggt atcgccgctc ccgattcgca gcgcatcgcc ttctatcgcc ttcttgacga 4800

gttcttctga gcgggactct ggggttcgaa atgaccgacc aagcgacgcc caacctgcca 4860

tcacgagatt tcgattccac cgccgccttc tatgaaaggt tgggcttcgg aatcgttttc 4920

cgggacgccg gctggatgat cctccagcgc ggggatctca tgctggagtt cttcgcccac 4980

cccaacttgt ttattgcagc ttataatggt tacaaataaa gcaatagcat cacaaatttc 5040

acaaataaag catttttttc actgcattct agttgtggtt tgtccaaact catcaatgta 5100

tcttatcatg tctgtatacc gtcgacctct agctagagct tggcgtaatc atggtcatag 5160

ctgtttcctg tgtgaaattg ttatccgctc acaattccac acaacatacg agccggaagc 5220

ataaagtgta aagcctgggg tgcctaatga gtgagctaac tcacattaat tgcgttgcgc 5280

tcactgcccg ctttccagtc gggaaacctg tcgtgccagc tgcattaatg aatcggccaa 5340

cgcgcgggga gaggcggttt gcgtattggg cgctcttccg cttcctcgct cactgactcg 5400

ctgcgctcgg tcgttcggct gcggcgagcg gtatcagctc actcaaaggc ggtaatacgg 5460

ttatccacag aatcagggga taacgcagga aagaacatgt gagcaaaagg ccagcaaaag 5520

gccaggaacc gtaaaaaggc cgcgttgctg gcgtttttcc ataggctccg cccccctgac 5580

gagcatcaca aaaatcgacg ctcaagtcag aggtggcgaa acccgacagg actataaaga 5640

taccaggcgt ttccccctgg aagctccctc gtgcgctctc ctgttccgac cctgccgctt 5700

accggatacc tgtccgcctt tctcccttcg ggaagcgtgg cgctttctca atgctcacgc 5760

tgtaggtatc tcagttcggt gtaggtcgtt cgctccaagc tgggctgtgt gcacgaaccc 5820

cccgttcagc ccgaccgctg cgccttatcc ggtaactatc gtcttgagtc caacccggta 5880

agacacgact tatcgccact ggcagcagcc actggtaaca ggattagcag agcgaggtat 5940

gtaggcggtg ctacagagtt cttgaagtgg tggcctaact acggctacac tagaaggaca 6000

gtatttggta tctgcgctct gctgaagcca gttaccttcg gaaaaagagt tggtagctct 6060

tgatccggca aacaaaccac cgctggtagc ggtggttttt ttgtttgcaa gcagcagatt 6120

acgcgcagaa aaaaaggatc tcaagaagat cctttgatct tttctacggg gtctgacgct 6180

cagtggaacg aaaactcacg ttaagggatt ttggtcatga gattatcaaa aaggatcttc 6240

acctagatcc ttttaaatta aaaatgaagt tttaaatcaa tctaaagtat atatgagtaa 6300

acttggtctg acagttacca atgcttaatc agtgaggcac ctatctcagc gatctgtcta 6360

tttcgttcat ccatagttgc ctgactcccc gtcgtgtaga taactacgat acgggagggc 6420

ttaccatctg gccccagtgc tgcaatgata ccgcgagacc cacgctcacc ggctccagat 6480

ttatcagcaa taaaccagcc agccggaagg gccgagcgca gaagtggtcc tgcaacttta 6540

tccgcctcca tccagtctat taattgttgc cgggaagcta gagtaagtag ttcgccagtt 6600

aatagtttgc gcaacgttgt tgccattgct acaggcatcg tggtgtcacg ctcgtcgttt 6660

ggtatggctt cattcagctc cggttcccaa cgatcaaggc gagttacatg atcccccatg 6720

ttgtgcaaaa aagcggttag ctccttcggt cctccgatcg ttgtcagaag taagttggcc 6780

gcagtgttat cactcatggt tatggcagca ctgcataatt ctcttactgt catgccatcc 6840

gtaagatgct tttctgtgac tggtgagtac tcaaccaagt cattctgaga atagtgtatg 6900

cggcgaccga gttgctcttg cccggcgtca atacgggata ataccgcgcc acatagcaga 6960

actttaaaag tgctcatcat tggaaaacgt tcttcggggc gaaaactctc aaggatctta 7020

ccgctgttga gatccagttc gatgtaaccc actcgtgcac ccaactgatc ttcagcatct 7080

tttactttca ccagcgtttc tgggtgagca aaaacaggaa ggcaaaatgc cgcaaaaaag 7140

ggaataaggg cgacacggaa atgttgaata ctcatactct tcctttttca atattattga 7200

agcatttatc agggttattg tctcatgagc ggatacatat ttgaatgtat ttagaaaaat 7260

aaacaaatag gggttccgcg cacatttccc cgaaaagtgc cacctgacgt c 7311

SEQUENCE LISTING

<110>  Centre National de la Recherche Scientifique (CNRS)

<120>  Outils pour l'identification de ligands de Lingo-1, Lingo-2,
       Lingo-3 et Lingo-4, et utilisations

<130>  BIP205699PC00

<150>  FR 10/01374
<151>  2010-04-01

<160>  19

<170>  PatentIn version 3.5

<210>  1
<211>  620
<212>  PRT
<213>  Homo sapiens

<400>  1

Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
        180                 185                 190

Leu Glu Gln Leu Thr Leu Glu Lys Cys Asn Leu Thr Ser Ile Pro Thr
    195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
    370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile
    610                 615                 620

<210>  2
<211>  606
<212>  PRT
<213>  Homo sapiens

<400>  2

Met Leu His Thr Ala Ile Ser Cys Trp Gln Pro Phe Leu Gly Leu Ala
1               5                   10                  15

Val Val Leu Ile Phe Met Gly Ser Thr Ile Gly Cys Pro Ala Arg Cys
            20                  25                  30

Glu Cys Ser Ala Gln Asn Lys Ser Val Ser Cys His Arg Arg Arg Leu

35                  40                  45

Ile Ala Ile Pro Glu Gly Ile Pro Ile Glu Thr Lys Ile Leu Asp Leu
    50                  55                  60

Ser Lys Asn Arg Leu Lys Ser Val Asn Pro Glu Glu Phe Ile Ser Tyr
65                  70                  75                  80

Pro Leu Leu Glu Glu Ile Asp Leu Ser Asp Asn Ile Ile Ala Asn Val
                85                  90                  95

Glu Pro Gly Ala Phe Asn Asn Leu Phe Asn Leu Arg Ser Leu Arg Leu
            100                 105                 110

Lys Gly Asn Arg Leu Lys Leu Val Pro Leu Gly Val Phe Thr Gly Leu
        115                 120                 125

Ser Asn Leu Thr Lys Leu Asp Ile Ser Glu Asn Lys Ile Val Ile Leu
    130                 135                 140

Leu Asp Tyr Met Phe Gln Asp Leu His Asn Leu Lys Ser Leu Glu Val
145                 150                 155                 160

Gly Asp Asn Asp Leu Val Tyr Ile Ser His Arg Ala Phe Ser Gly Leu
                165                 170                 175

Leu Ser Leu Glu Gln Leu Thr Leu Glu Lys Cys Asn Leu Thr Ala Val
            180                 185                 190

Pro Thr Glu Ala Leu Ser His Leu Arg Ser Leu Ile Ser Leu His Leu
        195                 200                 205

Lys His Leu Asn Ile Asn Asn Met Pro Val Tyr Ala Phe Lys Arg Leu
    210                 215                 220

Phe His Leu Lys His Leu Glu Ile Asp Tyr Trp Pro Leu Leu Asp Met
225                 230                 235                 240

Met Pro Ala Asn Ser Leu Tyr Gly Leu Asn Leu Thr Ser Leu Ser Val
                245                 250                 255

Thr Asn Thr Asn Leu Ser Thr Val Pro Phe Leu Ala Phe Lys His Leu
            260                 265                 270

Val Tyr Leu Thr His Leu Asn Leu Ser Tyr Asn Pro Ile Ser Thr Ile
        275                 280                 285

Glu Ala Gly Met Phe Ser Asp Leu Ile Arg Leu Gln Glu Leu His Ile
    290                 295                 300

Val Gly Ala Gln Leu Arg Thr Ile Glu Pro His Ser Phe Gln Gly Leu
305                 310                 315                 320

Arg Phe Leu Arg Val Leu Asn Val Ser Gln Asn Leu Leu Glu Thr Leu
                325                 330                 335

Glu Glu Asn Val Phe Ser Ser Pro Arg Ala Leu Glu Val Leu Ser Ile
            340                 345                 350

Asn Asn Asn Pro Leu Ala Cys Asp Cys Arg Leu Leu Trp Ile Leu Gln
        355                 360                 365

Arg Gln Pro Thr Leu Gln Phe Gly Gly Gln Gln Pro Met Cys Ala Gly
    370                 375                 380

Pro Asp Thr Ile Arg Glu Arg Ser Phe Lys Asp Phe His Ser Thr Ala
385                 390                 395                 400

Leu Ser Phe Tyr Phe Thr Cys Lys Lys Pro Lys Ile Arg Glu Lys Lys
                405                 410                 415

Leu Gln His Leu Leu Val Asp Glu Gly Gln Thr Val Gln Leu Glu Cys
            420                 425                 430

Ser Ala Asp Gly Asp Pro Gln Pro Val Ile Ser Trp Val Thr Pro Arg
        435                 440                 445

Arg Arg Phe Ile Thr Thr Lys Ser Asn Gly Arg Ala Thr Val Leu Gly
    450                 455                 460

Asp Gly Thr Leu Glu Ile Arg Phe Ala Gln Asp Gln Asp Ser Gly Met
465                 470                 475                 480

Tyr Val Cys Ile Ala Ser Asn Ala Ala Gly Asn Asp Thr Phe Thr Ala
                485                 490                 495

Ser Leu Thr Val Lys Gly Phe Ala Ser Asp Arg Phe Leu Tyr Ala Asn
            500                 505                 510

Arg Thr Pro Met Tyr Met Thr Asp Ser Asn Asp Thr Ile Ser Asn Gly
        515                 520                 525

Thr Asn Ala Asn Thr Phe Ser Leu Asp Leu Lys Thr Ile Leu Val Ser
    530                 535                 540

Thr Ala Met Gly Cys Phe Thr Phe Leu Gly Val Val Leu Phe Cys Phe
545                 550                 555                 560

Leu Leu Leu Phe Val Trp Ser Arg Gly Lys Gly Lys His Lys Asn Ser
                565                 570                 575

Ile Asp Leu Glu Tyr Val Pro Arg Lys Asn Asn Gly Ala Val Val Glu
            580                 585                 590

Gly Glu Val Ala Gly Pro Arg Arg Phe Asn Met Lys Met Ile
        595                 600                 605

<210>  3
<211>  592
<212>  PRT
<213>  Homo sapiens

<400>  3

Met Thr Cys Trp Leu Cys Val Leu Ser Leu Pro Leu Leu Leu Leu Pro
1               5                   10                  15

Ala Ala Pro Pro Pro Ala Gly Gly Cys Pro Ala Arg Cys Glu Cys Thr
            20                  25                  30

Val Gln Thr Arg Ala Val Ala Cys Thr Arg Arg Arg Leu Thr Ala Val
        35                  40                  45

Pro Asp Gly Ile Pro Ala Glu Thr Arg Leu Leu Glu Leu Ser Arg Asn
    50                  55                  60

Arg Ile Arg Cys Leu Asn Pro Gly Asp Leu Ala Ala Leu Pro Ala Leu
65                  70                  75                  80

Glu Glu Leu Asp Leu Ser Glu Asn Ala Ile Ala His Val Glu Pro Gly
                85                  90                  95

Ala Phe Ala Asn Leu Pro Arg Leu Arg Val Leu Arg Leu Arg Gly Asn
            100                 105                 110

Gln Leu Lys Leu Ile Pro Pro Gly Val Phe Thr Arg Leu Asp Asn Leu
        115                 120                 125

Thr Leu Leu Asp Leu Ser Glu Asn Lys Leu Val Ile Leu Leu Asp Tyr
    130                 135                 140

Thr Phe Gln Asp Leu His Ser Leu Arg Arg Leu Glu Val Gly Asp Asn
145                 150                 155                 160

Asp Leu Val Phe Val Ser Arg Arg Ala Phe Ala Gly Leu Leu Ala Leu
                165                 170                 175

Glu Glu Leu Thr Leu Glu Arg Cys Asn Leu Thr Ala Leu Ser Gly Glu

            180                 185                 190

Ser Leu Gly His Leu Arg Ser Leu Gly Ala Leu Arg Leu Arg His Leu
        195                 200                 205

Ala Ile Ala Ser Leu Glu Asp Gln Asn Phe Arg Arg Leu Pro Gly Leu
    210                 215                 220

Leu His Leu Glu Ile Asp Asn Trp Pro Leu Leu Glu Glu Val Ala Ala
225                 230                 235                 240

Gly Ser Leu Arg Gly Leu Asn Leu Thr Ser Leu Ser Val Thr His Thr
                245                 250                 255

Asn Ile Thr Ala Val Pro Ala Ala Ala Leu Arg His Gln Ala His Leu
            260                 265                 270

Thr Cys Leu Asn Leu Ser His Asn Pro Ile Ser Thr Val Pro Arg Gly
        275                 280                 285

Ser Phe Arg Asp Leu Val Arg Leu Arg Glu Leu His Leu Ala Gly Ala
    290                 295                 300

Leu Leu Ala Val Val Glu Pro Gln Ala Phe Leu Gly Leu Arg Gln Ile
305                 310                 315                 320

Arg Leu Leu Asn Leu Ser Asn Asn Leu Leu Ser Thr Leu Glu Glu Ser
                325                 330                 335

Thr Phe His Ser Val Asn Thr Leu Glu Thr Leu Arg Val Asp Gly Asn
            340                 345                 350

Pro Leu Ala Cys Asp Cys Arg Leu Leu Trp Ile Val Gln Arg Arg Lys
        355                 360                 365

Thr Leu Asn Phe Asp Gly Arg Leu Pro Ala Cys Ala Thr Pro Ala Glu
    370                 375                 380

Val Arg Gly Asp Ala Leu Arg Asn Leu Pro Asp Ser Val Leu Phe Glu
385                 390                 395                 400

Tyr Phe Val Cys Arg Lys Pro Lys Ile Arg Glu Arg Arg Leu Gln Arg
                405                 410                 415

Val Thr Ala Thr Ala Gly Glu Asp Val Arg Phe Leu Cys Arg Ala Glu
            420                 425                 430

Gly Glu Pro Ala Pro Thr Val Ala Trp Val Thr Pro Gln His Arg Pro
        435                 440                 445

Val Thr Ala Thr Ser Ala Gly Arg Ala Arg Val Leu Pro Gly Gly Thr
450                 455                 460

Leu Glu Ile Gln Asp Ala Arg Pro Gln Asp Ser Gly Thr Tyr Thr Cys
465                 470                 475                 480

Val Ala Ser Asn Ala Gly Gly Asn Asp Thr Tyr Phe Ala Thr Leu Thr
                485                 490                 495

Val Arg Pro Glu Pro Ala Ala Asn Arg Thr Pro Gly Glu Ala His Asn
            500                 505                 510

Glu Thr Leu Ala Ala Leu Arg Ala Pro Leu Asp Leu Thr Thr Ile Leu
        515                 520                 525

Val Ser Thr Ala Met Gly Cys Ile Thr Phe Leu Gly Val Val Leu Phe
    530                 535                 540

Cys Phe Val Leu Leu Phe Val Trp Ser Arg Gly Arg Gly Gln His Lys
545                 550                 555                 560

Asn Asn Phe Ser Val Glu Tyr Ser Phe Arg Lys Val Asp Gly Pro Ala
                565                 570                 575

Ala Ala Ala Gly Gln Gly Gly Ala Arg Lys Phe Asn Met Lys Met Ile
            580                 585                 590

<210>  4
<211>  593
<212>  PRT
<213>  Homo sapiens

<400>  4

Met Asp Ala Ala Thr Ala Pro Lys Gln Ala Trp Pro Pro Trp Pro Pro
1               5                   10                  15

Leu Leu Phe Leu Leu Leu Leu Pro Gly Gly Ser Gly Gly Ser Cys Pro
            20                  25                  30

Ala Val Cys Asp Cys Thr Ser Gln Pro Gln Ala Val Leu Cys Gly His
        35                  40                  45

Arg Gln Leu Glu Ala Val Pro Gly Gly Leu Pro Leu Asp Thr Glu Leu
    50                  55                  60

Leu Asp Leu Ser Gly Asn Arg Leu Trp Gly Leu Gln Gln Gly Met Leu
65                  70                  75                  80

Ser Arg Leu Ser Leu Leu Gln Glu Leu Asp Leu Ser Tyr Asn Gln Leu
                85                  90                  95

Ser Thr Leu Glu Pro Gly Ala Phe His Gly Leu Gln Ser Leu Leu Thr
            100                 105                 110

Leu Arg Leu Gln Gly Asn Arg Leu Arg Ile Met Gly Pro Gly Val Phe
        115                 120                 125

Ser Gly Leu Ser Ala Leu Thr Leu Leu Asp Leu Arg Leu Asn Gln Ile
    130                 135                 140

Val Leu Phe Leu Asp Gly Ala Phe Gly Glu Leu Gly Ser Leu Gln Lys
145                 150                 155                 160

Leu Glu Val Gly Asp Asn His Leu Val Phe Val Ala Pro Gly Ala Phe
                165                 170                 175

Ala Gly Leu Ala Lys Leu Ser Thr Leu Thr Leu Glu Arg Cys Asn Leu
            180                 185                 190

Ser Thr Val Pro Gly Leu Ala Leu Ala Arg Leu Pro Ala Leu Val Ala
        195                 200                 205

Leu Arg Leu Arg Glu Leu Asp Ile Gly Arg Leu Pro Ala Gly Ala Leu
    210                 215                 220

Arg Gly Leu Gly Gln Leu Lys Glu Leu Glu Ile His Leu Trp Pro Ser
225                 230                 235                 240

Leu Glu Ala Leu Asp Pro Gly Ser Leu Val Gly Leu Asn Leu Ser Ser
                245                 250                 255

Leu Ala Ile Thr Arg Cys Asn Leu Ser Ser Val Pro Phe Gln Ala Leu
            260                 265                 270

Tyr His Leu Ser Phe Leu Arg Val Leu Asp Leu Ser Gln Asn Pro Ile
        275                 280                 285

Ser Ala Ile Pro Ala Arg Arg Leu Ser Pro Leu Val Arg Leu Gln Glu
    290                 295                 300

Leu Arg Leu Ser Gly Ala Cys Leu Thr Ser Ile Ala Ala His Ala Phe
305                 310                 315                 320

His Gly Leu Thr Ala Phe His Leu Leu Asp Val Ala Asp Asn Ala Leu
                325                 330                 335

Gln Thr Leu Glu Glu Thr Ala Phe Pro Ser Pro Asp Lys Leu Val Thr

            340                 345                 350

Leu Arg Leu Ser Gly Asn Pro Leu Thr Cys Asp Cys Arg Leu Leu Trp
        355                 360                 365

Leu Leu Arg Leu Arg Arg His Leu Asp Phe Gly Met Ser Pro Pro Ala
    370                 375                 380

Cys Ala Gly Pro His His Val Gln Gly Lys Ser Leu Lys Glu Phe Ser
385                 390                 395                 400

Asp Ile Leu Pro Pro Gly His Phe Thr Cys Lys Pro Ala Leu Ile Arg
                405                 410                 415

Lys Ser Gly Pro Arg Trp Val Ile Ala Glu Glu Gly Gly His Ala Val
            420                 425                 430

Phe Ser Cys Ser Gly Asp Gly Asp Pro Ala Pro Thr Val Ser Trp Met
        435                 440                 445

Arg Pro His Gly Ala Trp Leu Gly Arg Ala Gly Arg Val Arg Val Leu
    450                 455                 460

Glu Asp Gly Thr Leu Glu Ile Arg Ser Val Gln Leu Arg Asp Arg Gly
465                 470                 475                 480

Ala Tyr Val Cys Val Val Ser Asn Val Ala Gly Asn Asp Ser Leu Arg
                485                 490                 495

Thr Trp Leu Glu Val Ile Gln Val Glu Pro Pro Asn Gly Thr Leu Ser
            500                 505                 510

Asp Pro Asn Ile Thr Val Pro Gly Ile Pro Gly Pro Phe Phe Leu Asp
        515                 520                 525

Ser Arg Gly Val Ala Met Val Leu Ala Val Gly Phe Leu Pro Phe Leu
    530                 535                 540

Thr Ser Val Thr Leu Cys Phe Gly Leu Ile Ala Leu Trp Ser Lys Gly
545                 550                 555                 560

Lys Gly Arg Val Lys His His Met Thr Phe Asp Phe Val Ala Pro Arg
                565                 570                 575

Pro Ser Gly Asp Lys Asn Ser Gly Gly Asn Arg Val Thr Ala Lys Leu
            580                 585                 590

Phe

<210>  5
<211>  2932
<212>  DNA
<213>  Homo sapiens

<400>  5
ggagagacat gcgattggtg accgagccga gcggaccgaa ggcgcgcccg agatgcaggt      60

gagcaagagg atgctggcgg ggggcgtgag gagcatgccc agccccctcc tggcctgctg     120

gcagcccatc ctcctgctgg tgctgggctc agtgctgtca ggctcggcca cgggctgccc     180

gccccgctgc gagtgctccg cccaggaccg cgctgtgctg tgccaccgca agcgctttgt     240

ggcagtcccc gagggcatcc ccaccgagac gcgcctgctg gacctaggca agaaccgcat     300

caaaacgctc aaccaggacg agttcgccag cttcccgcac ctggaggagc tggagctcaa     360

cgagaacatc gtgagcgccg tggagcccgg cgccttcaac aacctcttca acctccggac     420

gctgggtctc cgcagcaacc gcctgaagct catcccgcta ggcgtcttca ctggcctcag     480

caacctgacc aagctggaca tcagcgagaa caagatcgtt atcctactgg actacatgtt     540

tcaggacctg tacaacctca agtcactgga ggttggcgac aatgacctcg tctacatctc     600

tcaccgcgcc ttcagcggcc tcaacagcct ggagcagctg acgctggaga aatgcaacct     660

gacctccatc cccaccgagg cgctgtccca cctgcacggc ctcatcgtcc tgaggctccg     720

gcacctcaac atcaatgcca tccgggacta ctccttcaag aggctgtacc gactcaaggt     780

cttggagatc tcccactggc cctacttgga caccatgaca cccaactgcc tctacggcct     840

caacctgacg tccctgtcca tcacacactg caatctgacc gctgtgccct acctggccgt     900

ccgccaccta gtctatctcc gcttcctcaa cctctcctac aaccccatca gcaccattga     960

gggctccatg ttgcatgagc tgctccggct gcaggagatc cagctggtgg gcgggcagct    1020

ggccgtggtg gagccctatg ccttccgcgg cctcaactac ctgcgcgtgc tcaatgtctc    1080

tggcaaccag ctgaccacac tggaggaatc agtcttccac tcggtgggca acctggagac    1140

actcatcctg gactccaacc cgctggcctg cgactgtcgg ctcctgtggg tgttccggcg    1200

ccgctggcgg ctcaacttca accggcagca gcccacgtgc gccacgcccg agtttgtcca    1260

gggcaaggag ttcaaggact tccctgatgt gctactgccc aactacttca cctgccgccg    1320

cgcccgcatc cgggaccgca aggcccagca ggtgtttgtg gacgagggcc acacggtgca    1380

gtttgtgtgc cgggccgatg gcgacccgcc gcccgccatc ctctggctct caccccgaaa    1440

gcacctggtc tcagccaaga gcaatgggcg gctcacagtc ttccctgatg gcacgctgga    1500

ggtgcgctac gcccaggtac aggacaacgg cacgtacctg tgcatcgcgg ccaacgcggg    1560

cggcaacgac tccatgcccg cccacctgca tgtgcgcagc tactcgcccg actggcccca    1620

tcagcccaac aagaccttcg ctttcatctc caaccagccg ggcgagggag aggccaacag    1680

cacccgcgcc actgtgcctt tccccttcga catcaagacc ctcatcatcg ccaccaccat    1740

gggcttcatc tctttcctgg gcgtcgtcct cttctgcctg gtgctgctgt ttctctggag 1800

ccggggcaag ggcaacacaa agcacaacat cgagatcgag tatgtgcccc gaaagtcgga 1860

cgcaggcatc agctccgccg acgcgccccg caagttcaac atgaagatga tatgaggccg 1920

gggcgggggg caggaccccc cgggcggccg ggcaggggaa ggggcctggc cgccacctgc 1980

tcactctcca gtccttccca cctcctccct acccttctac acacgttctc tttctccctc 2040

ccgcctccgt cccctgctgc cccccgccag ccctcaccac ctgccctcct tctaccagga 2100

cctcagaagc ccagacctgg ggaccccacc tacacagggg cattgacaga ctggagttga 2160

aagccgacga accgacacgc ggcagagtca ataattcaat aaaaaagtta cgaactttct 2220

ctgtaacttg ggtttcaata attatggatt tttatgaaaa cttgaaataa taaaaagaga 2280

aaaaaactat ttcctatagc tagtcggaat gcaaactttt gacgtcctga ttgctccagg 2340

gccctcttcc aactcagttt cttgtttttc tcttcctcct cctcctcttc ttcctccttt 2400

ctcttctctt ccccagtggg gagggatcac tcaggaaaac aggaaaggag gttccagccc 2460

cacccacctg cccaccccgc cccaggcacc atcaggagca ggctaggggg caggcctggg 2520

cccagctccg ggctggcttt ttgcagggcg caggtggagg ggacaggtct gccgatgggg 2580

gtgggagcct gtctgctggg ctgccaggcg gcaccactgc aaggggtggg agcctggctt 2640

gggtgtggct gagactctgg acagaggctg gggtcctcct gggggacagc acagtcagtg 2700

gagagagcca ggggctggag gtggggccca ccccagcctc tggtcccagc tctgctgctc 2760

acttgctgtg tggcctcaag caggtcactg gcctctctgg gcctcagtct ccacatctgt 2820

acaaatggga acattacccc ctgccctgcc tacctcacag ggctgttgtg aggaattgat 2880

gagatgatgt atgtgaaaca ctttgtaacc tgtaaagcgc tgtgcacacg tg 2932

<210> 6
<211> 2792
<212> DNA
<213> Homo sapiens

<400> 6
aatttagaga agatgtaggg agtgttcaac atgttcgttg tggaagagaa agagctaaga 60

gagaggagct taaagacaca aacgggtaga atcaaggagt gtgctctcaa atgagaggaa 120

caggagtgac attaaccttg aaatgctcgg agactctact ccttcatgac agtaggagga 180

taattaacaa tagatacaaa tgcaggaatt gatgagtgcc atcagaaagc tgtatcatga 240

gctgcctgca cttctaaagt gtccagtgga tttttaatca catgagcctg gaaatagggt 300

tatgaaaaga agctcagagc agagcaccga aagtggccac taccagcatg aagagcccaa 360

caattcaaac tggtgaagtg agaaaaacag aatgcagctt tcaaggttcg tttcaagcag 420

ttggcttgtg ggactctgag agatgctgct gcccatgaca tgcgggaatt atcatgatca 480

actacccagc ttggatttca tccagtggcc aagagctttg tgtgggagac ggcaagggtt 540

ggatttttca aaagagtaaa ccaggataaa tcatgaggaa cctataaccc ttttggccac 600

atgcaaaaaa gcaagacccg tgaccaaggt gtagactaag aagtggagtc atgcttcaca 660

cggccatatc atgctggcag ccattcctgg gtctggctgt ggtgttaatc ttcatgggat 720

ccaccattgg ctgccccgct cgctgtgagt gctctgccca gaacaaatct gttagctgtc 780

acagaaggcg attgatcgcc atcccagagg gcattcccat cgaaaccaaa atcttggacc 840

tcagtaaaaa caggctaaaa agcgtcaacc ctgaagaatt catatcatat cctctgctgg 900

aagagataga cttgagtgac aacatcattg ccaatgtgga accaggagca ttcaacaatc 960

tctttaacct gcgttccctc cgcctaaaag gcaatcgtct aaagctggtc cctttgggag 1020

tattcacggg gctgtccaat ctcactaagc ttgacattag tgagaataag attgtcattt 1080

tactagacta catgttccaa gatctacata acctgaagtc tctagaagtg ggggacaatg 1140

atttggttta tatatcacac agggcattca gtgggcttct tagcttggag cagctcaccc 1200

tggagaaatg caacttaaca gcagtaccaa cagaagccct ctcccacctc cgcagcctca 1260

tcagcctgca tctgaagcat ctcaatatca acaatatgcc tgtgtatgcc tttaaaagat 1320

tgttccacct gaaacaccta gagattgact attggccttt actggatatg atgcctgcca 1380

atagcctcta cggtctcaac ctcacatccc tttcagtcac caacaccaat ctgtctactg 1440

tacccttcct tgcctttaaa cacctggtat acctgactca ccttaacctc tcctacaatc 1500

ccatcagcac tattgaagca ggcatgttct ctgacctgat ccgccttcag gagcttcata 1560

tagtgggggc ccagcttcgc accattgagc ctcactcctt ccaagggctc cgcttcctac 1620

gcgtgctcaa tgtgtctcag aacctgctgg aaactttgga agagaatgtc ttctcctccc 1680

ctagggctct ggaggtcttg agcattaaca acaaccctct ggcctgtgac tgccgccttc 1740

tctggatctt gcagcgacag cccaccctgc agtttggtgg ccagcaacct atgtgtgctg 1800

gcccagacac catccgtgag aggtctttca aggatttcca tagcactgcc ctttcttttt 1860

actttacctg caaaaaaccc aaaatccgtg aaaagaagtt gcagcatctg ctagtagatg 1920

aagggcagac agtccagcta gaatgcagtg cagatggaga cccgcagcct gtgatttcct 1980

gggtgacacc ccgaaggcgt ttcatcacca ccaagtccaa tggaagagcc accgtgttgg 2040

gtgatggcac cttggaaatc cgctttgccc aggatcaaga cagcgggatg tatgtttgca 2100

tcgctagcaa tgctgctggg aatgatacct tcacagcctc cttaactgtg aaaggattcg 2160

cttcagatcg ttttctttat gcgaacagga cccctatgta catgaccgac tccaatgaca 2220

ccatttccaa tggcaccaat gccaatactt tttccctgga ccttaaaaca atactggtgt 2280

ctacagctat gggctgcttc acattcctgg gagtggtttt atttgttttt cttctccttt 2340

ttgtgtggag ccgagggaaa ggcaagcaca aaaacagcat tgaccttgag tatgtgccca 2400

gaaaaaacaa tggtgctgtt gtggaaggag aggtagctgg acccaggagg ttcaacatga 2460

aaatgatttg aaggcccacc cctcacatta ctgtctcttt gtcaatgtgg gtaatcagta 2520

agacagtatg gcacagtaaa ttactagatt aagaggcagc catgtgcagc tgcccctgta 2580

tcaaaagcag ggtctatgga agcaggagga cttccaatgg agactctcca tcgaaaggca 2640

ggcaggcagg catgtgtcag agcccttcac acagtgggat actaagtgtt tgcgttgcaa 2700

atattggcgt tctggggatc tcagtaatga acctgaatat ttggctcaca ctcacggaca 2760

attattcagc attttctacc actgcaaaaa ac 2792

<210> 7
<211> 2130
<212> DNA
<213> Homo sapiens

<400> 7
   ggcggcggcg gcggctgctc gtgtcggctc cgggcggccc ccgcgccgca gcccgcgccg 60
   tgtccgcggc ggagccgccc aggtgcgcag gaggatggtg gcgcggccct aggcccacgc 120
   tccgcaccat gacctgctgg ctgtgcgtcc tgagcctgcc cctgctcctg ctgcccgcgg 180
   cgccgccccc ggctggaggc tgcccggccc gctgcgagtg caccgtgcag acccgcgcgg 240
   tggcctgcac gcgccgccgc ctgaccgccg tgcccgacgg catcccggcc gagacccgcc 300
   tgctggagct cagccgcaac cgcatccgct gcctgaaccc gggcgacctg gccgcgctgc 360
   ccgcgctgga ggagctggac ctgagcgaga acgccatcgc gcacgtggag cccggcgcct 420
   tcgccaacct gccgcgcctg cgcgtcctgc gtctccgtgg caaccagctg aagctcatcc 480
   cgcccggggt cttcacgcgc ctggacaacc tcacgctgct ggacctgagc gagaacaagc 540
   tggtaatcct gctggactac actttccagg acctgcacag cctgcgccgg ctggaagtgg 600
   gcgacaacga cctggtattc gtctcgcgcc gcgccttcgc ggggctgctg gccctggagg 660
   agctgaccct ggagcgctgc aacctcacgg ctctgtccgg ggagtcgctg ggccatctgc 720
   gcagcctggg cgccctgcgg ctgcgccacc tggccatcgc ctccctggag gaccagaact 780
   tccgcaggct gcccgggctg ctgcacctgg agattgacaa ctggccgctg ctggaggagg 840
   tggcggcggg cagcctgcgg ggcctgaacc tgacctcgct gtcggtcacc cacaccaaca 900
   tcaccgccgt gccggccgcc gcgctgcggc accaggcgca cctcacctgc ctcaatctgt 960
   cgcacaaccc catcagcacg gtgccgcggg ggtcgttccg ggacctggtc cgcctgcgcg 1020
   agctgcacct ggccggggcc ctgctggctg tggtggagcc gcaggccttc ctgggcctgc 1080
   gccagatccg cctgctcaac ctctccaaca acctgctctc cacgttggag gagagcacct 1140
   tccactcggt gaacacgcta gagacgctgc gcgtggacgg gaacccgctg gcctgcgact 1200
   gtcgcctgct gtggatcgtg cagcgtcgca agaccctcaa cttcgacggg cggctgccgg 1260
   cctgcgccac ccccgccgag gtgcgcggcg acgcgctgcg aaacctgccg gactccgtgc 1320
   tgttcgagta cttcgtgtgc cgcaaaccca agatccggga gcggcggctg cagcgcgtca 1380
   cggccaccgc gggcgaagac gtccgcttcc tctgccgcgc cgagggcgag ccggcgccca 1440
   ccgtggcctg ggtgaccccc cagcaccggc cggtgacggc caccagcgcg ggccgggcgc 1500
   gcgtgctccc cggggggacg ctggagatcc aggacgcgcg gccgcaggac agcggcacct 1560
   acacgtgcgt ggccagcaac gcgggcggca acgacaccta cttcgccacg ctgaccgtgc 1620
   gccccgagcc ggccgccaac cggaccccgg gcgaggccca caacgagacg ctggcggccc 1680
   tgcgcgcgcc gctcgacctc accaccatcc tggtgtccac cgccatgggc tgcatcacct 1740
   tcctgggcgt ggtcctcttc tgcttcgtgc tgctgttcgt gtggagccgc ggccgcgggc 1800
   agcacaaaaa caacttctcg gtggagtact ccttccgcaa ggtggatggg ccggccgccg 1860
   cggcgggcca gggaggcgcg cgcaagttca acatgaagat gatctgaggg gtccccaggg 1920
   cggaccctcc cctcccctcc cccgcgggcc ggccgctcgc gtgtccacct atgcatttcc 1980
   cggaggggaa ggggacggct gcacggcgct ccccaggcag aacttcccct ttttttgtag 2040
   acgcccaacc gcaggactgt ttttcatcag catgcgtctt ttttgcagtt tctcaagcgt 2100
   tttctaaaga tttcaacccg tcttcctgtc 2130

<210> 8
<211> 2606
<212> DNA
<213> Homo sapiens

<400> 8
gcggccgcag cagcaacagc agcagcagca gcggcaggca gcagccgggc agccaggcag 60

cgggggttga ggcacacagg gaaggtgcag gggcctgagg tgcagctcga atgggacagg 120

gcccccagcg ctggacagat gcagtgccaa acttgatgcc accttccagc ttctccggac 180

tgaagaggga atggatgcag ccacagctcc aaagcaagcc tggcccccat ggcccccgct 240

ccttttcctc ctcctcctac ctggagggag cggtggcagc tgccctgctg tgtgtgactg 300

cacctcccag ccccaggctg tgctctgtgg ccacaggcaa ctggaggctg tacctggagg 360

actcccactg gacactgagc tcctggacct gagtgggaac cgcctgtggg ggctccagca 420

gggaatgctc tcccgcctga gcctgctcca ggaattggac ctcagctaca accagctctc 480

aacccttgag cctggggcct tccatggcct acaaagccta ctcaccctga ggctgcaggg 540

caatcggctc agaatcatgg ggcctggggt cttctcaggc ctctctgctc tgaccctgct 600

ggacctccgc ctcaaccaga ttgttctctt cctagatgga gcttttgggg agctaggcag 660

cctccagaag ctggaggttg gggacaacca cctggtattt gtggctccgg gggcctttgc 720

agggctagcc aagttgagca ccctcaccct ggagcgctgc aacctcagca cagtgcctgg 780

cctagccctt gcccgtctcc cggcactagt ggccctaagg cttagagaac tggatattgg 840

gaggctgcca gctggggccc tgcgggggct ggggcagctc aaggagctgg agatccacct 900

ctggccatct ctggaggctc tggaccctgg gagcctggtt gggctcaatc tcagcagcct 960

ggccatcact cgctgcaatc tgagctcggt gcccttccaa gcactgtacc acctcagctt 1020

cctcagggtc ctggatctgt cccagaatcc catctcagcc atcccagccc gaaggctcag 1080

ccccctggtg cggctccagg agctacgcct gtcaggggca tgcctcacct ccattgctgc 1140

ccatgccttc catggcttga ctgccttcca cctcctggat gtggcagata acgcccttca 1200

gacactagag gaaacagctt tcccttctcc agacaaactg gtcaccttga ggctgtctgg 1260

caacccccta acctgtgact gccgcctcct ctggctgctc cggctccgcc gccacctgga 1320

ctttggcatg tccccccctg cctgtgctgg cccccatcat gtccagggga agagcctgaa 1380

ggagttttca gacatcctgc ctccagggca cttcacctgc aaaccagccc tgatccgaaa 1440

gtcggggcct cgatgggtca ttgcagagga gggcgggcat gcggttttct cctgctctgg 1500

agatggagac ccagccccca ctgtctcctg gatgaggcct catggggctt ggctgggcag 1560

ggctgggaga gtaagggtcc tagaggatgg gacactggag atccgctcag tgcagctacg 1620

ggacagaggg gcctatgtct gtgtggttag caatgtcgct gggaatgact ccctgaggac 1680

ctggctggaa gtcatccagg tggaaccacc aaacggcaca ctttctgacc ccaacatcac 1740

cgtgccaggg atcccagggc ctttttttct ggatagcaga ggtgtggcca tggtgctggc 1800

agtcggcttc ctccccttcc tcacctcagt gaccctctgc tttggcctga ttgccctttg 1860

gagcaagggc aaaggtcggg tcaaacatca catgaccttt gactttgtgg cacctcggcc 1920

ctctggggat aaaaactctg gggtaaccgg ggtcactgcc aagctcttct gacctttcct 1980

tccccagtgg ggaacccacc aagtccgctt cagataccaa aggggaagac agaaccaagg 2040

ctgcttgaac cagaacctag tcccgagcag caccgctctc ctgcacctcc cgcctgcgtt 2100

gtgcctcctg ccggagagtc tgcttcctga gcttttccgg tctgaggata gcattgtcat 2160

ttcttctctg agggtcccag ggagctgcag atgcagaccc cgttgttagt ccagcccccg 2220

cttcaccccc tccacacaca aaacaggaaa cataatcaaa gcgctagtca gctagtctaa 2280

ccactaggct ttcttcacac atgcttatat cctttaataa ccaattgcca accacggcta 2340

taagattatt tcagaggtgg ggctgggaag tgccacttgc tccttagagt ctgtttgtca 2400

accaggcaga gtccctttct tttctgctcc ccaccccaac cctgccccta tgtacaggaa 2460

taagagcaaa ggacccacag gctacagaga agaggatggg gacagagtgt gggatggaga 2520

ggacagacca tatactgcac tgtgtttgca tgagcctcta ccaccttcct ctatctacca 2580

gatcattaaa cctgctgtca aagggc 2606

<210> 9
<211> 720
<212> DNA
<213> synthetic

<400> 9
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcggcta cggcctgcag tgcttcgccc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagct accagtccgc cctgagcaaa gaccccaacg agaagcgcga tcacatggtc 660

ctgctggagt tcgtgaccgc cgccgggatc actctcggca tggacgagct gtacaagtaa 720

<210> 10
<211> 2604
<212> DNA
<213> synthetic

<400> 10
atgcaggtga gcaagaggat gctggcgggg ggcgtgagga gcatgcccag ccccctcctg 60

gcctgctggc agcccatcct cctgctggtg ctgggctcag tgctgtcagg ctcggccacg 120

ggctgcccgc cccgctgcga gtgctccgcc caggaccgcg ctgtgctgtg ccaccgcaag 180

cgctttgtgg cagtccccga gggcatcccc accgagacgc gcctgctgga cctaggcaag 240

aaccgcatca aaacgctcaa ccaggacgag ttcgccagct tcccgcacct ggaggagctg 300

gagctcaacg agaacatcgt gagcgccgtg gagcccggcg ccttcaacaa cctcttcaac 360

ctccggacgc tgggtctccg cagcaaccgc ctgaagctca tcccgctagg cgtcttcact 420

ggcctcagca acctgaccaa gctggacatc agcgagaaca agatcgttat cctactggac 480

tacatgtttc aggacctgta caacctcaag tcactggagg ttggcgacaa tgacctcgtc 540

tacatctctc accgcgcctt cagcggcctc aacagcctgg agcagctgac gctggagaaa 600

tgcaacctga cctccatccc caccgaggcg ctgtcccacc tgcacggcct catcgtcctg 660

aggctccggc acctcaacat caatgccatc cgggactact ccttcaagag gctgtaccga 720

ctcaaggtct tggagatctc ccactggccc tacttggaca ccatgacacc caactgcctc 780

tacggcctca acctgacgtc cctgtccatc acacactgca atctgaccgc tgtgccctac 840

ctggccgtcc gccacctagt ctatctccgc ttcctcaacc tctcctacaa ccccatcagc 900

accattgagg gctccatgtt gcatgagctg ctccggctgc aggagatcca gctggtgggc 960

gggcagctgg ccgtggtgga gccctatgcc ttccgcggcc tcaactacct gcgcgtgctc 1020

aatgtctctg gcaaccagct gaccacactg gaggaatcag tcttccactc ggtgggcaac 1080

ctggagacac tcatcctgga ctccaacccg ctggcctgcg actgtcggct cctgtgggtg 1140

ttccggcgcc gctggcggct caacttcaac cggcagcagc ccacgtgcgc cacgcccgag 1200

tttgtccagg gcaaggagtt caaggacttc cctgatgtgc tactgcccaa ctacttcacc 1260

tgccgccgcg cccgcatccg ggaccgcaag gcccagcagg tgtttgtgga cgagggccac 1320

acggtgcagt ttgtgtgccg ggccgatggc gacccgccgc ccgccatcct ctggctctca 1380

ccccgaaagc acctggtctc agccaagagc aatgggcggc tcacagtctt ccctgatggc 1440

acgctggagg tgcgctacgc ccaggtacag gacaacggca cgtacctgtg catcgcggcc 1500

aacgcgggcg gcaacgactc catgcccgcc cacctgcatg tgcgcagcta ctcgcccgac 1560

tggccccatc agcccaacaa gaccttcgct ttcatctcca accagccggg cgagggagag 1620

gccaacagca cccgcgccac tgtgcctttc cccttcgaca tcaagaccct catcatcgcc 1680

accaccatgg gcttcatctc tttcctgggc gtcgtcctct tctgcctggt gctgctgttt 1740

ctctggagcc ggggcaaggg caacacaaag cacaacatcg agatcgagta tgtgccccga 1800

aagtcggacg caggcatcag ctccgccgac gcgccccgca agttcaacat gaagatgata 1860

tcacgggatc caccggtcgc caccatggtg agcaagggcg aggagctgtt caccggggtg 1920

gtgcccatcc tggtcgagct ggacggcgac gtaaacggcc acaagttcag cgtgtccggc 1980

gagggcgagg gcgatgccac ctacggcaag ctgaccctga agttcatctg caccaccggc 2040

aagctgcccg tgccctggcc caccctcgtg accaccttcg gctacggcct gcagtgcttc 2100

gcccgctacc ccgaccacat gaagcagcac gacttcttca agtccgccat gcccgaaggc 2160

tacgtccagg agcgcaccat cttcttcaag gacgacggca actacaagac ccgcgccgag 2220

gtgaagttcg agggcgacac cctggtgaac cgcatcgagc tgaagggcat cgacttcaag 2280

gaggacggca acatcctggg gcacaagctg gagtacaact acaacagcca caacgtctat 2340

atcatggccg acaagcagaa gaacggcatc aaggtgaact tcaagatccg ccacaacatc 2400

gaggacggca gcgtgcagct cgccgaccac taccagcaga acacccccat cggcgacggc 2460

cccgtgctgc tgcccgacaa ccactacctg agctaccagt ccgccctgag caaagacccc 2520

aacgagaagc gcgatcacat ggtcctgctg gagttcgtga ccgccgccgg gatcactctc 2580

ggcatggacg agctgtacaa gtaa 2604

<210> 11
<211> 867
<212> PRT
<213> synthetic

<400> 11

Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly

            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg
    370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile Ser Arg Asp Pro
    610                 615                 620

Pro Val Ala Thr Met Val Ser Lys Gly Glu Glu Leu Phe Thr Gly Val
625                 630                 635                 640

Val Pro Ile Leu Val Glu Leu Asp Gly Asp Val Asn Gly His Lys Phe
                645                 650                 655

Ser Val Ser Gly Glu Gly Glu Gly Asp Ala Thr Tyr Gly Lys Leu Thr
            660                 665                 670

Leu Lys Phe Ile Cys Thr Thr Gly Lys Leu Pro Val Pro Trp Pro Thr
        675                 680                 685

Leu Val Thr Thr Phe Gly Tyr Gly Leu Gln Cys Phe Ala Arg Tyr Pro
    690                 695                 700

Asp His Met Lys Gln His Asp Phe Phe Lys Ser Ala Met Pro Glu Gly
705                 710                 715                 720

Tyr Val Gln Glu Arg Thr Ile Phe Phe Lys Asp Asp Gly Asn Tyr Lys
                725                 730                 735

Thr Arg Ala Glu Val Lys Phe Glu Gly Asp Thr Leu Val Asn Arg Ile
            740                 745                 750

Glu Leu Lys Gly Ile Asp Phe Lys Glu Asp Gly Asn Ile Leu Gly His
        755                 760                 765

Lys Leu Glu Tyr Asn Tyr Asn Ser His Asn Val Tyr Ile Met Ala Asp
    770                 775                 780

Lys Gln Lys Asn Gly Ile Lys Val Asn Phe Lys Ile Arg His Asn Ile
785                 790                 795                 800

Glu Asp Gly Ser Val Gln Leu Ala Asp His Tyr Gln Gln Asn Thr Pro
                805                 810                 815

Ile Gly Asp Gly Pro Val Leu Leu Pro Asp Asn His Tyr Leu Ser Tyr
            820                 825                 830

Gln Ser Ala Leu Ser Lys Asp Pro Asn Glu Lys Arg Asp His Met Val
        835                 840                 845

Leu Leu Glu Phe Val Thr Ala Ala Gly Ile Thr Leu Gly Met Asp Glu
    850                 855                 860

Leu Tyr Lys
865

<210>  12
<211>  936
<212>  DNA
<213>  synthetic

<400>  12
atgaccagca aggtgtacga ccccgagcag aggaagagga tgatcaccgg cccccagtgg      60

tgggccaggt gcaagcagat gaacgtgctg gacagcttca tcaactacta cgacagcgag     120

aagcacgccg agaacgccgt gatcttcctg cacggcaacg ccgctagtag ctacctgtgg     180

aggcacgtgg tgccccacat cgagcccgtg gccaggtgca tcatccccga tctgatcggc     240

atgggcaaga gcggcaagag cggcaacggc agctacaggc tgctggacca ctacaagtac     300

ctgaccgcct ggttcgagct cctgaacctg cccaagaaga tcatcttcgt gggccacgac     360

tggggcgcct gcctggcctt ccactacagc tacgagcacc aggacaagat caaggccatc     420

gtgcacgccg agagcgtggt ggacgtgatc gagagctggg acgagtggcc agacatcgag     480

gaggacatcg ccctgatcaa gagcgaggag ggcgagaaga tggtgctgga gaacaacttc     540

ttcgtggaga ccatgctgcc cagcaagatc atgagaaagc tggagcccga ggagttcgcc     600

gcctacctgg agcccttcaa ggagaagggc gaggtgagaa gacccaccct gagctggccc     660

agagagatcc ccctggtgaa gggcggcaag cccgacgtgg tgcagatcgt gagaaactac     720

aacgcctacc tgagagccag cgacgacctg cccaagatgt tcatcgagag cgaccccggc     780

ttcttcagca acgccatcgt ggagggcgcc aagaagttcc ccaacaccga gttcgtgaag     840

gtgaagggcc tgcacttcag ccaggaggac gcccccgacg agatgggcaa gtacatcaag     900

agcttcgtgg agagagtgct gaagaacgag cagtaa                               936

<210>  13
<211>  2820
<212>  DNA
<213>  synthetic

<400>  13

atgcaggtga gcaagaggat gctggcgggg ggcgtgagga gcatgcccag ccccctcctg 60

gcctgctggc agcccatcct cctgctggtg ctgggctcag tgctgtcagg ctcggccacg 120

ggctgcccgc cccgctgcga gtgctccgcc caggaccgcg ctgtgctgtg ccaccgcaag 180

cgctttgtgg cagtccccga gggcatcccc accgagacgc gcctgctgga cctaggcaag 240

aaccgcatca aaacgctcaa ccaggacgag ttcgccagct tcccgcacct ggaggagctg 300

gagctcaacg agaacatcgt gagcgccgtg gagcccggcg ccttcaacaa cctcttcaac 360

ctccggacgc tgggtctccg cagcaaccgc ctgaagctca tcccgctagg cgtcttcact 420

ggcctcagca acctgaccaa gctggacatc agcgagaaca agatcgttat cctactggac 480

tacatgtttc aggacctgta caacctcaag tcactggagg ttggcgacaa tgacctcgtc 540

tacatctctc accgcgcctt cagcggcctc aacagcctgg agcagctgac gctggagaaa 600

tgcaacctga cctccatccc caccgaggcg ctgtcccacc tgcacggcct catcgtcctg 660

aggctccggc acctcaacat caatgccatc cgggactact ccttcaagag gctgtaccga 720

ctcaaggtct tggagatctc ccactggccc tacttggaca ccatgacacc caactgcctc 780

tacggcctca acctgacgtc cctgtccatc acacactgca atctgaccgc tgtgccctac 840

ctggccgtcc gccacctagt ctatctccgc ttcctcaacc tctcctacaa ccccatcagc 900

accattgagg gctccatgtt gcatgagctg ctccggctgc aggagatcca gctggtgggc 960

gggcagctgg ccgtggtgga gccctatgcc ttccgcggcc tcaactacct gcgcgtgctc 1020

aatgtctctg gcaaccagct gaccacactg gaggaatcag tcttccactc ggtgggcaac 1080

ctggagacac tcatcctgga ctccaacccg ctggcctgcg actgtcggct cctgtgggtg 1140

ttccggcgcc gctggcggct caacttcaac cggcagcagc ccacgtgcgc cacgcccgag 1200

tttgtccagg gcaaggagtt caaggacttc cctgatgtgc tactgcccaa ctacttcacc 1260

tgccgccgcg cccgcatccg ggaccgcaag gcccagcagg tgtttgtgga cgagggccac 1320

acggtgcagt ttgtgtgccg ggccgatggc gacccgccgc ccgccatcct ctggctctca 1380

ccccgaaagc acctggtctc agccaagagc aatgggcggc tcacagtctt ccctgatggc 1440

acgctggagg tgcgctacgc ccaggtacag gacaacggca cgtacctgtg catcgcggcc 1500

aacgcgggcg gcaacgactc catgcccgcc cacctgcatg tgcgcagcta ctcgcccgac 1560

tggccccatc agcccaacaa gaccttcgct ttcatctcca accagccggg cgagggagag 1620

gccaacagca cccgcgccac tgtgcctttc cccttcgaca tcaagaccct catcatcgcc 1680

accaccatgg gcttcatctc tttcctgggc gtcgtcctct tctgcctggt gctgctgttt 1740

ctctggagcc ggggcaaggg caacacaaag cacaacatcg agatcgagta tgtgccccga 1800

aagtcggacg caggcatcag ctccgccgac gcgccccgca agttcaacat gaagatgata 1860

tcacgggatc caccggtagc aaccatgacc agcaaggtgt acgaccccga gcagaggaag 1920

aggatgatca ccggccccca gtggtgggcc aggtgcaagc agatgaacgt gctggacagc 1980

ttcatcaact actacgacag cgagaagcac gccgagaacg ccgtgatctt cctgcacggc 2040

aacgccgcta gtagctacct gtggaggcac gtggtgcccc acatcgagcc cgtggccagg 2100

tgcatcatcc ccgatctgat cggcatgggc aagagcggca agagcggcaa cggcagctac 2160

aggctgctgg accactacaa gtacctgacc gcctggttcg agctcctgaa cctgcccaag 2220

aagatcatct tcgtgggcca cgactggggc gcctgcctgg ccttccacta cagctacgag 2280

caccaggaca agatcaaggc catcgtgcac gccgagagcg tggtggacgt gatcgagagc 2340

tgggacgagt ggccagacat cgaggaggac atcgccctga tcaagagcga ggagggcgag 2400

aagatggtgc tggagaacaa cttcttcgtg gagaccatgc tgcccagcaa gatcatgaga 2460

aagctggagc ccgaggagtt cgccgcctac ctggagccct tcaaggagaa gggcgaggtg 2520

agaagaccca ccctgagctg gcccagagag atccccctgg tgaagggcgg caagcccgac 2580

gtggtgcaga tcgtgagaaa ctacaacgcc tacctgagag ccagcgacga cctgcccaag 2640

atgttcatcg agagcgaccc cggcttcttc agcaacgcca tcgtggaggg cgccaagaag 2700

ttccccaaca ccgagttcgt gaaggtgaag ggcctgcact tcagccagga ggacgccccc 2760

gacgagatgg gcaagtacat caagagcttc gtggagagag tgctgaagaa cgagcagtaa 2820

<210> 14
<211> 939
<212> PRT
<213> synthetic

<400> 14

Met Gln Val Ser Lys Arg Met Leu Ala Gly Gly Val Arg Ser Met Pro
1               5                   10                  15

Ser Pro Leu Leu Ala Cys Trp Gln Pro Ile Leu Leu Leu Val Leu Gly
            20                  25                  30

Ser Val Leu Ser Gly Ser Ala Thr Gly Cys Pro Pro Arg Cys Glu Cys
        35                  40                  45

Ser Ala Gln Asp Arg Ala Val Leu Cys His Arg Lys Arg Phe Val Ala
    50                  55                  60

Val Pro Glu Gly Ile Pro Thr Glu Thr Arg Leu Leu Asp Leu Gly Lys
65                  70                  75                  80

Asn Arg Ile Lys Thr Leu Asn Gln Asp Glu Phe Ala Ser Phe Pro His
                85                  90                  95

Leu Glu Glu Leu Glu Leu Asn Glu Asn Ile Val Ser Ala Val Glu Pro
            100                 105                 110

Gly Ala Phe Asn Asn Leu Phe Asn Leu Arg Thr Leu Gly Leu Arg Ser
        115                 120                 125

Asn Arg Leu Lys Leu Ile Pro Leu Gly Val Phe Thr Gly Leu Ser Asn
    130                 135                 140

Leu Thr Lys Leu Asp Ile Ser Glu Asn Lys Ile Val Ile Leu Leu Asp
145                 150                 155                 160

Tyr Met Phe Gln Asp Leu Tyr Asn Leu Lys Ser Leu Glu Val Gly Asp
                165                 170                 175

Asn Asp Leu Val Tyr Ile Ser His Arg Ala Phe Ser Gly Leu Asn Ser
            180                 185                 190

Leu Glu Gln Leu Thr Leu Glu Lys Cys Asn Leu Thr Ser Ile Pro Thr
        195                 200                 205

Glu Ala Leu Ser His Leu His Gly Leu Ile Val Leu Arg Leu Arg His
    210                 215                 220

Leu Asn Ile Asn Ala Ile Arg Asp Tyr Ser Phe Lys Arg Leu Tyr Arg
225                 230                 235                 240

Leu Lys Val Leu Glu Ile Ser His Trp Pro Tyr Leu Asp Thr Met Thr
                245                 250                 255

Pro Asn Cys Leu Tyr Gly Leu Asn Leu Thr Ser Leu Ser Ile Thr His
            260                 265                 270

Cys Asn Leu Thr Ala Val Pro Tyr Leu Ala Val Arg His Leu Val Tyr
        275                 280                 285

Leu Arg Phe Leu Asn Leu Ser Tyr Asn Pro Ile Ser Thr Ile Glu Gly
    290                 295                 300

Ser Met Leu His Glu Leu Leu Arg Leu Gln Glu Ile Gln Leu Val Gly
305                 310                 315                 320

Gly Gln Leu Ala Val Val Glu Pro Tyr Ala Phe Arg Gly Leu Asn Tyr
                325                 330                 335

Leu Arg Val Leu Asn Val Ser Gly Asn Gln Leu Thr Thr Leu Glu Glu
            340                 345                 350

Ser Val Phe His Ser Val Gly Asn Leu Glu Thr Leu Ile Leu Asp Ser
        355                 360                 365

Asn Pro Leu Ala Cys Asp Cys Arg Leu Leu Trp Val Phe Arg Arg Arg

370                 375                 380

Trp Arg Leu Asn Phe Asn Arg Gln Gln Pro Thr Cys Ala Thr Pro Glu
385                 390                 395                 400

Phe Val Gln Gly Lys Glu Phe Lys Asp Phe Pro Asp Val Leu Leu Pro
                405                 410                 415

Asn Tyr Phe Thr Cys Arg Arg Ala Arg Ile Arg Asp Arg Lys Ala Gln
            420                 425                 430

Gln Val Phe Val Asp Glu Gly His Thr Val Gln Phe Val Cys Arg Ala
        435                 440                 445

Asp Gly Asp Pro Pro Pro Ala Ile Leu Trp Leu Ser Pro Arg Lys His
    450                 455                 460

Leu Val Ser Ala Lys Ser Asn Gly Arg Leu Thr Val Phe Pro Asp Gly
465                 470                 475                 480

Thr Leu Glu Val Arg Tyr Ala Gln Val Gln Asp Asn Gly Thr Tyr Leu
                485                 490                 495

Cys Ile Ala Ala Asn Ala Gly Gly Asn Asp Ser Met Pro Ala His Leu
            500                 505                 510

His Val Arg Ser Tyr Ser Pro Asp Trp Pro His Gln Pro Asn Lys Thr
        515                 520                 525

Phe Ala Phe Ile Ser Asn Gln Pro Gly Glu Gly Glu Ala Asn Ser Thr
    530                 535                 540

Arg Ala Thr Val Pro Phe Pro Phe Asp Ile Lys Thr Leu Ile Ile Ala
545                 550                 555                 560

Thr Thr Met Gly Phe Ile Ser Phe Leu Gly Val Val Leu Phe Cys Leu
                565                 570                 575

Val Leu Leu Phe Leu Trp Ser Arg Gly Lys Gly Asn Thr Lys His Asn
            580                 585                 590

Ile Glu Ile Glu Tyr Val Pro Arg Lys Ser Asp Ala Gly Ile Ser Ser
        595                 600                 605

Ala Asp Ala Pro Arg Lys Phe Asn Met Lys Met Ile Ser Arg Asp Pro
    610                 615                 620

Pro Val Ala Thr Met Thr Ser Lys Val Tyr Asp Pro Glu Gln Arg Lys
625                 630                 635                 640

Arg Met Ile Thr Gly Pro Gln Trp Trp Ala Arg Cys Lys Gln Met Asn
                645                 650                 655

Val Leu Asp Ser Phe Ile Asn Tyr Tyr Asp Ser Glu Lys His Ala Glu
            660                 665                 670

Asn Ala Val Ile Phe Leu His Gly Asn Ala Ala Ser Ser Tyr Leu Trp
        675                 680                 685

Arg His Val Val Pro His Ile Glu Pro Val Ala Arg Cys Ile Ile Pro
    690                 695                 700

Asp Leu Ile Gly Met Gly Lys Ser Gly Lys Ser Gly Asn Gly Ser Tyr
705                 710                 715                 720

Arg Leu Leu Asp His Tyr Lys Tyr Leu Thr Ala Trp Phe Glu Leu Leu
                725                 730                 735

Asn Leu Pro Lys Lys Ile Ile Phe Val Gly His Asp Trp Gly Ala Cys
            740                 745                 750

Leu Ala Phe His Tyr Ser Tyr Glu His Gln Asp Lys Ile Lys Ala Ile
        755                 760                 765

Val His Ala Glu Ser Val Val Asp Val Ile Glu Ser Trp Asp Glu Trp
    770                 775                 780

Pro Asp Ile Glu Glu Asp Ile Ala Leu Ile Lys Ser Glu Glu Gly Glu
785                 790                 795                 800

Lys Met Val Leu Glu Asn Asn Phe Phe Val Glu Thr Met Leu Pro Ser
                805                 810                 815

Lys Ile Met Arg Lys Leu Glu Pro Glu Glu Phe Ala Ala Tyr Leu Glu
            820                 825                 830

Pro Phe Lys Glu Lys Gly Glu Val Arg Arg Pro Thr Leu Ser Trp Pro
        835                 840                 845

Arg Glu Ile Pro Leu Val Lys Gly Gly Lys Pro Asp Val Val Gln Ile
    850                 855                 860

Val Arg Asn Tyr Asn Ala Tyr Leu Arg Ala Ser Asp Asp Leu Pro Lys
865                 870                 875                 880

Met Phe Ile Glu Ser Asp Pro Gly Phe Phe Ser Asn Ala Ile Val Glu
                885                 890                 895

Gly Ala Lys Lys Phe Pro Asn Thr Glu Phe Val Lys Val Lys Gly Leu
            900                 905                 910

His Phe Ser Gln Glu Asp Ala Pro Asp Glu Met Gly Lys Tyr Ile Lys
        915                 920                 925

Ser Phe Val Glu Arg Val Leu Lys Asn Glu Gln
    930                 935

<210>  15
<211>  4960
<212>  DNA
<213>  synthetic

<400>  15
tagttattaa tagtaatcaa ttacggggtc attagttcat agcccatata tggagttccg      60

cgttacataa cttacggtaa atggcccgcc tggctgaccg cccaacgacc cccgcccatt     120

gacgtcaata atgacgtatg ttcccatagt aacgccaata gggactttcc attgacgtca     180

atgggtggag tatttacggt aaactgccca cttggcagta catcaagtgt atcatatgcc     240

aagtacgccc cctattgacg tcaatgacgg taaatggccc gcctggcatt atgcccagta     300

catgacctta tgggactttc ctacttggca gtacatctac gtattagtca tcgctattac     360

catggtgatg cggttttggc agtacatcaa tgggcgtgga tagcggtttg actcacgggg     420

atttccaagt ctccacccca ttgacgtcaa tgggagtttg ttttggcacc aaaatcaacg     480

ggactttcca aaatgtcgta acaactccgc cccattgacg caaatgggcg gtaggcgtgt     540

acggtgggag gtctatataa gcagagctgg tttagtgaac cgtcagatcc gctagcgcta     600

ccggactcag atctcgagct caagcttcga attctgcagt cgacggtacc gcgggcccgg     660

gatccaccgg tagcaaccat gaccagcaag gtgtacgacc ccgagcagag gaagaggatg     720

atcaccggcc cccagtggtg ggccaggtgc aagcagatga acgtgctgga cagcttcatc     780

aactactacg acagcgagaa gcacgccgag aacgccgtga tcttcctgca cggcaacgcc     840

gctagtagct acctgtggag gcacgtggtg ccccacatcg agcccgtggc caggtgcatc     900

atccccgatc tgatcggcat gggcaagagc ggcaagagcg gcaacggcag ctacaggctg     960

ctggaccact acaagtacct gaccgcctgg ttcgagctcc tgaacctgcc caagaagatc    1020

atcttcgtgg gccacgactg gggcgcctgc ctggccttcc actacagcta cgagcaccag    1080

gacaagatca aggccatcgt gcacgccgag agcgtggtgg acgtgatcga gagctgggac    1140

gagtggccag acatcgagga ggacatcgcc ctgatcaaga gcgaggaggg cgagaagatg    1200

gtgctggaga acaacttctt cgtggagacc atgctgccca gcaagatcat gagaaagctg    1260

gagcccgagg agttcgccgc ctacctggag cccttcaagg agaagggcga ggtgagaaga    1320

cccaccctga gctggcccag agagatcccc ctggtgaagg gcggcaagcc cgacgtggtg    1380

cagatcgtga gaaactacaa cgcctacctg agagccagcg acgacctgcc caagatgttc 1440

atcgagagcg accccggctt cttcagcaac gccatcgtgg agggcgccaa gaagttcccc 1500

aacaccgagt tcgtgaaggt gaagggcctg cacttcagcc aggaggacgc ccccgacgag 1560

atgggcaagt acatcaagag cttcgtggag agagtgctga agaacgagca gtaatgtaca 1620

agtaaagcgg ccgcgactct agatcataat cagccatacc acatttgtag aggttttact 1680

tgctttaaaa aacctcccac acctccccct gaacctgaaa cataaaatga atgcaattgt 1740

tgttgttaac ttgtttattg cagcttataa tggttacaaa taaagcaata gcatcacaaa 1800

tttcacaaat aaagcatttt tttcactgca ttctagttgt ggtttgtcca aactcatcaa 1860

tgtatcttaa ggcgtaaatt gtaagcgtta atattttgtt aaaattcgcg ttaaattttt 1920

gttaaatcag ctcatttttt aaccaatagg ccgaaatcgg caaaatccct tataaatcaa 1980

aagaatagac cgagataggg ttgagtgttg ttccagtttg gaacaagagt ccactattaa 2040

agaacgtgga ctccaacgtc aaagggcgaa aaaccgtcta tcagggcgat ggcccactac 2100

gtgaaccatc accctaatca agttttttgg ggtcgaggtg ccgtaaagca ctaaatcgga 2160

accctaaagg gagcccccga tttagagctt gacggggaaa gccggcgaac gtggcgagaa 2220

aggaagggaa gaaagcgaaa ggagcgggcg ctagggcgct ggcaagtgta gcggtcacgc 2280

tgcgcgtaac caccacaccc gccgcgctta atgcgccgct acagggcgcg tcaggtggca 2340

cttttcgggg aaatgtgcgc ggaaccccta tttgtttatt tttctaaata cattcaaata 2400

tgtatccgct catgagacaa taaccctgat aaatgcttca ataatattga aaaaggaaga 2460

gtcctgaggc ggaaagaacc agctgtggaa tgtgtgtcag ttagggtgtg gaaagtcccc 2520

aggctcccca gcaggcagaa gtatgcaaag catgcatctc aattagtcag caaccaggtg 2580

tggaaagtcc ccaggctccc cagcaggcag aagtatgcaa agcatgcatc tcaattagtc 2640

agcaaccata gtcccgcccc taactccgcc catcccgccc ctaactccgc ccagttccgc 2700

ccattctccg ccccatggct gactaatttt ttttatttat gcagaggccg aggccgcctc 2760

ggcctctgag ctattccaga agtagtgagg aggctttttt ggaggcctag gcttttgcaa 2820

agatcgatca agagacagga tgaggatcgt ttcgcatgat tgaacaagat ggattgcacg 2880

caggttctcc ggccgcttgg gtggagaggc tattcggcta tgactgggca caacagacaa 2940

tcggctgctc tgatgccgcc gtgttccggc tgtcagcgca ggggcgcccg gttctttttg 3000

tcaagaccga cctgtccggt gccctgaatg aactgcaaga cgaggcagcg cggctatcgt 3060

ggctggccac gacgggcgtt ccttgcgcag ctgtgctcga cgttgtcact gaagcgggaa 3120

gggactggct gctattgggc gaagtgccgg ggcaggatct cctgtcatct caccttgctc 3180

ctgccgagaa agtatccatc atggctgatg caatgcggcg gctgcatacg cttgatccgg 3240

ctacctgccc attcgaccac caagcgaaac atcgcatcga gcgagcacgt actcggatgg 3300

aagccggtct tgtcgatcag gatgatctgg acgaagagca tcaggggctc gcgccagccg 3360

aactgttcgc caggctcaag gcgagcatgc ccgacggcga ggatctcgtc gtgacccatg 3420

gcgatgcctg cttgccgaat atcatggtgg aaaatggccg cttttctgga ttcatcgact 3480

gtggccggct gggtgtggcg gaccgctatc aggacatagc gttggctacc cgtgatattg 3540

ctgaagagct tggcggcgaa tgggctgacc gcttcctcgt gctttacggt atcgccgctc 3600

ccgattcgca gcgcatcgcc ttctatcgcc ttcttgacga gttcttctga gcgggactct 3660

ggggttcgaa atgaccgacc aagcgacgcc caacctgcca tcacgagatt tcgattccac 3720

cgccgccttc tatgaaaggt tgggcttcgg aatcgttttc cgggacgccg gctggatgat 3780

cctccagcgc ggggatctca tgctggagtt cttcgcccac cctaggggga ggctaactga 3840

aacacggaag gagacaatac cggaaggaac ccgcgctatg acggcaataa aaagacagaa 3900

taaaacgcac ggtgttgggt cgtttgttca taaacgcggg gttcggtccc agggctggca 3960

ctctgtcgat accccaccga gaccccattg gggccaatac gcccgcgttt cttccttttc 4020

cccaccccac cccccaagtt cgggtgaagg cccagggctc gcagccaacg tcggggcggc 4080

aggccctgcc atagcctcag gttactcata tatactttag attgatttaa aacttcattt 4140

ttaatttaaa aggatctagg tgaagatcct ttttgataat ctcatgacca aaatccctta 4200

acgtgagttt tcgttccact gagcgtcaga ccccgtagaa aagatcaaag gatcttcttg 4260

agatcctttt tttctgcgcg taatctgctg cttgcaaaca aaaaaaccac cgctaccagc 4320

ggtggtttgt ttgccggatc aagagctacc aactcttttt ccgaaggtaa ctggcttcag 4380

cagagcgcag ataccaaata ctgtccttct agtgtagccg tagttaggcc accacttcaa 4440

gaactctgta gcaccgccta catacctcgc tctgctaatc ctgttaccag tggctgctgc 4500

cagtggcgat aagtcgtgtc ttaccgggtt ggactcaaga cgatagttac cggataaggc 4560

gcagcggtcg ggctgaacgg ggggttcgtg cacacagccc agcttggagc gaacgaccta 4620

caccgaactg agatacctac agcgtgagct atgagaaagc gccacgcttc ccgaagggag 4680

aaaggcggac aggtatccgg taagcggcag ggtcggaaca ggagagcgca cgagggagct 4740

tccaggggga aacgcctggt atctttatag tcctgtcggg tttcgccacc tctgacttga 4800

gcgtcgattt ttgtgatgct cgtcaggggg gcggagccta tggaaaaacg ccagcaacgc 4860

ggccttttta cggttcctgg ccttttgctg gccttttgct cacatgttct ttcctgcgtt 4920

atcccctgat tctgtggata accgtattac cgccatgcat 4960

<210> 16
<211> 4733
<212> DNA
<213> synthetic

<400> 16
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660

gatccaccgg tcgccaccat ggtgagcaag ggcgaggagc tgttcaccgg ggtggtgccc 720

atcctggtcg agctggacgg cgacgtaaac ggccacaagt tcagcgtgtc cggcgagggc 780

gagggcgatg ccacctacgg caagctgacc ctgaagttca tctgcaccac cggcaagctg 840

cccgtgccct ggcccaccct cgtgaccacc ttcggctacg gcctgcagtg cttcgcccgc 900

taccccgacc acatgaagca gcacgacttc ttcaagtccg ccatgcccga aggctacgtc 960

caggagcgca ccatcttctt caaggacgac ggcaactaca agacccgcgc cgaggtgaag 1020

ttcgagggcg acaccctggt gaaccgcatc gagctgaagg gcatcgactt caaggaggac 1080

ggcaacatcc tggggcacaa gctggagtac aactacaaca gccacaacgt ctatatcatg 1140

gccgacaagc agaagaacgg catcaaggtg aacttcaaga tccgccacaa catcgaggac 1200

ggcagcgtgc agctcgccga ccactaccag cagaacaccc ccatcggcga cggccccgtg 1260

ctgctgcccg acaaccacta cctgagctac cagtccgccc tgagcaaaga ccccaacgag 1320

aagcgcgatc acatggtcct gctggagttc gtgaccgccg ccgggatcac tctcggcatg 1380

gacgagctgt acaagtaaag cggccgcgac tctagatcat aatcagccat accacatttg 1440

tagaggtttt acttgcttta aaaaacctcc cacacctccc cctgaacctg aaacataaaa 1500

tgaatgcaat tgttgttgtt aacttgttta ttgcagctta taatggttac aaataaagca 1560

atagcatcac aaatttcaca aataaagcat ttttttcact gcattctagt tgtggtttgt 1620

ccaaactcat caatgtatct taaggcgtaa attgtaagcg ttaatatttt gttaaaattc 1680

gcgttaaatt tttgttaaat cagctcattt tttaaccaat aggccgaaat cggcaaaatc 1740

ccttataaat caaaagaata gaccgagata gggttgagtg ttgttccagt ttggaacaag 1800

agtccactat taaagaacgt ggactccaac gtcaaagggc gaaaaaccgt ctatcagggc 1860

gatggcccac tacgtgaacc atcaccctaa tcaagttttt tggggtcgag gtgccgtaaa 1920

gcactaaatc ggaaccctaa agggagcccc cgatttagag cttgacgggg aaagccggcg 1980

aacgtggcga gaaaggaagg gaagaaagcg aaaggagcgg gcgctagggc gctggcaagt 2040

gtagcggtca cgctgcgcgt aaccaccaca cccgccgcgc ttaatgcgcc gctacagggc 2100

gcgtcaggtg gcacttttcg gggaaatgtg cgcggaaccc ctatttgttt atttttctaa 2160

atacattcaa atatgtatcc gctcatgaga caataaccct gataaatgct tcaataatat 2220

tgaaaaagga agagtcctga ggcggaaaga accagctgtg gaatgtgtgt cagttagggt 2280

gtggaaagtc cccaggctcc ccagcaggca gaagtatgca aagcatgcat ctcaattagt 2340

cagcaaccag gtgtggaaag tccccaggct ccccagcagg cagaagtatg caaagcatgc 2400

atctcaatta gtcagcaacc atagtcccgc ccctaactcc gcccatcccg cccctaactc 2460

cgcccagttc cgcccattct ccgccccatg gctgactaat tttttttatt tatgcagagg 2520

ccgaggccgc ctcggcctct gagctattcc agaagtagtg aggaggcttt tttggaggcc 2580

taggcttttg caaagatcga tcaagagaca ggatgaggat cgtttcgcat gattgaacaa 2640

gatggattgc acgcaggttc tccggccgct tgggtggaga ggctattcgg ctatgactgg 2700

gcacaacaga caatcggctg ctctgatgcc gccgtgttcc ggctgtcagc gcaggggcgc 2760

ccggttcttt ttgtcaagac cgacctgtcc ggtgccctga atgaactgca agacgaggca 2820

gcgcggctat cgtggctggc cacgacgggc gttccttgcg cagctgtgct cgacgttgtc 2880

actgaagcgg gaagggactg gctgctattg ggcgaagtgc cggggcagga tctcctgtca 2940

tctcaccttg ctcctgccga gaaagtatcc atcatggctg atgcaatgcg gcggctgcat 3000

acgcttgatc cggctacctg cccattcgac caccaagcga aacatcgcat cgagcgagca 3060

cgtactcgga tggaagccgg tcttgtcgat caggatgatc tggacgaaga gcatcagggg 3120

ctcgcgccag ccgaactgtt cgccaggctc aaggcgagca tgcccgacgg cgaggatctc 3180

gtcgtgaccc atggcgatgc ctgcttgccg aatatcatgg tggaaaatgg ccgcttttct 3240

ggattcatcg actgtggccg gctgggtgtg gcggaccgct atcaggacat agcgttggct 3300

acccgtgata ttgctgaaga gcttggcggc gaatgggctg accgcttcct cgtgctttac 3360

ggtatcgccg ctcccgattc gcagcgcatc gccttctatc gccttcttga cgagttcttc 3420

tgagcgggac tctggggttc gaaatgaccg accaagcgac gcccaacctg ccatcacgag 3480

atttcgattc caccgccgcc ttctatgaaa ggttgggctt cggaatcgtt ttccgggacg 3540

ccggctggat gatcctccag cgcggggatc tcatgctgga gttcttcgcc caccctaggg 3600

ggaggctaac tgaaacacgg aaggagacaa taccggaagg aacccgcgct atgacggcaa 3660

taaaaagaca gaataaaacg cacggtgttg ggtcgtttgt tcataaacgc ggggttcggt 3720

cccagggctg gcactctgtc gataccccac cgagacccca ttggggccaa tacgcccgcg 3780

tttcttcctt ttccccaccc caccccccaa gttcgggtga aggcccaggg ctcgcagcca 3840

acgtcggggc ggcaggccct gccatagcct caggttactc atatatactt tagattgatt 3900

taaaacttca tttttaattt aaaaggatct aggtgaagat cctttttgat aatctcatga 3960

ccaaaatccc ttaacgtgag ttttcgttcc actgagcgtc agaccccgta gaaaagatca 4020

aaggatcttc ttgagatcct ttttttctgc gcgtaatctg ctgcttgcaa acaaaaaaac 4080

caccgctacc agcggtggtt tgtttgccgg atcaagagct accaactctt tttccgaagg 4140

taactggctt cagcagagcg cagataccaa atactgtcct tctagtgtag ccgtagttag 4200

gccaccactt caagaactct gtagcaccgc ctacatacct cgctctgcta atcctgttac 4260

cagtggctgc tgccagtggc gataagtcgt gtcttaccgg gttggactca agacgatagt 4320

taccggataa ggcgcagcgg tcgggctgaa cggggggttc gtgcacacag cccagcttgg 4380

agcgaacgac ctacaccgaa ctgagatacc tacagcgtga gctatgagaa agcgccacgc 4440

ttcccgaagg gagaaaggcg gacaggtatc cggtaagcgg cagggtcgga acaggagagc 4500

gcacgaggga gcttccaggg ggaaacgcct ggtatcttta tagtcctgtc gggtttcgcc 4560

acctctgact tgagcgtcga tttttgtgat gctcgtcagg ggggcggagc ctatggaaaa 4620

acgccagcaa cgcggccttt ttacggttcc tggccttttg ctggcctttt gctcacatgt 4680

tctttcctgc gttatcccct gattctgtgg ataaccgtat taccgccatg cat 4733

<210> 17
<211> 6816
<212> DNA
<213> synthetic

<400> 17
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacgccacc atgcaggtga 660

gcaagaggat gctggcgggg ggcgtgagga gcatgcccag ccccctcctg gcctgctggc 720

agcccatcct cctgctggtg ctgggctcag tgctgtcagg ctcggccacg ggctgcccgc 780

cccgctgcga gtgctccgcc caggaccgcg ctgtgctgtg ccaccgcaag cgctttgtgg 840

cagtccccga gggcatcccc accgagacgc gcctgctgga cctaggcaag aaccgcatca 900

aaacgctcaa ccaggacgag ttcgccagct tcccgcacct ggaggagctg gagctcaacg 960

agaacatcgt gagcgccgtg gagcccggcg ccttcaacaa cctcttcaac ctccggacgc 1020

tgggtctccg cagcaaccgc ctgaagctca tcccgctagg cgtcttcact ggcctcagca 1080

acctgaccaa gctggacatc agcgagaaca agatcgttat cctactggac tacatgtttc 1140

aggacctgta caacctcaag tcactggagg ttggcgacaa tgacctcgtc tacatctctc 1200

accgcgcctt cagcggcctc aacagcctgg agcagctgac gctggagaaa tgcaacctga 1260

cctccatccc caccgaggcg ctgtcccacc tgcacggcct catcgtcctg aggctccggc 1320

acctcaacat caatgccatc cgggactact ccttcaagag gctgtaccga ctcaaggtct 1380

tggagatctc ccactggccc tacttggaca ccatgacacc caactgcctc tacggcctca 1440

acctgacgtc cctgtccatc acacactgca atctgaccgc tgtgccctac ctggccgtcc 1500

gccacctagt ctatctccgc ttcctcaacc tctcctacaa ccccatcagc accattgagg 1560

gctccatgtt gcatgagctg ctccggctgc aggagatcca gctggtgggc gggcagctgg 1620

ccgtggtgga gccctatgcc ttccgcggcc tcaactacct gcgcgtgctc aatgtctctg 1680

gcaaccagct gaccacactg gaggaatcag tcttccactc ggtgggcaac ctggagacac 1740

tcatcctgga ctccaacccg ctggcctgcg actgtcggct cctgtgggtg ttccggcgcc 1800

gctggcggct caacttcaac cggcagcagc ccacgtgcgc cacgcccgag tttgtccagg 1860

gcaaggagtt caaggacttc cctgatgtgc tactgcccaa ctacttcacc tgccgccgcg 1920

cccgcatccg ggaccgcaag gcccagcagg tgtttgtgga cgagggccac acggtgcagt 1980

ttgtgtgccg ggccgatggc gacccgccgc ccgccatcct ctggctctca ccccgaaagc 2040

acctggtctc agccaagagc aatgggcggc tcacagtctt ccctgatggc acgctggagg 2100

tgcgctacgc ccaggtacag gacaacggca cgtacctgtg catcgcggcc aacgcgggcg 2160

gcaacgactc catgcccgcc cacctgcatg tgcgcagcta ctcgcccgac tggccccatc 2220

agcccaacaa gaccttcgct ttcatctcca accagccggg cgagggagag gccaacagca 2280

cccgcgccac tgtgcctttc cccttcgaca tcaagaccct catcatcgcc accaccatgg 2340

gcttcatctc tttcctgggc gtcgtcctct tctgcctggt gctgctgttt ctctggagcc 2400

ggggcaaggg caacacaaag cacaacatcg agatcgagta tgtgccccga aagtcggacg 2460

caggcatcag ctccgccgac gcgccccgca agttcaacat gaagatgata tcacgggatc 2520

caccggtagc aaccatgacc agcaaggtgt acgaccccga gcagaggaag aggatgatca 2580

ccggccccca gtggtgggcc aggtgcaagc agatgaacgt gctggacagc ttcatcaact 2640

actacgacag cgagaagcac gccgagaacg ccgtgatctt cctgcacggc aacgccgcta 2700

gtagctacct gtggaggcac gtggtgcccc acatcgagcc cgtggccagg tgcatcatcc 2760

ccgatctgat cggcatgggc aagagcggca agagcggcaa cggcagctac aggctgctgg 2820

accactacaa gtacctgacc gcctggttcg agctcctgaa cctgcccaag aagatcatct 2880

tcgtgggcca cgactggggc gcctgcctgg ccttccacta cagctacgag caccaggaca 2940

agatcaaggc catcgtgcac gccgagagcg tggtggacgt gatcgagagc tgggacgagt 3000

ggccagacat cgaggaggac atcgccctga tcaagagcga ggagggcgag aagatggtgc 3060

tggagaacaa cttcttcgtg gagaccatgc tgcccagcaa gatcatgaga aagctggagc 3120

ccgaggagtt cgccgcctac ctggagccct tcaaggagaa gggcgaggtg agaagaccca 3180

ccctgagctg gcccagagag atccccctgg tgaagggcgg caagcccgac gtggtgcaga 3240

tcgtgagaaa ctacaacgcc tacctgagag ccagcgacga cctgcccaag atgttcatcg 3300

agagcgaccc cggcttcttc agcaacgcca tcgtggaggg cgccaagaag ttccccaaca 3360

ccgagttcgt gaaggtgaag ggcctgcact tcagccagga ggacgccccc gacgagatgg 3420

gcaagtacat caagagcttc gtggagagag tgctgaagaa cgagcagtaa tgtacaagta 3480

aagcggccgc gactctagat cataatcagc cataccacat ttgtagaggt tttacttgct 3540

ttaaaaaacc tcccacacct ccccctgaac ctgaaacata aaatgaatgc aattgttgtt 3600

gttaacttgt ttattgcagc ttataatggt tacaaataaa gcaatagcat cacaaatttc 3660

acaaataaag catttttttc actgcattct agttgtggtt tgtccaaact catcaatgta 3720

tcttaaggcg taaattgtaa gcgttaatat tttgttaaaa ttcgcgttaa atttttgtta 3780

aatcagctca ttttttaacc aataggccga aatcggcaaa atcccttata aatcaaaaga 3840

atagaccgag atagggttga gtgttgttcc agtttggaac aagagtccac tattaaagaa 3900

cgtggactcc aacgtcaaag ggcgaaaaac cgtctatcag ggcgatggcc cactacgtga 3960

accatcaccc taatcaagtt ttttggggtc gaggtgccgt aaagcactaa atcggaaccc 4020

taaagggagc ccccgattta gagcttgacg gggaaagccg gcgaacgtgg cgagaaagga 4080

agggaagaaa gcgaaaggag cgggcgctag ggcgctggca agtgtagcgg tcacgctgcg 4140

cgtaaccacc acacccgccg cgcttaatgc gccgctacag ggcgcgtcag gtggcacttt 4200

tcggggaaat gtgcgcggaa cccctatttg tttatttttc taaatacatt caaatatgta 4260

tccgctcatg agacaataac cctgataaat gcttcaataa tattgaaaaa ggaagagtcc 4320

tgaggcggaa agaaccagct gtggaatgtg tgtcagttag ggtgtggaaa gtccccaggc 4380

tccccagcag gcagaagtat gcaaagcatg catctcaatt agtcagcaac caggtgtgga 4440

aagtccccag gctccccagc aggcagaagt atgcaaagca tgcatctcaa ttagtcagca 4500

accatagtcc cgcccctaac tccgcccatc ccgcccctaa ctccgcccag ttccgcccat 4560

tctccgcccc atggctgact aatttttttt atttatgcag aggccgaggc cgcctcggcc 4620

tctgagctat tccagaagta gtgaggaggc ttttttggag gcctaggctt ttgcaaagat 4680

cgatcaagag acaggatgag gatcgtttcg catgattgaa caagatggat tgcacgcagg 4740

ttctccggcc gcttgggtgg agaggctatt cggctatgac tgggcacaac agacaatcgg 4800

ctgctctgat gccgccgtgt tccggctgtc agcgcagggg cgcccggttc tttttgtcaa 4860

gaccgacctg tccggtgccc tgaatgaact gcaagacgag gcagcgcggc tatcgtggct 4920

ggccacgacg ggcgttcctt gcgcagctgt gctcgacgtt gtcactgaag cgggaaggga 4980

ctggctgcta ttgggcgaag tgccggggca ggatctcctg tcatctcacc ttgctcctgc 5040

cgagaaagta tccatcatgg ctgatgcaat gcggcggctg catacgcttg atccggctac 5100

ctgcccattc gaccaccaag cgaaacatcg catcgagcga gcacgtactc ggatggaagc 5160

cggtcttgtc gatcaggatg atctggacga agagcatcag gggctcgcgc cagccgaact 5220

gttcgccagg ctcaaggcga gcatgcccga cggcgaggat ctcgtcgtga cccatggcga 5280

tgcctgcttg ccgaatatca tggtggaaaa tggccgcttt tctggattca tcgactgtgg 5340

ccggctgggt gtggcggacc gctatcagga catagcgttg gctacccgtg atattgctga 5400

agagcttggc ggcgaatggg ctgaccgctt cctcgtgctt tacggtatcg ccgctcccga 5460

ttcgcagcgc atcgccttct atcgccttct tgacgagttc ttctgagcgg gactctgggg 5520

ttcgaaatga ccgaccaagc gacgcccaac ctgccatcac gagatttcga ttccaccgcc 5580

gccttctatg aaaggttggg cttcggaatc gttttccggg acgccggctg gatgatcctc 5640

cagcgcgggg atctcatgct ggagttcttc gcccacccta gggggaggct aactgaaaca 5700

cggaaggaga caataccgga aggaacccgc gctatgacgg caataaaaag acagaataaa 5760

acgcacggtg ttgggtcgtt tgttcataaa cgcggggttc ggtcccaggg ctggcactct 5820

gtcgataccc caccgagacc ccattggggc caatacgccc gcgtttcttc cttttcccca 5880

ccccaccccc caagttcggg tgaaggccca gggctcgcag ccaacgtcgg ggcggcaggc 5940

cctgccatag cctcaggtta ctcatatata ctttagattg atttaaaact tcatttttaa 6000

tttaaaagga tctaggtgaa gatccttttt gataatctca tgaccaaaat cccttaacgt 6060

gagttttcgt tccactgagc gtcagacccc gtagaaaaga tcaaaggatc ttcttgagat 6120

cctttttttc tgcgcgtaat ctgctgcttg caaacaaaaa aaccaccgct accagcggtg 6180

gtttgtttgc cggatcaaga gctaccaact ctttttccga aggtaactgg cttcagcaga 6240

gcgcagatac caaatactgt ccttctagtg tagccgtagt taggccacca cttcaagaac 6300

tctgtagcac cgcctacata cctcgctctg ctaatcctgt taccagtggc tgctgccagt 6360

ggcgataagt cgtgtcttac cgggttggac tcaagacgat agttaccgga taaggcgcag 6420

cggtcgggct gaacgggggg ttcgtgcaca cagcccagct tggagcgaac gacctacacc 6480

gaactgagat acctacagcg tgagctatga gaaagcgcca cgcttcccga agggagaaag 6540

gcggacaggt atccggtaag cggcagggtc ggaacaggag agcgcacgag ggagcttcca 6600

gggggaaacg cctggtatct ttatagtcct gtcgggtttc gccacctctg acttgagcgt 6660

cgatttttgt gatgctcgtc aggggggcgg agcctatgga aaaacgccag caacgcggcc 6720

tttttacggt tcctggcctt ttgctggcct tttgctcaca tgttctttcc tgcgttatcc 6780

cctgattctg tggataaccg tattaccgcc atgcat 6816

<210>  18
<211>  6589
<212>  DNA
<213>  synthetic

<400>  18
tagttattaa tagtaatcaa ttacggggtc attagttcat agcccatata tggagttccg        60

cgttacataa cttacggtaa atggcccgcc tggctgaccg cccaacgacc cccgcccatt       120

gacgtcaata atgacgtatg ttcccatagt aacgccaata gggactttcc attgacgtca       180

atgggtggag tatttacggt aaactgccca cttggcagta catcaagtgt atcatatgcc       240

aagtacgccc cctattgacg tcaatgacgg taaatggccc gcctggcatt atgcccagta       300

catgacctta tgggactttc ctacttggca gtacatctac gtattagtca tcgctattac       360

catggtgatg cggttttggc agtacatcaa tgggcgtgga tagcggtttg actcacgggg       420

atttccaagt ctccacccca ttgacgtcaa tgggagtttg ttttggcacc aaaatcaacg       480

ggactttcca aaatgtcgta acaactccgc cccattgacg caaatgggcg gtaggcgtgt       540

acggtgggag gtctatataa gcagagctgg tttagtgaac cgtcagatcc gctagcgcta       600

ccggactcag atctcgagct caagcttcga attctgcagt cgacgccacc atgcaggtga       660

gcaagaggat gctggcgggg ggcgtgagga gcatgcccag ccccctcctg gcctgctggc       720

agcccatcct cctgctggtg ctgggctcag tgctgtcagg ctcggccacg ggctgcccgc       780

cccgctgcga gtgctccgcc caggaccgcg ctgtgctgtg ccaccgcaag cgctttgtgg       840

cagtccccga gggcatcccc accgagacgc gcctgctgga cctaggcaag aaccgcatca       900

aaacgctcaa ccaggacgag ttcgccagct tcccgcacct ggaggagctg gagctcaacg       960

agaacatcgt gagcgccgtg gagcccggcg ccttcaacaa cctcttcaac ctccggacgc      1020

tgggtctccg cagcaaccgc ctgaagctca tcccgctagg cgtcttcact ggcctcagca      1080

acctgaccaa gctggacatc agcgagaaca agatcgttat cctactggac tacatgtttc      1140

aggacctgta caacctcaag tcactggagg ttggcgacaa tgacctcgtc tacatctctc      1200

accgcgcctt cagcggcctc aacagcctgg agcagctgac gctggagaaa tgcaacctga      1260

cctccatccc caccgaggcg ctgtcccacc tgcacggcct catcgtcctg aggctccggc      1320

acctcaacat caatgccatc cgggactact ccttcaagag gctgtaccga ctcaaggtct      1380

tggagatctc ccactggccc tacttggaca ccatgacacc caactgcctc tacggcctca      1440

acctgacgtc cctgtccatc acacactgca atctgaccgc tgtgccctac ctggccgtcc      1500

gccacctagt ctatctccgc ttcctcaacc tctcctacaa ccccatcagc accattgagg      1560

gctccatgtt gcatgagctg ctccggctgc aggagatcca gctggtgggc gggcagctgg      1620

ccgtggtgga gccctatgcc ttccgcggcc tcaactacct gcgcgtgctc aatgtctctg      1680

gcaaccagct gaccacactg gaggaatcag tcttccactc ggtgggcaac ctggagacac      1740

tcatcctgga ctccaacccg ctggcctgcg actgtcggct cctgtgggtg ttccggcgcc 1800

gctggcggct caacttcaac cggcagcagc ccacgtgcgc cacgcccgag tttgtccagg 1860

gcaaggagtt caaggacttc cctgatgtgc tactgcccaa ctacttcacc tgccgccgcg 1920

cccgcatccg ggaccgcaag gcccagcagg tgtttgtgga cgagggccac acggtgcagt 1980

ttgtgtgccg ggccgatggc gacccgccgc ccgccatcct ctggctctca ccccgaaagc 2040

acctggtctc agccaagagc aatgggcggc tcacagtctt ccctgatggc acgctggagg 2100

tgcgctacgc ccaggtacag gacaacggca cgtacctgtg catcgcggcc aacgcgggcg 2160

gcaacgactc catgcccgcc cacctgcatg tgcgcagcta ctcgcccgac tggccccatc 2220

agcccaacaa gaccttcgct ttcatctcca accagccggg cgagggagag gccaacagca 2280

cccgcgccac tgtgcctttc cccttcgaca tcaagaccct catcatcgcc accaccatgg 2340

gcttcatctc tttcctgggc gtcgtcctct tctgcctggt gctgctgttt ctctggagcc 2400

ggggcaaggg caacacaaag cacaacatcg agatcgagta tgtgccccga aagtcggacg 2460

caggcatcag ctccgccgac gcgccccgca agttcaacat gaagatgata tcacgggatc 2520

caccggtcgc caccatggtg agcaagggcg aggagctgtt caccggggtg gtgcccatcc 2580

tggtcgagct ggacggcgac gtaaacggcc acaagttcag cgtgtccggc gagggcgagg 2640

gcgatgccac ctacggcaag ctgaccctga agttcatctg caccaccggc aagctgcccg 2700

tgccctggcc caccctcgtg accaccttcg gctacggcct gcagtgcttc gcccgctacc 2760

ccgaccacat gaagcagcac gacttcttca agtccgccat gcccgaaggc tacgtccagg 2820

agcgcaccat cttcttcaag gacgacggca actacaagac ccgcgccgag gtgaagttcg 2880

agggcgacac cctggtgaac cgcatcgagc tgaagggcat cgacttcaag gaggacggca 2940

acatcctggg gcacaagctg gagtacaact acaacagcca caacgtctat atcatggccg 3000

acaagcagaa gaacggcatc aaggtgaact tcaagatccg ccacaacatc gaggacggca 3060

gcgtgcagct cgccgaccac taccagcaga acacccccat cggcgacggc cccgtgctgc 3120

tgcccgacaa ccactacctg agctaccagt ccgccctgag caaagacccc aacgagaagc 3180

gcgatcacat ggtcctgctg gagttcgtga ccgccgccgg gatcactctc ggcatggacg 3240

agctgtacaa gtaaagcggc cgcgactcta gatcataatc agccatacca catttgtaga 3300

ggttttactt gctttaaaaa acctcccaca cctccccctg aacctgaaac ataaaatgaa 3360

tgcaattgtt gttgttaact tgtttattgc agcttataat ggttacaaat aaagcaatag 3420

catcacaaat ttcacaaata aagcattttt ttcactgcat tctagttgtg gtttgtccaa 3480

actcatcaat gtatcttaag gcgtaaattg taagcgttaa tattttgtta aaattcgcgt 3540

taaatttttg ttaaatcagc tcatttttta accaataggc cgaaatcggc aaaatccctt 3600

ataaatcaaa agaatagacc gagatagggt tgagtgttgt tccagtttgg aacaagagtc 3660

cactattaaa gaacgtggac tccaacgtca aagggcgaaa aaccgtctat cagggcgatg 3720

gcccactacg tgaaccatca ccctaatcaa gttttttggg gtcgaggtgc cgtaaagcac 3780

taaatcggaa ccctaaaggg agcccccgat ttagagcttg acggggaaag ccggcgaacg 3840

tggcgagaaa ggaagggaag aaagcgaaag gagcgggcgc tagggcgctg gcaagtgtag 3900

cggtcacgct gcgcgtaacc accacacccg ccgcgcttaa tgcgccgcta cagggcgcgt 3960

caggtggcac ttttcgggga aatgtgcgcg gaacccctat ttgtttattt ttctaaatac 4020

attcaaatat gtatccgctc atgagacaat aaccctgata aatgcttcaa taatattgaa 4080

aaaggaagag tcctgaggcg gaaagaacca gctgtggaat gtgtgtcagt tagggtgtgg 4140

aaagtcccca ggctccccag caggcagaag tatgcaaagc atgcatctca attagtcagc 4200

aaccaggtgt ggaaagtccc caggctcccc agcaggcaga agtatgcaaa gcatgcatct 4260

caattagtca gcaaccatag tcccgcccct aactccgccc atcccgcccc taactccgcc 4320

cagttccgcc cattctccgc cccatggctg actaattttt tttatttatg cagaggccga 4380

ggccgcctcg gcctctgagc tattccagaa gtagtgagga ggcttttttg gaggcctagg 4440

cttttgcaaa gatcgatcaa gagacaggat gaggatcgtt tcgcatgatt gaacaagatg 4500

gattgcacgc aggttctccg gccgcttggg tggagaggct attcggctat gactgggcac 4560

aacagacaat cggctgctct gatgccgccg tgttccggct gtcagcgcag gggcgcccgg 4620

ttctttttgt caagaccgac ctgtccggtg ccctgaatga actgcaagac gaggcagcgc 4680

ggctatcgtg gctggccacg acgggcgttc cttgcgcagc tgtgctcgac gttgtcactg 4740

aagcgggaag ggactggctg ctattgggcg aagtgccggg gcaggatctc ctgtcatctc 4800

accttgctcc tgccgagaaa gtatccatca tggctgatgc aatgcggcgg ctgcatacgc 4860

ttgatccggc tacctgccca ttcgaccacc aagcgaaaca tcgcatcgag cgagcacgta 4920

ctcggatgga agccggtctt gtcgatcagg atgatctgga cgaagagcat caggggctcg 4980

cgccagccga actgttcgcc aggctcaagg cgagcatgcc cgacggcgag gatctcgtcg 5040

tgacccatgg cgatgcctgc ttgccgaata tcatggtgga aaatggccgc ttttctggat 5100

tcatcgactg tggccggctg ggtgtggcgg accgctatca ggacatagcg ttggctaccc 5160

gtgatattgc tgaagagctt ggcggcgaat gggctgaccg cttcctcgtg ctttacggta 5220

tcgccgctcc cgattcgcag cgcatcgcct tctatcgcct tcttgacgag ttcttctgag 5280

cgggactctg gggttcgaaa tgaccgacca agcgacgccc aacctgccat cacgagattt 5340

cgattccacc gccgccttct atgaaaggtt gggcttcgga atcgttttcc gggacgccgg 5400

ctggatgatc ctccagcgcg gggatctcat gctggagttc ttcgcccacc ctagggggag 5460

gctaactgaa acacggaagg agacaatacc ggaaggaacc cgcgctatga cggcaataaa 5520

aagacagaat aaaacgcacg gtgttgggtc gtttgttcat aaacgcgggg ttcggtccca 5580

gggctggcac tctgtcgata ccccaccgag accccattgg ggccaatacg cccgcgtttc 5640

ttccttttcc ccaccccacc ccccaagttc gggtgaaggc ccagggctcg cagccaacgt 5700

cggggcggca ggccctgcca tagcctcagg ttactcatat atactttaga ttgatttaaa 5760

acttcatttt taatttaaaa ggatctaggt gaagatcctt tttgataatc tcatgaccaa 5820

aatcccttaa cgtgagtttt cgttccactg agcgtcagac cccgtagaaa agatcaaagg 5880

atcttcttga gatccttttt ttctgcgcgt aatctgctgc ttgcaaacaa aaaaaccacc 5940

gctaccagcg gtggtttgtt tgccggatca agagctacca actctttttc cgaaggtaac 6000

tggcttcagc agagcgcaga taccaaatac tgtccttcta gtgtagccgt agttaggcca 6060

ccacttcaag aactctgtag caccgcctac atacctcgct ctgctaatcc tgttaccagt 6120

ggctgctgcc agtggcgata agtcgtgtct taccgggttg gactcaagac gatagttacc 6180

ggataaggcg cagcggtcgg gctgaacggg gggttcgtgc acacagccca gcttggagcg 6240

aacgacctac accgaactga gatacctaca gcgtgagcta tgagaaagcg ccacgcttcc 6300

cgaagggaga aaggcggaca ggtatccggt aagcggcagg gtcggaacag gagagcgcac 6360

gagggagctt ccagggggaa acgcctggta tctttatagt cctgtcgggt ttcgccacct 6420

ctgacttgag cgtcgatttt tgtgatgctc gtcagggggg cggagcctat ggaaaaacgc 6480

cagcaacgcg gcctttttac ggttcctggc cttttgctgg ccttttgctc acatgttctt 6540

tcctgcgtta tcccctgatt ctgtggataa ccgtattacc gccatgcat 6589

<210> 19
<211> 7311
<212> DNA
<213> synthetic

<400> 19
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gcttgccacc 900

atgcaggtga gcaagaggat gctggcgggg ggcgtgagga gcatgcccag ccccctcctg 960

gcctgctggc agcccatcct cctgctggtg ctgggctcag tgctgtcagg ctcggccacg 1020

ggctgcccgc cccgctgcga gtgctccgcc caggaccgcg ctgtgctgtg ccaccgcaag 1080

cgctttgtgg cagtccccga gggcatcccc accgagacgc gcctgctgga cctaggcaag 1140

aaccgcatca aaacgctcaa ccaggacgag ttcgccagct tcccgcacct ggaggagctg 1200

gagctcaacg agaacatcgt gagcgccgtg gagcccggcg ccttcaacaa cctcttcaac 1260

ctccggacgc tgggtctccg cagcaaccgc ctgaagctca tcccgctagg cgtcttcact 1320

ggcctcagca acctgaccaa gctggacatc agcgagaaca agatcgttat cctactggac 1380

tacatgtttc aggacctgta caacctcaag tcactggagg ttggcgacaa tgacctcgtc 1440

tacatctctc accgcgcctt cagcggcctc aacagcctgg agcagctgac gctggagaaa 1500

tgcaacctga cctccatccc caccgaggcg ctgtcccacc tgcacggcct catcgtcctg 1560

aggctccggc acctcaacat caatgccatc cgggactact ccttcaagag gctgtaccga 1620

ctcaaggtct tggagatctc ccactggccc tacttggaca ccatgacacc caactgcctc 1680

tacggcctca acctgacgtc cctgtccatc acacactgca atctgaccgc tgtgccctac 1740

ctggccgtcc gccacctagt ctatctccgc ttcctcaacc tctcctacaa ccccatcagc 1800

accattgagg gctccatgtt gcatgagctg ctccggctgc aggagatcca gctggtgggc 1860

gggcagctgg ccgtggtgga gccctatgcc ttccgcggcc tcaactacct gcgcgtgctc 1920

aatgtctctg gcaaccagct gaccacactg gaggaatcag tcttccactc ggtgggcaac 1980

ctggagacac tcatcctgga ctccaacccg ctggcctgcg actgtcggct cctgtgggtg 2040

ttccggcgcc gctggcggct caacttcaac cggcagcagc ccacgtgcgc cacgcccgag 2100

tttgtccagg gcaaggagtt caaggacttc cctgatgtgc tactgcccaa ctacttcacc 2160

tgccgccgcg cccgcatccg ggaccgcaag gcccagcagg tgtttgtgga cgagggccac 2220

acggtgcagt ttgtgtgccg ggccgatggc gacccgccgc ccgccatcct ctggctctca 2280

ccccgaaagc acctggtctc agccaagagc aatgggcggc tcacagtctt ccctgatggc 2340

acgctggagg tgcgctacgc ccaggtacag gacaacggca cgtacctgtg catcgcggcc 2400

aacgcgggcg gcaacgactc catgcccgcc cacctgcatg tgcgcagcta ctcgcccgac 2460

tggccccatc agcccaacaa gaccttcgct ttcatctcca accagccggg cgagggagag 2520

gccaacagca cccgcgccac tgtgcctttc cccttcgaca tcaagaccct catcatcgcc 2580

accaccatgg gcttcatctc tttcctgggc gtcgtcctct tctgcctggt gctgctgttt 2640

ctctggagcc ggggcaaggg caacacaaag cacaacatcg agatcgagta tgtgccccga 2700

aagtcggacg caggcatcag ctccgccgac gcgccccgca agttcaacat gaagatgata 2760

ggatccgcta gctatccata tgatgttcct gattatgctt gagaattctg cagatatcca 2820

tcacactggc ggccgctcga gcatgcatct agagggccct attctatagt gtcacctaaa 2880

tgctagagct cgctgatcag cctcgactgt gccttctagt tgccagccat ctgttgtttg 2940

cccctccccc gtgccttcct tgaccctgga aggtgccact cccactgtcc tttcctaata 3000

aaatgaggaa attgcatcgc attgtctgag taggtgtcat tctattctgg ggggtggggt 3060

ggggcaggac agcaaggggg aggattggga agacaatagc aggcatgctg gggatgcggt 3120

gggctctatg gcttctgagg cggaaagaac cagctggggc tctagggggt atccccacgc 3180

gccctgtagc ggcgcattaa gcgcggcggg tgtggtggtt acgcgcagcg tgaccgctac 3240

acttgccagc gccctagcgc ccgctccttt cgctttcttc ccttcctttc tcgccacgtt 3300

cgccggcttt ccccgtcaag ctctaaatcg gggcatccct ttagggttcc gatttagtgc 3360

tttacggcac ctcgacccca aaaaacttga ttagggtgat ggttcacgta gtgggccatc 3420

gccctgatag acggtttttc gccctttgac gttggagtcc acgttcttta atagtggact 3480

cttgttccaa actggaacaa cactcaaccc tatctcggtc tattcttttg atttataagg 3540

gattttgggg atttcggcct attggttaaa aaatgagctg atttaacaaa aatttaacgc 3600

gaattaattc tgtggaatgt gtgtcagtta gggtgtggaa agtccccagg ctccccaggc 3660

aggcagaagt atgcaaagca tgcatctcaa ttagtcagca accaggtgtg gaaagtcccc 3720

aggctcccca gcaggcagaa gtatgcaaag catgcatctc aattagtcag caaccatagt 3780

cccgccccta actccgccca tcccgcccct aactccgccc agttccgccc attctccgcc 3840

ccatggctga ctaatttttt ttatttatgc agaggccgag gccgcctctg cctctgagct 3900

attccagaag tagtgaggag gcttttttgg aggcctaggc ttttgcaaaa agctcccggg 3960

agcttgtata tccattttcg gatctgatca agagacagga tgaggatcgt ttcgcatgat 4020

tgaacaagat ggattgcacg caggttctcc ggccgcttgg gtggagaggc tattcggcta 4080

tgactgggca caacagacaa tcggctgctc tgatgccgcc gtgttccggc tgtcagcgca 4140

ggggcgcccg gttctttttg tcaagaccga cctgtccggt gccctgaatg aactgcagga 4200

cgaggcagcg cggctatcgt ggctggccac gacgggcgtt ccttgcgcag ctgtgctcga 4260

cgttgtcact gaagcgggaa gggactggct gctattgggc gaagtgccgg ggcaggatct 4320

cctgtcatct caccttgctc ctgccgagaa agtatccatc atggctgatg caatgcggcg 4380

gctgcatacg cttgatccgg ctacctgccc attcgaccac caagcgaaac atcgcatcga 4440

gcgagcacgt actcggatgg aagccggtct tgtcgatcag gatgatctgg acgaagagca 4500

tcaggggctc gcgccagccg aactgttcgc caggctcaag gcgcgcatgc ccgacggcga 4560

ggatctcgtc gtgacccatg gcgatgcctg cttgccgaat atcatggtgg aaaatggccg 4620

cttttctgga ttcatcgact gtggccggct gggtgtggcg gaccgctatc aggacatagc 4680

gttggctacc cgtgatattg ctgaagagct tggcggcgaa tgggctgacc gcttcctcgt 4740

gctttacggt atcgccgctc ccgattcgca gcgcatcgcc ttctatcgcc ttcttgacga 4800

gttcttctga gcgggactct ggggttcgaa atgaccgacc aagcgacgcc caacctgcca 4860

tcacgagatt tcgattccac cgccgccttc tatgaaaggt tgggcttcgg aatcgttttc 4920

cgggacgccg gctggatgat cctccagcgc ggggatctca tgctggagtt cttcgcccac 4980

cccaacttgt ttattgcagc ttataatggt tacaaataaa gcaatagcat cacaaatttc 5040

acaaataaag catttttttc actgcattct agttgtggtt tgtccaaact catcaatgta 5100

tcttatcatg tctgtatacc gtcgacctct agctagagct tggcgtaatc atggtcatag 5160

ctgtttcctg tgtgaaattg ttatccgctc acaattccac acaacatacg agccggaagc 5220

ataaagtgta aagcctgggg tgcctaatga gtgagctaac tcacattaat tgcgttgcgc 5280

tcactgcccg ctttccagtc gggaaacctg tcgtgccagc tgcattaatg aatcggccaa 5340

cgcgcgggga gaggcggttt gcgtattggg cgctcttccg cttcctcgct cactgactcg 5400

ctgcgctcgg tcgttcggct gcggcgagcg gtatcagctc actcaaaggc ggtaatacgg 5460

ttatccacag aatcagggga taacgcagga aagaacatgt gagcaaaagg ccagcaaaag 5520

gccaggaacc gtaaaaaggc cgcgttgctg gcgtttttcc ataggctccg cccccctgac 5580

gagcatcaca aaaatcgacg ctcaagtcag aggtggcgaa acccgacagg actataaaga 5640

taccaggcgt ttccccctgg aagctccctc gtgcgctctc ctgttccgac cctgccgctt 5700

accggatacc tgtccgcctt tctcccttcg ggaagcgtgg cgctttctca atgctcacgc 5760

tgtaggtatc tcagttcggt gtaggtcgtt cgctccaagc tgggctgtgt gcacgaaccc 5820

cccgttcagc ccgaccgctg cgccttatcc ggtaactatc gtcttgagtc caacccggta 5880

agacacgact tatcgccact ggcagcagcc actggtaaca ggattagcag agcgaggtat 5940

gtaggcggtg ctacagagtt cttgaagtgg tggcctaact acggctacac tagaaggaca 6000

gtatttggta tctgcgctct gctgaagcca gttaccttcg gaaaaagagt tggtagctct 6060

tgatccggca aacaaaccac cgctggtagc ggtggttttt ttgtttgcaa gcagcagatt 6120

acgcgcagaa aaaaaggatc tcaagaagat cctttgatct tttctacggg gtctgacgct 6180

cagtggaacg aaaactcacg ttaagggatt ttggtcatga gattatcaaa aaggatcttc 6240

acctagatcc ttttaaatta aaaatgaagt tttaaatcaa tctaaagtat atatgagtaa 6300

acttggtctg acagttacca atgcttaatc agtgaggcac ctatctcagc gatctgtcta 6360

tttcgttcat ccatagttgc ctgactcccc gtcgtgtaga taactacgat acgggagggc 6420

ttaccatctg gccccagtgc tgcaatgata ccgcgagacc cacgctcacc ggctccagat 6480

ttatcagcaa taaaccagcc agccggaagg gccgagcgca gaagtggtcc tgcaacttta 6540

tccgcctcca tccagtctat taattgttgc cgggaagcta gagtaagtag ttcgccagtt 6600

aatagtttgc gcaacgttgt tgccattgct acaggcatcg tggtgtcacg ctcgtcgttt 6660

ggtatggctt cattcagctc cggttcccaa cgatcaaggc gagttacatg atcccccatg 6720

ttgtgcaaaa aagcggttag ctccttcggt cctccgatcg ttgtcagaag taagttggcc 6780

gcagtgttat cactcatggt tatggcagca ctgcataatt ctcttactgt catgccatcc 6840

gtaagatgct tttctgtgac tggtgagtac tcaaccaagt cattctgaga atagtgtatg 6900

cggcgaccga gttgctcttg cccggcgtca atacgggata ataccgcgcc acatagcaga 6960

actttaaaag tgctcatcat tggaaaacgt tcttcggggc gaaaactctc aaggatctta 7020

ccgctgttga gatccagttc gatgtaaccc actcgtgcac ccaactgatc ttcagcatct 7080

tttactttca ccagcgtttc tgggtgagca aaaacaggaa ggcaaaatgc cgcaaaaaag 7140

ggaataaggg cgacacggaa atgttgaata ctcatactct tcctttttca atattattga 7200

agcatttatc agggttattg tctcatgagc ggatacatat ttgaatgtat ttagaaaaat 7260

aaacaaatag gggttccgcg cacatttccc cgaaaagtgc cacctgacgt c 7311

## Description

### DOMAINE TECHNIQUE

La présente invention se rapporte à un produit de couplage comprenant un monomère d'une protéine choisie parmi Lingo-1, Lingo-2, Lingo-3 et Lingo-4 et une sonde émettant un signal lors de changements conformationnels dudit monomère, à des séquences d'acides nucléiques codant pour ce produit, des vecteurs permettant sa préparation, un système le comprenant et un procédé de criblage permettant d'identifier des ligands d'une protéine choisie parmi Lingo-1, Lingo-2, Lingo-3 et Lingo-4.

La présente invention trouve ses applications industrielles dans le domaine des procédés de détection de molécules, de détection d'interaction entre molécules et de criblage moléculaire, ainsi que dans le domaine médical.

Dans la description ci-dessous, les références entre crochets ([]) renvoient à la liste des références présentée après les exemples.

### ETAT DE LA TECHNIQUE

Les maladies neurodégénératives se caractérisent par la dégénérescence de certains neurones et la destruction du système nerveux. La maladie de Parkinson et la sclérose en plaque sont deux de ces maladies.

La maladie de Parkinson touche 1 à 2 individus pour 1000. La dégénérescence concerne les neurones dopaminergiques qui produisent un neurotransmetteur, la dopamine, qui intervient notamment dans le contrôle des mouvements du corps. Actuellement, les traitements développés contre la maladie, essentiellement des dopaminergiques et des agonistes de la dopamine, permettent une amélioration des troubles en ralentissant le développement de la maladie. Toutefois, ces traitements ne permettent pas de guérir la maladie.

La sclérose en plaques est une maladie auto-immune qui a une prévalence de 1 pour 1000 et dans laquelle la myéline des axones du cerveau et de la moelle épinière est détruite. Cela provoque des difficultés de conduction de l'influx nerveux affectant ainsi pratiquement toutes les fonctions biologiques. Les traitements actuels font appel à des immunosuppresseurs **ou** immunomodulateurs. Mais là encore, ces traitements ne parviennent qu'à ralentir la progression de la maladie et pas à la guérir.

Une seule et même protéine, appelée Lingo-1, est au carrefour de ces deux maladies. Par ailleurs, la protéine Lingo-4 a un rôle dans les maladies impliquant la myéline (WO2009/061500). Lingo-1 est une protéine transmembranaire de 580 résidus (sans le peptide signal) . Elle possède une importante région extracellulaire (516 acides aminés) constituée de 12 motifs LRR (leucine-rich repeat) et d'un domaine Ig (immunoglobuline). La portion cytoplasmique, comprenant 38 résidus, contient un site canonique de phosphorylation par l'EGFR (Epidermal Growth Factor Receptor). Lingo-1 appartient à la famille des protéines LRR qui jouent des rôles clefs dans la biologie du système nerveux central (SNC) et constituent des cibles attractives pour le traitement de maladies neurologiques et neurodégénératives.

Le profil d'expression de Lingo-1 est spécifique du système nerveux avec une expression abondante dans le SNC et plus particulièrement dans les neurones et les oligodendrocytes. Cette protéine transmembranaire exerce plusieurs fonctions inhibitrices: celle de la survie neuronale, celle de la régénération des axones et des neurites après une lésion et celle de la maturation des oligodendrocytes et de la myélinisation.

Lingo-1 est un composant du complexe NgR1/p75 et son inhibition favorise la croissance des neurites et des axones. En effet, le gène codant pour Lingo-1 a été cloné en 2003 par Carim-Todd et al., ("LRRN6A/LERN1 (leucine-rich repeat neuronal protein 1), a novel gene with enriched expression in limbic system and neocortex". 2003, Eur. J; Neurosci. 18 (12):3167-82, [1]) et c'est en 2004 que Mi et al. (Mi et al., "LINGO-1 is a component of the Nogo-66 receptor/p75 signaling complex". 2004, Nat Neurosci. 7(3):221-8, [2]) ont montré que Lingo-1 faisait partie intégrante du complexe récepteur NgR1/p75 et NgR1/TROY. Ces complexes, localisés dans la membrane des neurones, inhibent la régénération des neurites et des axones en présence de composants de la myéline (OMGp, Nogo et MAG) via l'activation de la kinase RhoA. Cela a été confirmé dans un modèle expérimental *in vivo* de régénération axonale chez le rat. Dans ce modèle, la moëlle épinière-est lésée et cela se traduit par une augmentation de l'expression de Lingo-1 et une forte apoptose des oligodendrocytes et des neurones entourant la lésion. Le traitement des rats lésés par un antagoniste de la fonction de Lingo-1, Lingo-1-Fc (une forme soluble de Lingo-1 capable de rentrer en compétition avec Lingo-1 endogène) permet des améliorations importantes de leur mobilité, une régénération conséquente des axones et une augmentation de la survie des neurones et des oligodendrocytes autour de la lésion (Ji et al., "LINGO-1 antagonist promotes functional recovery and axonal sprouting after spinal cord injury", 2006, Mol Cell Neurosci. 33(3):311-20, [3]).

Par ailleurs, Lingo-1, exprimée dans les oligodendrocytes ou les axones inhibe la différenciation des oligodendrocytes et la myélinisation (Mi et al., "Lingo-1 negatively regulates myelination by oligodendrocytes", 2005, Nature Neuroscience 8: 745- 751, [4]). Cela a été confirmé ensuite par Lee et al. ("NGF regulates the expression of axonal LINGO-1 to inhibit oligodendrocyte differentiation and myelination". 2007, J Neurosci. 27(1):220-5, [5]) et Zhao et al. ("An in vitro study on the involvement of LINGO-1 and Rho GTPases in Nogo-A regulated differentiation of oligodendrocyte precursor cells", 2007, Mol Cell Neurosci. 36(2):260-9, [6]). En effet, alors que l'utilisation d'antagonistes de Lingo-1 (Lingo-1-Fc, un anticorps anti-Lingo-1, DN-Lingo-1 (dominant négatif) correspondant à Lingo-1 sans son domaine cytoplasmique, RNAi de Lingo-1, knockout de Lingo-1) induisent une différenciation accrue des oligodendrocytes et la formation d'abondants feuillets de myéline, la surproduction de Lingo-1 entière par contre provoque des effets opposés. Lee et al. (2007) ([5]) ont de plus montré que l'expression de la protéine Lingo-1 au niveau des axones jouait un rôle aussi important dans la différenciation des oligodendrocytes adjacents et la myélinisation qu'une protéine Lingo-1 exprimée dans les oligodendrocytes. L'expression de Lingo-1 dans les axones est activée par le NGF et son récepteur TrkA. Afin de tester le rôle que pourrait jouer l'inhibition de Lingo-1 dans des maladies affectant la myélinisation telle que la sclérose en plaques, un modèle animal expérimental de la maladie appelé « encéphalomyélite autoimmune expérimentale murine induite par la MOG » (EAE) a été testé. L'utilisation d'un anticorps anti-Lingo-1 (antagoniste de Lingo-1) et de souris KO pour Lingo-1 dans ce modèle a permis de montrer que la diminution de la fonction de Lingo-1 était associée à une meilleure intégrité des axones et leur remyélinisation accrue (Mi et al., "Lingo-1 antagonist promotes spinal cord remyelination and axonal integrity in MOG-induced experimental autoimmune encephalomyelitis", 2007, Nature medicine 13: 1228-1233, [7]).

Par ailleurs, Lingo-1 inhibe la survie des neurones dopaminergiques (DA) et des cellules ganglionnaires de la rétine (RGC). Plusieurs travaux réalisés dans des modèles animaux ou des cultures de cellules à l'aide d'antagonistes de Lingo-1 ont montré que Lingo-1 jouait un rôle important dans la survie neuronale et plus particulièrement dans la survie des cellules des ganglions de la rétine (RGC) (détruites dans le glaucome) (Fu et al., "Blocking LINGO-1 function promotes retinal ganglion cell survival following ocular hypertension and optic nerve transection". Invest Ophthalmol Vis Sci. 2008, 49(3):975-85, [8] ; Fu et al., "Combined effect of brain-derived neurotrophic factor and Lingo-1 fusion protein on long-term survival of retinal ganglion cells in chronic glaucoma", 2009, Neuroscience 162: 375-382, [9]), dans les neurones du cervelet (Zhao et al. "Inactivation of glycogen synthase kinase-3beta and up-regulation of LINGO-1 are involved in LINGO-1 antagonist regulated survival of cerebellar granular neurons", 2008, Cell Mol Neurobiol. 28(5):727-35, [10]), ainsi que dans les neurones DA (impliqués dans la maladie de Parkinson) (Inoue et al., "Inhibition of the leucine-rich repeat protein Lingo-1 enhances survival, structure, and function of dopaminergic neurons in Parkinson's disease models", 2007, PNAS 104: 14430-14435, [11]). Dans les neurones DA de patients atteints de la maladie de Parkinson et dans des modèles expérimentaux de souris, il apparaît que Lingo-1 est surproduite. Lingo-1 inhibant l'EGFR dans ces neurones, et l'EGFR activant la voie Pl3k/Akt, l'action de Lingo-1 sur la survie neuronale pourrait provenir d'une inhibition de la voie Pl3k/Akt.

Dans le modèle animal du glaucome, il est connu que le BDNF (Brain-derived neurotrophic factor) est un important facteur de survie des RGC qui retarde leur mort. Fu et al. (2009) ([9]) ont récemment établi que l'effet conjugué de Lingo-Fc et de BDNF était beaucoup plus efficace que le BDNF seul et permettait une survie accrue à long terme des RGC. Lingo-1 étant capable d'interagir avec le récepteur du BDNF, le TrkB, et de l'inhiber, ce mécanisme moléculaire pourrait être celui impliqué dans la protection à long terme des RGC.

Les protéines Lingo, et en particulier Lingo-1, constitue une nouvelle cible thérapeutique particulièrement intéressante contre ces maladies puisque son inhibition engendre des effets neuroprotecteurs et stimule la synthèse de myéline. Des expériences menées *in vitro* et *in vivo* sur des modèles animaux expérimentaux ont validé l'hypothèse que l'inhibition de Lingo-1 était une approche innovante et prometteuse pour guérir plusieurs maladies du système nerveux, notamment la maladie de Parkinson et la sclérose en plaques.

Les ligands naturels des protéines Lingo, et en particulier de la protéine Lingo-1, sont actuellement inconnus. L'identification de nouvelles molécules chimiques antagonistes de Lingo-1 permettrait donc de développer de nouvelles thérapies contre ces deux maladies du système nerveux. Toutefois, aucune méthode permettant de mesurer la fixation de ligands de Lingo-1 n'est actuellement disponible. De plus, aucune méthode d'identification automatisable et adaptable au haut débit de ligands et d'antagonistes de Lingo-1 n'existe actuellement. Les seuls moyens actuels d'identifier des ligands ou des antagonistes de Lingo-1 sont des tests biologiques lourds à mettre en oeuvre sur des neurones granulaires du cervelet (Mi et al, 2004, [2]; Zho at al., 2008, [10]), des cellules ganglionnaires de la rétine (Fu et al, 2009, [9]), des oligodendrocytes (Mi et al, 2005, [4]) ou dans des modèles murins expérimentaux d'encéphalomyélite autoimmune (Mi et al, 2007, [7]) ou de la maladie de Parkinson (Inoue et al, 2007, [11]). Ces tests ne sont pas adaptables au haut débit.

C'est pourquoi la Demanderesse a cherché à mettre au point une méthode d'identification de ligands, et notamment d'antagonistes, de Lingo, et notamment Lingo-1, automatisable et adaptable au haut débit.

### DESCRIPTION DE L'INVENTION

L'invention permet précisément de pallier les inconvénients de l'art antérieur et de répondre à ces besoins.

La Demanderesse a observé, de manière surprenante et après d'importantes recherches, que Lingo-1 forme un dimère *in vivo.* Il était décrit dans la littérature que la partie extracellulaire de Lingo-1 était capable de se tétramériser (Mosyak et al., "The structure of Lingo-1 ectodomain, a module implicated in CNS repair inhibition", 2006, J. Biol. Chem. 281: 36378-36390, [12]). La Demanderesse a montré pour la première fois que la forme entière et membranaire de Lingo-1 forme un dimère *in vivo*.

La Demanderesse a ainsi mis au point un procédé d'identification d'un ligand d'une protéine choisie parmi Lingo-1, Lingo-2, Lingo-3 et Lingo-4, aisément automatisable et adaptable au haut débit, qui permettra de cribler de grandes librairies de molécules chimiques pour découvrir les inhibiteurs les mieux adaptés et les exploiter à des fins thérapeutiques.

Ce procédé est basé sur le principe que, lorsque Lingo, et notamment Lingo-1, interagit avec un ligand, la conformation du dimère de Lingo-1 change. Le procédé d'identification permet de détecter ce changement de conformation et d'identifier des ligands de Lingo-1.

Le procédé de l'invention comprend notamment les étapes suivantes :
a) incuber un système comprenant des produits de couplage formés d'un monomère d'une protéine choisie parmi Lingo-1, Lingo-2, Lingo-3 et Lingo-4 et d'une sonde susceptible d'émettre un signal détectable lors de changements conformationnels dudit monomère, et une molécule candidate,
b) détecter une modification du signal émis par au moins une des sondes, la modification du signal étant révélateur de la liaison de ladite molécule candidate à au moins un desdits produits de couplage.

L'invention se rapporte à un produit de couplage comprenant un monomère d'une protéine choisie parmi Lingo-1, Lingo-2, Lingo-3 et Lingo-4, et une sonde susceptible d'émettre un signal lors de changements conformationnels du monomère.

On entend par « monomère », au sens de la présente invention, une protéine composée d'une molécule polypeptidique unique. En d'autres termes, il s'agit d'un arrangement tridimensionnel d'atomes dans une seule chaîne polypeptidique. Avantageusement, les monomères sont capables de former des dimères. En d'autres termes, les monomères sont capables de s'associer entre eux pour former une molécule composée de deux monomères, c'est-à-dire de deux sous-unités monomériques. Il peut s'agir par exemple d'un homodimère, c'est-à-dire de deux monomères de la même protéine liés entre eux. Alternativement, il peut s'agir d'un hétérodimère, c'est-à-dire de deux monomères de deux protéines différentes liés entre eux. Avantageusement, le dimère peut être formé par polymérisation des monomères *in vivo,* dans des conditions appropriées. Par exemple, ces conditions peuvent être propres à la formation de ponts disulfure ou à celle d'interactions hydrophobes.

On entend par « Lingo-1, Lingo-2, Lingo-3 et Lingo-4 », au sens de la présente invention, toute protéine possédant substantiellement la séquence de la protéine sauvage. Il peut s'agir d'une protéine possédant tout ou partie de la protéine sauvage, ou une protéine possédant une ou plusieurs mutations, par exemple par addition, délétion ou subtitution. Il peut s'agir d'une protéine humaine ou non-humaine, par exemple une protéine murine, équine, par exemple de cheval, porcine, de lamin, de rat, ovine, par exemple de mouton, bovine, par exemple de vache, de singe, par exemple de macaque.

Dans le cas où la protéine est Lingo-1, il peut s'agir de la protéine humaine sauvage, notamment composée de 580 résidus, comprenant une région extracellulaire (516 acides aminés) constituée de 12 motifs LRR (leucine-rich repeat) et d'un domaine Ig (immunoglobuline) ; et une portion cytoplasmique de 38 résidus. Avantageusement, la séquence du monomère de Lingo-1 peut être la séquence SEQ ID NO : 1. Il peut s'agir de la séquence décrite dans Genbank (http://www.ncbi.nlm.nih.gov/Genbank/) par le numéro d'accession NP_116197.4. Il peut également s'agir de la protéine issue de la séquence polynucléotidique correspondant à l'ARNm décrite par la séquence SEQ ID NO : 5, ou par la séquence NM_032808 dans Genbank.

Dans le cas où la protéine est Lingo-2, il peut s'agir de la protéine humaine sauvage, notamment composée de 606 acides aminés, et comprenant un domaine extracellulaire composé de 515 acides aminés, et une portion cytoplasmique de 40 résidus. Avantageusement, la séquence peut être la séquence SEQ ID NO : 2. Il peut s'agir de la séquence décrite dans Genbank par le numéro d'accession NP_689783.1. Il peut également s'agir de la protéine issue de la séquence polynucléotidique correspondant à l'ARNm décrite par la séquence SEQ ID NO : 6, ou par la séquence NM_152570 dans Genbank.

Dans le cas où la protéine est Lingo-3, il peut s'agir de la protéine humaine sauvage. Avantageusement, la séquence peut être la séquence SEQ ID NO : 3. Il peut s'agir de la séquence décrite dans Genbank par le numéro d'accession NP_001094861. Il peut également s'agir de la protéine issue de la séquence polynucléotidique correspondant à l'ARNm décrite par la séquence SEQ ID NO : 7, ou par la séquence NM_001101391 dans Genbank.

Dans le cas où la protéine est Lingo-4, il peut s'agir de la protéine humaine sauvage, notamment composée de 593 acides aminés. Avantageusement, la séquence peut être la séquence SEQ ID NO : 4. Il peut également s'agir de la protéine issue de la séquence polynucléotidique correspondant à l'ARNm décrite par la séquence SEQ ID NO : 8, ou par la séquence NM_001004432 (sequence humaine), ou la séquence murine NP 796224 dans Genbank..

Avantageusement, l'interaction du monomère ou du dimère avec un ligand de la protéine peut provoquer un changement d'une caractéristique physique du monomère et/ou du dimère. Il peut s'agir par exemple de la conformation spatiale du monomère et/ou dimère.

On entend par « changement conformationnel du monomère », au sens de la présente invention, toute modification spatiale du monomère. Avantageusement, cette modification peut résulter de l'interaction du monomère et/ou du dimère avec un ligand.

On entend par « sonde susceptible d'émettre un signal », au sens de la présente invention, toute molécule capable de produire un effet lors du changement d'une caractéristique physique du monomère, notamment lors du changement conformationnel du monomère. Avantageusement, il peut s'agir d'un marqueur détectable sensible d'un point de vue conformationnel. Avantageusement, l'interaction du ligand avec le monomère ou le dimère provoque un changement conformationnel du monomère ou du dimère, ce changement impliquant l'émission d'un signal détectable.

Avantageusement, la sonde peut être une molécule choisie parmi une étiquette chimique, par exemple un anticorps, une molécule luminescente ou une molécule fluorescente, un fragment intégré dans le monomère, par exemple un site de clivage de protéase, ou un fragment immunodétectable.

On entend par « molécule luminescente », au sens de la présente invention, toute molécule possédant la propriété de restituer sous forme de photons d'énergie d'origine non thermique une partie de l'énergie absorbée au cours d'une excitation. Il s'agit donc de la désactivation d'une molécule excitée vers un état énergétique moins élevé. En d'autres termes, une molécule luminescente est une molécule capable d'agir sur une substance appropriée pour générer de la luminescence.

La molécule luminescente peut par exemple être une protéine, ou un composé chimique. Avantageusement, la protéine luminescente possède la propriété d'émettre une lumière bleue, jaune ou verte. La protéine peut être choisie parmi celles connues de l'homme du métier, décrites par exemple dans les documents Kamal et al., « Improved donor/acceptor BRET couples for monitoring beta-arrestin recruitment to G protein-coupled receptors ». Biotechnol J. 2009 Sep;4(9):1337-44, [17] ; Kocan M et al., « Demonstration of improvements to the bioluminescence resonance energy transfer (BRET) technology for the monitoring of G protein-coupled receptors in live cells", J Biomol Screen. 2008 Oct;13(9):888-98, [18]; Michelini E et al., « Luminescent probes and visualization of bioluminescence », Methods Mol Biol. 2009;574:1-13, [19]). Par exemple, la protéine luminescente peut être la luciférase. La luciférase peut être choisie parmi des molécules connues de l'homme du métier, par exemple celles citées dans WO 01/046691. Il peut s'agir par exemple de la *Renilla* luciferase, la RLuc2, la RLuc8, la firefly luciferase, la Gaussia luciferase ou l'Aequorin, ainsi que leurs mutants ou dérivés. S'il s'agit de la Renilla luciférase, il peut s'agir d'une protéine codée par la séquence SEQ ID NO : 12.

Par exemple, la protéine luminescente peut être la Renilla luciferase, et le monomère peut être Lingo-1. Dans ce cas, le produit de couplage peut avoir la séquence peptidique SEQ ID NO : 14.

On entend par « signal », au sens de la présente invention, tout effet physique ou chimique. Il peut s'agir par exemple d'un signal lumineux, par exemple fluorescent, luminescent, colorimétrique, électrique, cette liste n'étant pas limitative.

On entend par « molécule fluorescente », au sens de la présente invention, toute molécule possédant la propriété d'absorber de l'énergie lumineuse (lumière d'excitation) et de la restituer rapidement sous forme de lumière fluorescente, par émission d'un photon de manière très rapide (lumière d'émission). Une fois l'énergie du photon absorbée, la molécule se trouve alors généralement dans un état électroniquement excité. En d'autres termes, il peut s'agir d'un fluophore ou d'un fluorochrome.

Avantageusement, la protéine luminescente ou fluorescente peut être une protéine capable d'émettre une lumière bleue, jaune ou verte.

De manière particulièrement avantageuse, la protéine luminescente ou fluorescente peut être une protéine fluorescente capable d'émettre une lumière jaune ou verte. La protéine fluorescente peut par exemple être choisie parmi la GFP (« Green Fluorescent Protein » ou « Protéine fluorescente verte »), la YFP (« Yellow Fluorescent Protein » ou « Protéine fluorescente jaune »), Enhanced Yellow Fluorescent Protein (eYFP), eGFP, GFP2 , GFP10, RGFP (Renilla Green Fuorescent Protein), YPet, leurs mutants ou dérivés tels que décrits dans le document WO 01/46691. S'il s'agit de la eYFP, il peut s'agir d'une protéine codée par la séquence SEQ ID NO : 9.

Par exemple, la protéine fluorescente peut être la eYFP, et le monomère peut être Lingo-1. Dans ce cas, le produit de couplage peut avoir la séquence peptidique SEQ ID NO : 11.

On entend par « produit de couplage », au sens de la présente invention, tout produit dans lequel le monomère et la sonde sont liés entre eux. En d'autres termes, le produit de couplage peut être une combinaison du monomère et de la sonde entre eux. Encore en d'autres termes, le produit de couplage peut être un conjugué du monomère et de la sonde entre eux.

Avantageusement, la sonde et le monomère peuvent être couplés par une ou plusieurs liaisons stables dans les milieux biologiques. En d'autres termes, la sonde et le monomère peuvent être liés par une ou plusieurs liaisons qui ne s'altèrent pas au cours du temps dans le milieu dans lequel le produit de couplage est conservé ou produit. Avantageusement, le milieu peut être un milieu cellulaire, un milieu utilisé dans le cadre d'opérations *in vitro* ou *in vivo*.

La sonde et le monomère peuvent être liés de manière covalente. A cet égard, la liaison peut être une liaison entre deux atomes résultant de la mise en commun de deux électrons provenant séparément de chacun d'eux.

La sonde et le monomère peuvent former une protéine de fusion, c'est-à-dire une protéine artificielle obtenue par la combinaison de différentes protéines, ou partie de protéines.

Le produit de couplage peut être obtenu par des techniques connues de l'homme du métier, par exemple par expression par un organisme hôte d'une molécule d'acide nucléique codant pour le produit de couplage, ou par synthèse *in vitro* du peptide et couplage après ligation chimique. Par exemple, s'il s'agit d'une protéine de fusion, le produit de couplage peut être obtenu suite à la création par recombinaison de l'ADN d'un gène comportant les phases ouvertes de lectures correspondant à la sonde et au monomère, insertion du gène dans un vecteur d'expression, insertion du vecteur d'expression dans une cellule hôte, production de la protéine de fusion correspondante par la cellule hôte et purification de la protéine de fusion.

L'invention se rapporte également à une molécule d'acides nucléiques isolée et purifiée codant pour un produit de couplage tel que défini précédemment.

On entend par « molécule d'acides nucléiques », au sens de la présente invention, toute molécule constituée d'un enchaînement de nucléotides. Il peut s'agir par exemple d'acide désoxyribonucléique (ADN), d'acide ribonucléique (ARN), d'ARN messager (ARNm) ou d'ADN complémentaire (ADNc).

On entend par « isolé », au sens de la présente invention, le fait d'être séparé du milieu naturel. Avantageusement, l'acide nucléique peut subir les manipulations de routine réalisées dans le cadre de la technologie de l'ADN recombinant, par exemple le séquençage, la digestion par des enzymes de restriction, la mutagenèse, le clonage dans des vecteurs d'expression.

On entend par « purifié », au sens de la présente invention, la caractéristique d'être séparé substantiellement des contaminants.

La molécule d'acides nucléiques peut comprendre une séquence d'acides nucléiques codant pour une protéine luminescente ou fluorescente telle que définie précédemment, et une séquence d'acides nucléiques codant pour un monomère tel que défini précédemment. Avantageusement, la molécule d'acides nucléiques peut comprendre la séquence SEQ ID NO : 10 (le monomère étant Lingo-1 et la protéine étant eYFP) ou la séquence SEQ ID NO : 13 (le monomère étant Lingo-1 et la protéine étant la Renilla luciférase).

La molécule d'acides nucléiques peut être obtenue par des techniques connues de l'homme du métier. Elle peut notamment être construite artificiellement et obtenue par synthèse chimique ou génie génétique, par exemple comme décrit dans Lewin B. : Genes VI, De Boeck University, 6th édition, Chapter 20, 1999. Elle peut par exemple être obtenue par recombinaison de l'ADN d'un gène comportant les phases ouvertes de lectures correspondant à la sonde et au monomère. A titre indicatif, l'ADN recombinant peut être inséré dans un organisme hôte d'expression, tel que notamment une bactérie, par un vecteur d'expression, notamment un plasmide bactérien ou un bactériophage.

L'invention se rapporte également à un vecteur d'expression comprenant une molécule d'acides nucléiques telle que définie précédemment.

On entend par « vecteur d'expression », au sens de la présente invention, toute molécule possédant une structure permettant à une molécule d'acides nucléiques possédant une séquence codante d'être transcrite ou traduite en protéine. Avantageusement, il peut comprendre au moins une des séquences suivantes : un promoteur, une séquence d'initiation de la transcription, une séquence de terminaison de la transcription, comme une séquence de polyadénylation, un gène de sélection, un enhancer, une séquence régulatrice, une séquence inductible.

Le vecteur peut être tout vecteur connu de l'homme du métier, comme par exemple un virus, comme un adénovirus ou un rétrovirus, un plasmide, un bactériophage, un cosmide, un phagemide. Il peut s'agir par exemple de vecteur disponibles dans le commerce, comme les plasmides p3xFlag (Sigma), pCDNA₃ (Invitrogen) ou peYFP-N1 (Clontech).

Un tel vecteur peut être préparé selon des techniques connues de l'homme du métier, comme décrit par exemple Lewin B. : Genes VI, De Boeck University, 6th édition, Chapter 20,1999.

L'invention se rapporte également à une cellule hôte comprenant au moins une molécule d'acides nucléiques telle que définie précédemment ou un vecteur d'expression tel que défini précédemment.

On entend par « cellule hôte », au sens de la présente invention, tout organisme possédant la capacité de produire une protéine lorsqu'un vecteur d'expression codant pour la protéine y est introduit. Avantageusement, la cellule hôte peut exprimer le produit de couplage lorsqu'un vecteur d'expression codant pour le produit de couplage y est introduit.

Avantageusement, la cellule hôte peut exprimer au moins un produit de couplage tel que défini précédemment. Par exemple, la cellule hôte peut exprimer un produit de couplage comprenant la Renilla luciférase et Lingo-1, ou un produit de couplage comprenant la eYFP et Lingo-1.

Avantageusement, la cellule hôte peut comprendre deux molécules d'acides nucléiques ou un vecteur d'expression. Par exemple, la cellule hôte peut comprendre et exprimer une molécule d'acide nucléique codant pour un produit de couplage comprenant une protéine luminescente et un monomère, et une molécule d'acide nucléique codant pour un produit de couplage comprenant une protéine fluorescente et un monomère. Par exemple, la cellule hôte peut comprendre et exprimer une molécule d'acide nucléique codant pour un produit de couplage comprenant la Renilla luciférase et un monomère de Lingo-1, et une molécule d'acide nucléique codant pour un produit de couplage comprenant la eYFP et un monomère de Lingo-1.

Avantageusement, les produits de couplage peuvent former des dimères dans la cellule hôte. En effet, la Demanderesse a montré que, *in vivo,* les monomères exprimés dans les cellules forment des dimères.

La cellule hôte peut être tout organisme adaptée à la production de protéines recombinantes connu de l'homme du métier. Il peut s'agir d'une cellule eucaryote ou procaryote. Par exemple, il peut s'agir de YB2/0 (ATCC CRL-1662), CHO-K1 (ATCC CCL-61), par exemple HEK 293 (ATCC CRL-1573), BHK (ATCC CRL-12072) ou COS (ATCC CRL-1650), PC12 (ATCC-CRL-1721) ou SH-SY5Y (ATCC- CRL-2266) ou Hela (ATCC-CCL-2).

La cellule hôte peut être produite par insertion d'un vecteur d'expression tel que défini précédemment ou d'une molécule d'acides nucléiques telle que définie précédemment, dans la cellule hôte, au moyen de techniques connues de l'homme du métier. On peut citer par exemple l'électroporation, ou le phosphate de clacium, décrit dans le document " Molecular Cloning: A Laboratory Manual 2nd édition" (1989), Cold Spring Harbor Laboratory Press , " Current Protocols in Molecular Biology" (1987), John Wiley & Sons, Inc. ISBNO-471-50338-X, and the like, an electroporation method described in " Methods in Electroporation: Gene Pulser/E. coli Pulser System" Bio-Rad Laboratories (1993).

La cellule hôte peut être cultivée dans tout milieu approprié connu de l'homme du métier. On peut citer par exemple du milieu DMEM (Dulbecco's Modified Eagle Medium, InvitrogenTM), Ham's F-12, Minimal Essential Medium Eagle (MEM), Neuronal Base Médium (PAA Laboratories).

Le produit de couplage peut être purifié par tout moyen connu de l'homme du métier, par exemple par immunopurification ou par chromatographie d'affinité.

Un premier objet de l'invention se rapporte à un système comprenant
a) un premier produit de couplage comprenant un monomère d'une protéine choisie parmi Lingo-1, Lingo-2, Lingo-3 et Lingo-4, et une sonde susceptible d'émettre un signal détectable qui est modifié lors de changements conformationnels du monomère, tel que défini précédemment,
b) un deuxième produit de couplage comprenant un monomère d'une protéine choisie parmi Lingo-1, Lingo-2, Lingo-3 et Lingo-4, et une sonde susceptible d'émettre un signal détectable qui est modifié lors de changements conformationnels du monomère, tel que défini précédemment, dans lequel les premier produit de couplage et deuxième produit de couplage forment un dimère, et dans lequel un changement d'interaction entre les monomères provoquent un changement de signal.

On entend par « système » au sens de la présente invention, un dimère (i.e., complexe de protéines fait de deux sous-unités très proches l'une de l'autre du fait de l'interaction intermoléculaire) résultant de couplages formés d'un monomère d'une protéine choisie parmi Lingo-1, Lingo-2, Lingo-3 et Lingo-4 et d'une sonde susceptible d'émettre un signal détectable, ledit signal étant modifié lors de changements conformationnels dudit monomère et/ou dimère, par toute technique adaptée connue par l'homme du métier, par exemple un système adapté à une détection par transfert d'énergie par résonance bioluminescente (BRET pour Bioluminescence Resonance Energy Transfer), par transfert d'énergie par résonance fluorescente (FRET pour Fluorescence Resonance Energy Transfer), TR-FRET (Time-resolved-FRET) ou HTRF (homogeneous Time Resolved Fluorescence). De préférence, il s'agit d'un système de détection par transfert d'énergie par résonance bioluminescente (BRET pour Bioluminescence Resonance Energy Transfer). Ledit système de l'invention peut se présenter sous la forme d'une suspension cellulaire, d'une culture de cellules adhérentes, de membranes ou tissus, etc... exprimant le couple donneur/accepteur.

On entend par « dimère », au sens de la présente invention, toute molécule composée de deux monomères. Avantageusement, la distance entre les monomères composant le dimère est inférieure à 150 Å, par exemple inférieure à 100 A, avantageusement inférieure à 75 Å ou à 50 Å.

On entend par « changement d'interaction », au sens de l'invention, toute modification physique ou structurelle entre les monomères. Il peut s'agir notamment d'une modification spatiale ou conformationnelle des monomères. Avantageusement, ce changement peut résulter de la liaison d'un ligand sur le dimère ou sur au moins un des monomères, ou de la proximité d'un ligand sur le dimère ou sur au moins un des monomères.

On entend par « ligand », au sens de la présente invention, toute molécule se liant au produit de couplage. Avantageusement, cette liaison peut se produire lorsque le produit de couplage est sous forme de dimère. Le ligand peut être une hormone, un neurotransmetteur, un composé chimique, un médicament, un agent de diagnostique, un anticorps, une séquence peptidique. Il peut s'agir d'un agoniste ou d'un antagoniste, total ou partiel. Avantageusement, un ligand peut être capable de lier un monomère ou un dimère avec une affinité comprise entre 1 µM et 1 pM, préférentiellement entre 1 nM et 1 pM. Avantageusement, les ligands peuvent traiter ou prévenir la maladie de Parkinson ou la sclérose en plaques, les glaucomes ou les maladies impliquant la myéline.

La Demanderesse a en effet montré d'une part, que le produit de couplage, et plus particulièrement le monomère, se dimérise, notamment *in vivo,* et d'autre part, que la liaison d'un ligand au dimère peut provoquer un changement conformationnel du dimère.

Avantageusement, le changement conformationnel provoque un changement d"émission d'un signal par une sonde. Avantageusement, le signal est détectable par toute technique adaptée connue par l'homme du métier. Avantageusement, le système peut être adapté à la détection par transfert d'énergie par résonance bioluminescente (BRET pour Bioluminescence Resonance Energy Transfer), un système de transfert d'énergie par résonance fluorescente (FRET pour Fluorescence Resonance Energy Transfer), TR-FRET (Time-resolved-FRET) ou l'HTRF (homogeneous Time Resolved Fluorescence).

Par exemple, le système de l'invention peut être mis en oeuvre dans le cadre de la détection par BRET, selon un protocole connu de l'homme du métier. On peut citer par exemple Xu et al. « A bioluminescence resonance energy transfer (BRET) system: application to interacting circadian clock proteins » (1999) Proc. Natl. Acad. Sci. USA. 96, 151-156 [13] ; Angers et al. « Détection of beta 2-adrenergic receptor dimerization in living cells using bioluminescence resonance energy transfer (BRET)" (2000) Proc.Natl. Acad. Sci. USA 97 : 3684-3689 [14]). Notamment, le BRET peut être mis en oeuvre au moyen d'un produit de couplage comprenant Rluc et Lingo-1, et un produit de couplage comprenant eYFP et Lingo-1. Rluc est une enzyme qui produit de la lumière bleue en présence d'un substrat, par exemple la coélenterazine ou un de ses dérivés disponible dans le commerce (Molecular Probes Eugene OR, Biosynth Napierville IL). Quand l'orientation spatiale est appropriée, eYFP est capable d'absorber une portion de la lumière bleue et émet en conséquence une lumière de longueur d'onde différente, dans le jaune. En d'autres termes, en présence de coélenterazine, eYFP émet une lumière jaune si Rluc et eYFP sont suffisamment proches l'un de l'autre. Par « suffisamment proches » on entend, au sens de la présente invention, à une distance inférieure à 150 Å, ou encore inférieure à 100 Å, et avantageusement inférieure à 75 Å ou 50 Å. Si Rluc et eYFP sont trop éloignés, l'énergie n'est pas transférée efficacement et seule la lumière bleue de Rluc est détectée. Or, la disposition spatiale de Rluc et eYFP l'un par rapport à l'autre est dépendante de la disposition spatiale des monomères l'un par rapport à l'autre. Si une molécule se lie à un monomère ou au dimère, alors la conformation spatiale du dimère change. Notamment, les monomères se rapprochent ou s'éloignent. Lorsque les deux monomères, et notamment Rluc et eYFP sont suffisamment proches, eYFP peut absorber une portion de la lumière bleue émise par Rluc et émettre une longeur d'onde dans le jaune. Ainsi, pour détecter si une molécule se lie au dimère ou au monomère, on détermine l'efficacité du transfert d'énergie, par mesure du rendement lumineux à la longueur d'onde du donneur (Rluc) et à la longueur d'onde de l'accepteur (eYFP) après addition de coélenterazine. Par ailleurs, le ratio « rendement lumineux de l'accepteur/rendement lumineux du donneur » est calculé pour quantifier le BRET.

Alternativement, le système de l'invention peut être mis en oeuvre dans le cadre de la détection par FRET, selon un protocole connu de l'homme du métier. On peut citer par exemple US 7183066, (Kroeger et al., « Constitutive and agonist-dependent homo-oligomerization of the thyrotropin-releasing hormone receptor » (2001) J Biol Chem, 276, 12736-12743, [20]). Par exemple, un fluorophore donneur et un chromophore accepteur connus de l'homme du métier peuvent être mis en oeuvre, par exemple au moyen des couples fluorescéine/rhodamine ou CFP (cyan fluorescent protein)/YFP.

Avantageusement, le système de l'invention peut être mis en oeuvre dans le cadre de la détection par TR-FRET, selon un protocole connu de l'homme du métier, par exemple décrit dans Maurel D et al., "Cell-surface protein-protein interaction analysis with time-resolved FRET and snap-tag technologies: application to GPCR oligomerization" (2008), Nat Methods (6):561-7, [21]). Par exemple, il est possible d'améliorer le signal sur bruit du FRET en s'affranchissant d'une partie des signaux parasites grâce à une lecture en temps résolu. Ceci est possible grâce à l'utilisation de fluorophores à durée de vie longue tels que les chélates ou cryptates de terre rare.

Avantageusement, le système de l'invention peut être mis en oeuvre dans le cadre de la détection par HTRF®, selon un protocole connu de l'homme du métier, comme par exemple celui de Hermand P et al, "Functional adhesiveness of the CX3CL1 chemokine requires its aggregation. Role of the transmembrane domain" (2008) J Biol Chem 283(44):30225-34;, ([22]), ou Whitfield J et al. "High-throughput methods to detect dimerization of Bcl-2 family proteins" (2003), Anal Biochem, 322(2):170-8 ([23]). Par exemple, des anticorps marqués par un donneur de fluorescence comme le Cryptate d'europium ou un accepteur de fuorescence comme un pigment de phycobilliprotéine purifiée à partir d'une algue rouge (par exemple XL665) reconnaissent des étiquettes sur chacun des monomères. L'interaction des monomères est détectée par transfert d'énergie, par exemple par excitation à 337 nm et émission à 665 nm). Le système peut être obtenu par co-transfection d'une cellule avec deux vecteurs d'expression tels que définis précédemment. Le système peut par exemple être mis en oeuvre *in vitro,* par exemple sur des plaques.

Un autre objet de l'invention se rapporte à l'utilisation d'un système tel que défini précédemment, pour identifier des ligands d'une protéine choisie parmi Lingo-1, Lingo-2, Lingo-3 et Lingo-4.

Un autre objet de l'invention se rapporte à l'utilisation d'un système tel que défini précédemment, pour détecter des changements de conformation d'une protéine choisie parmi Lingo-1, Lingo-2, Lingo-3 et Lingo-4.

Un autre objet de l'invention se rapporte à un procédé d'identification d'un ligand d'une protéine choisie parmi Lingo-1, Lingo-2, Lingo-3 et Lingo-4, comprenant les étapes suivantes :
a) incuber un système tel que défini précédemment et une molécule candidate, ledit système comprenant :
   (i) un premier produit de couplage comprenant un monomère d'une protéine choisie parmi Lingo-1, Lingo-2, Lingo-3 et Lingo-4, et une sonde susceptible d'émettre un signal détectable qui est modifié lors de changements conformationnels dudit monomère,
   (ii) un deuxième produit de couplage comprenant un monomère d'une protéine choisie parmi Lingo-1, Lingo-2, Lingo-3 et Lingo-4, et une sonde susceptible d'émettre un signal détectable qui est modifié lors de changements conformationnels dudit monomère,
b) détecter un signal émis par au moins une des sondes,
le signal étant révélateur de la liaison de ladite molécule candidate à au moins un des produits de couplage.

On entend par « incuber », au sens de la présente invention, le fait de mettre en présence le premier produit de couplage, le deuxième produit de couplage, et la molécule candidate. Avantageusement, l'incubation est réalisée dans des conditions permettant la dimérisation des monomères des premier et deuxième produits de couplage. Avantageusement, l'incubation est réalisée dans des conditions connues permettant la liaison de la molécule candidate sur au moins un des monomères ou sur le dimère, si la molécule candidate est un ligand de la protéine. On entend par « molécule candidate » toute molécule à tester. Il peut s'agir par exemple d'une protéine, d'un peptide, d'une molécule chimique, d'un anticorps, cette liste n'étant pas limitative. La molécule peut par exemple être issue d'une banque de protéines, d'une suspension cellulaire, d'une chimiothèque, cette liste n'étant pas limitative. La molécule peut être un ligand de Lingo-1, Lingo-2, Lingo-3 ou Lingo-4.

Avantageusement, dans le cas où la molécule candidate n'est pas un ligand de la protéine, le signal de BRET, résultant de la formation du dimère n'est pas modifié suite à l'ajout de la molécule candidate. Avantageusement, il n'y a dans ce cas aucune modification d'une propriété physique des monomères, du dimère, des sondes ou des produits de couplage.

Avantageusement, les variations du signal BRET après ajout d'un ligand pourront être suivies au cours du temps, pendant 30 minutes par exemple.

Avantageusement, dans le cas où la molécule candidate est un ligand de la protéine, le signal de BRET, résultant de la formation du dimère est modifié, par exemple augmenté ou diminué, suite à l'ajout de la molécule candidate. Avantageusement, il y a dans ce cas une modification d'au moins une propriété physique d'au moins un monomère, du dimère, des sondes ou des produits de couplage. Avantageusement, la liaison de la molécule candidate provoque une modification conformationnelle d'au moins un monomère, du dimère, des sondes ou des produits de couplage.

Avantageusement, le signal peut être détecté dans le cadre d'un système BRET, FRET, TR-FRET ou l'HTRF.

Avantageusement, le procédé de l'invention peut comprendre en outre une étape d'addition d'un substrat permettant au signal de se produire. Il peut s'agir par exemple de coélenterazine ou d'un dérivé de coélenterazine, par exemple la coelenterazine 400a, la DeepBlueC. Le substrat peut être ajouté de toute manière connue de l'homme du métier, par exemple de manière exogène, ou ajouté sous forme d'acide nucléique codant pour le substrat.

Avantageusement, le procédé peut comprendre toute étape ou condition nécessaire à la mise en oeuvre d'un procédé BRET, FRET, TR-FRET ou l'HTRF. Ces étapes sont bien connues de l'homme du métier, et peuvent comprendre une étape de mesure du signal, de calcul des longueurs d'onde d'émission et d'absorption, d'utilisation de filtres pour discriminer les longueurs d'ondes, de calcul d'intensité de longueurs d'ondes au moyen par exemple d'un tube photomultiplicateur ou d'une caméra CDD (Charged Coupled Device), du calcul d'efficacité du transfert d'énergie, de détection des pics d'émission, d'utilisation d'instruments du type BRETCount, Mithras (Berthold) de compteurs microplaques de scintillation et de luminescence (Berthold, Packard Instruments).

Le procédé peut être mis en oeuvre *in vitro* ou *in vivo.* Avantageusement, le procédé peut être mis en oeuvre sur des cellules vivantes. Par exemple, le procédé peut être mis en oeuvre avec des lignées de cellules transfectées de façon stable ou transitoire, dans des conditions physiologiques.

Avantageusement, le procédé peut être mis en oeuvre sur des préparations de membranes, éventuellement préparées à l'avance et congelées ou sur des protéines purifiées.

Avantageusement, le procédé peut comprendre en outre un système de criblage à haut-débit. On entend par « système de criblage à hait débit », au sens de la présente invention, tout moyen d'automatisation permettant d'accélérer le procédé d'identification. Il peut s'agir de tout moyen de l'homme du métier, impliquant par exemple l'informatique, la bio-informatique, la génomique, la protéomique, la robotique, et parfois les nanotechnologies, cette liste n'étant pas limitative.

Avantageusement, le procédé peut comprendre en outre un test permettant d'identifier les ligands agonistes (activateurs) et les ligands antagonistes (inhibiteurs) des protéines. Il peut s'agir par exemple d'un test biologique. Il peut s'agir de tout test permettant d'identifier l'impact d'une molécule candidate sur au moins une voie de signalisation dans laquelle la protéine joue un rôle, par exemple la différenciation des oligodendrocytes, comme mis en oeuvre par exemple dans Mi et al., 2005, ([4]), la myélinisation des axones, la survie des neurones dopaminergiques et des cellules ganglionnaires de la rétine, comme mis en oeuvre par exemple dans Fu et al., 2009, ([9]).

Avantageusement, le procédé peut permettre de déterminer si, en aval, des voies de signalisation cellulaires impliquant les protéines kinases RhoA ou Akt sont activées ou inhibées par le ligand. L'effet des ligands peut par exemple être testé sur la voie Rho (test RhoA commercialisé par Pierce) ou sur la voie Akt (test par AlphaScreen de Perkin ou par analyse de western blot à l'aide d'anticorps spécifiques de Cellsignaling).

Avantageusement, le procédé peut permettre de déterminer si des ligands perturbent l'interaction entre la protéine et les récepteurs membranaires NgR1, EGFR, TrkB. A cet égard, l'interaction entre Lingo-1 et NgR ; Lingo1-EGFR et Lingo1/TrkB peut être mesurée.

L'invention se rapporte également à un dispositif, appelé également kit, pour la mise en oeuvre du procédé tel que défini ci-dessus, pouvant permettre d'étudier au moins une interaction décrites ci-avant. Le kit peut comporter tout ou partie des éléments nécessaire à la mise en oeuvre du procédé, par exemple les produits de couplage, substrats, au moins un moyen de détection ou de mesure du signal BRET, cette liste n'étant pas limitative. Le kit peut par exemple contenir les plasmides des protéines de fusion, par exemple Lingo1-RLuc, Lingo1-YFP, Lingo2-RLuc, Lingo2-YFP, Lingo3-RLuc, Lingo3-YFP, Lingo4-RLuc, Lingo4-YFP, NgR-RLuc, NgR-YFP, EGFR-RLuc, EGFR-YFP, Trkb-RLuc, Trkb-YFP, ainsi qu'un substrat, par exemple la coelenterazine.

### BRÈVE DESCRIPTION DES FIGURES

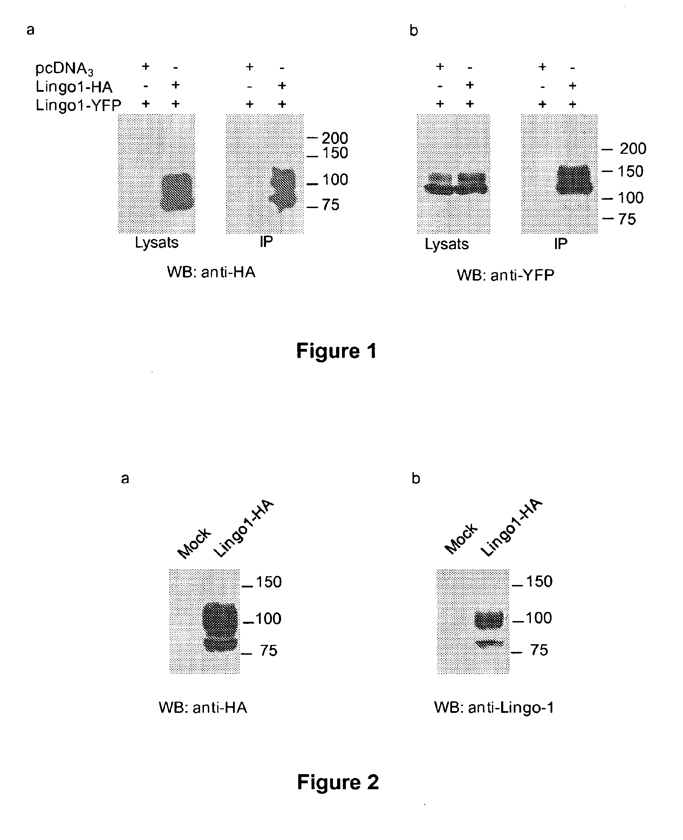

La figure 1 représente des expériences de co-immunoprécipitation montrant que Lingo-1 forme un dimère dans les cellules-HEK-293. Les cellules HEK-293 sont cotransfectées (signe +) avec 5µg des plasmides de pcDNA3, Lingo1-HA ou Lingo1-YFP. Une co-immunoprécipitation est réalisée avec des billes d'agarose couplées à un anticorps anti-HA (cf. partie a, montrant le Western Blot ou WB anti-HA) ou un anticorps anti-YFP (cf. partie b, montrant le Western Blot ou WB anti-YFP). L'incubation de la membrane correspondante avec un anticorps anti-HA montre que la protéine Lingo-HA a bien été immunoprécipitée (a). Deux bandes immunoréactives apparaissent spécifiquement à environ 120kDa et 140kDa et témoignent de la présence de Lingo-YFP dans l'immunoprécipité (b).
La figure 2 représente la validation de l'anticorps anti-Lingo-1. Les lysats cellulaires provenant de cellules HEK-293 transfectées avec un plasmide contrôle, le pcDNA₃ (mock), ou le plasmide codant pour la protéine Lingo-1 fusionnée en N-terminal à une étiquette HA sont analysés sur gel d'acrylamide (8%). L'utilisation d'un anticorps anti-HA permet de détecter deux bandes immunoréactives à 80 et 100 kDa, correspondant probablement à deux états de glycosylation différents (cf. partie a, montrant le Western Blot ou WB anti-HA). La présence des ces deux mêmes bandes immunoréactives peut être également observés lorsque les lysats cellulaires sont incubés avec un anticorps spécifique de Lingo-1 (cf. partie b, montrant le Western Blot ou WB anti-YFP).

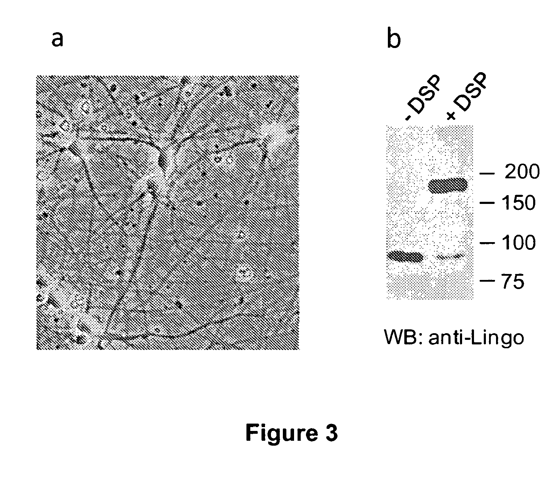

La figure 3 représente la détection de dimères de Lingo-1 dans des neurones de cortex de rat en culture. Douze jours après la mise en culture, les neurones de cortex émettent des prolongements et forment un réseau (cf. partie a). Les neurones sont traités (+ DSP) ou non (-DSP) avec un agent pontant, le DSP (cf partie b : Western Blot ou WB anti-Lingo-1b). Lingo-1 est bien exprimé dans les neurones de cortex comme l'atteste la présence d'une bande immunoréactive à 90 kDa lorsque le western blot est incubé en présence de l'anticorps anti-Lingo-1 (piste : -DSP). Lorsque les neurones sont traités avec le DSP, une bande immunoréactive supplémentaire apparaît à 180 kDa correspondant à la forme dimérique de Lingo-1, ceci au détriment du monomère à 90 kDa qui disparaît (piste : DSP).

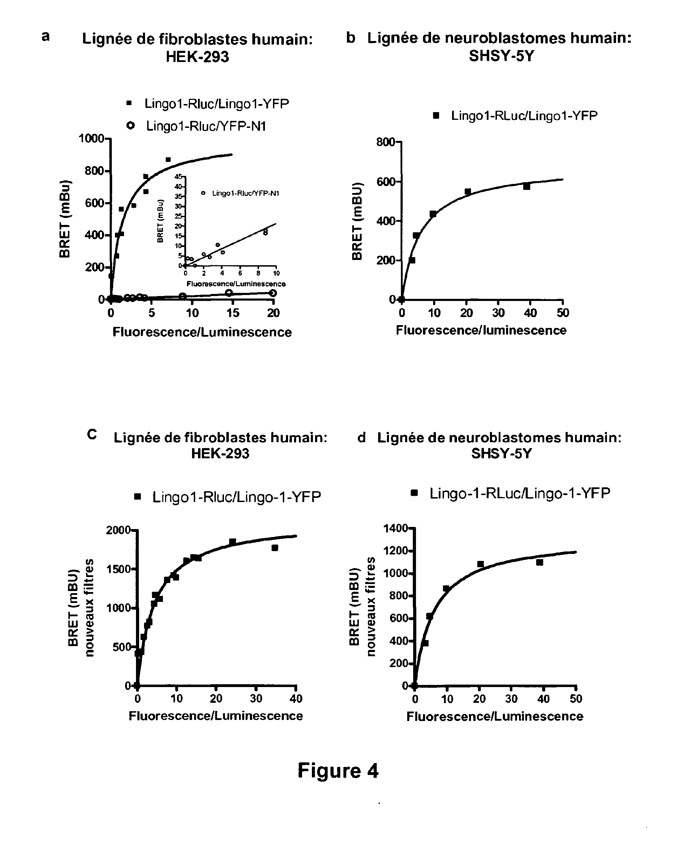

La figure 4 représente la détection de l'oligomérisation de la protéine Lingo-1 dans des cellules vivantes en utilisant la technique du BRET. Les cellules HEK-293 (partie a et c) et SHSY-5Y (partie b et d) ont été transfectées avec une concentration fixe de donneur (100ng de plasmide Lingo1-RLuc) et des concentrations croissantes d'accepteurs, Lingo1-YFP ou la protéine fluorescente YFP seule (de 50 ng à 4000 ng de plasmides YFP-N1). La mesure de BRET est réalisée 48 heures après transfection sur les cellules vivantes en utilisant les jeux d'anciens filtres (partie a et b) et de nouveaux filtres (partie c et d). Le signal BRET est mesuré en mBu, pour le produit Lingo1-RLuc/Lingo1-YFP (carrés) et Lingo1-RLuc/YFP-N1 (cercles).

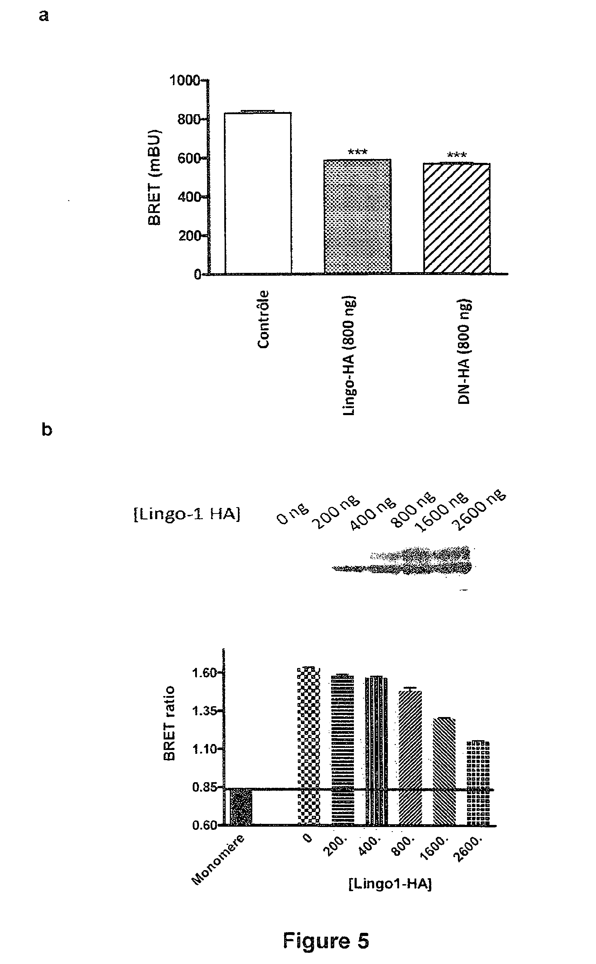

La figure 5 représente la spécificité de l'interaction entre Lingo1-RLuc et Lingo1-YFP. Les cellules HEK-293 sont transfectées avec les protéines de fusions Lingo1-RLuc et Lingo1-YFP (avec un ratio donneur/ accepteur constant) et des concentrations croissantes de la protéine entière de Lingo-1 (acides aminées 1-620, piste « Lingo-HA ») ou son dominant négatif (acides aminés 1-577, piste « DN-HA ») étiqueté HA. La mesure du BRET (mBu) est réalisée 48 heures après la transfection sur les cellules vivantes. Les constructions étiquetées HA diminuent significativement le signal de BRET obtenu entre Lingo1-RLuc et Lingo1-YFP (piste « Control ») (p<0.001 vs control, ANOVA, NewMan Keuls).

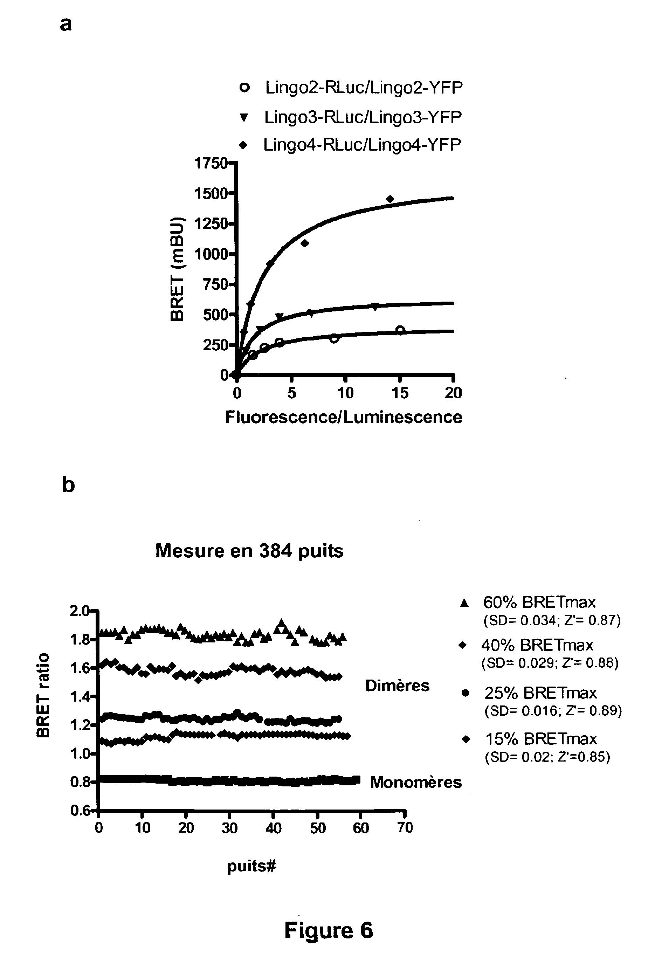

La figure 6 montre la détection de l'oligomérisation des protéines Lingo1, Lingo-2, Lingo-3 et Lingo-4 dans des cellules vivantes en utilisant la technique du BRET en plaques 96 puits (a) et en plaques 384 puits (b). Les cellules HEK-293 sont transfectées avec une concentration fixe de donneur (100ng de plasmide Lingo1-RLuc, Lingo2-RLuc ou Lingo3-RLuc ou Lingo4-RLuc) et des concentrations croissantes d'accepteurs (de 50 ng à 4000 ng des plasmides Lingo1-YFP, Lingo-2-YFP ou Lingo-3-YFP ou Lingo4-YFP). (a) Le signal BRET est mesuré (mBU) pour Lingo-2-RLuc/Lingo-2-YFP (cercles), Lingo-3-RLuc/Lingo-3-YFP (triangles) et Lingo-4-RLuc/Lingo-4-YFP (losanges). (b) Le signal BRET est mesuré (mBU) pour Lingo-1-RLuc/Lingo-1-YFP. La mesure de BRET est réalisée 48 heures après transfection sur les cellules vivantes. Le ratio BRET a été calculé avec un jeu de filtres, en utilisant pour la lumière émise par l'eYFP le filtre 540 ± 40nm et pour la lumière émise par la RLuc le filtre 480 ± 20nm.

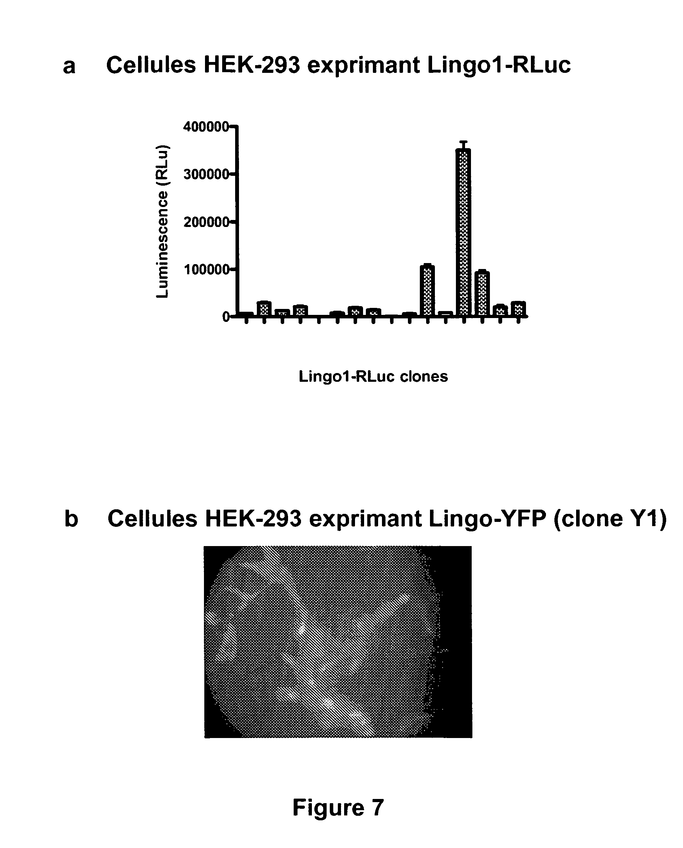

La figure 7 représente des lignées cellulaires HEK-293 stables exprimant hLingo1-RLuc ou hLingo1-YFP.

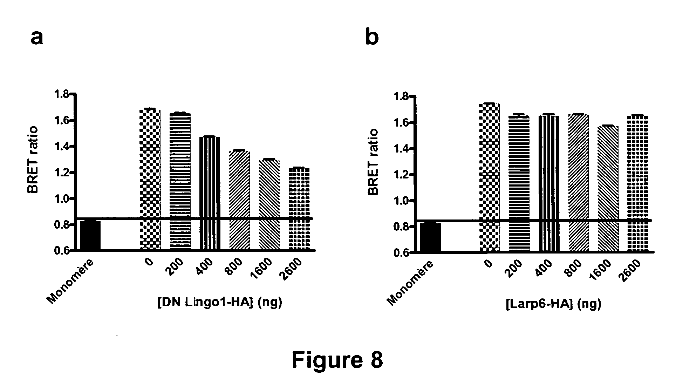

La figure 8 représente la validation et la spécificité du test de criblage utilisant le système de l'invention avec un inhibiteur connu de Lingo1 (DN-Lingo1).

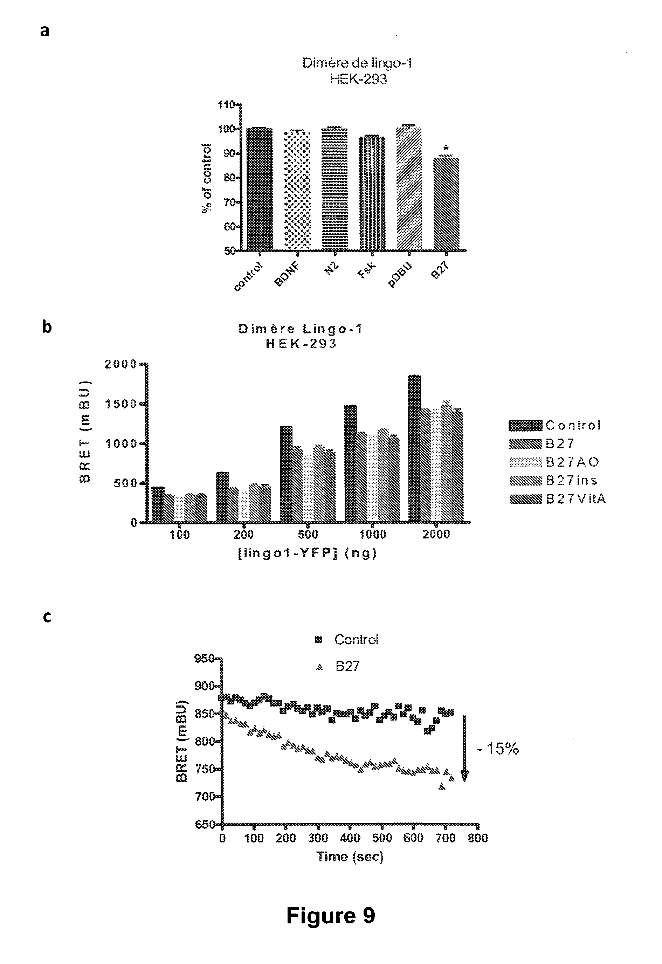

La figure 9 représente (a, b) l'identification et la validation de ligands potentiels de Lingo1 (inhibiteurs de dimérisation) en utilisant le système de l'invention, (c) la cinétique de l'effet du ligand spécifique B27 ainsi identifié.

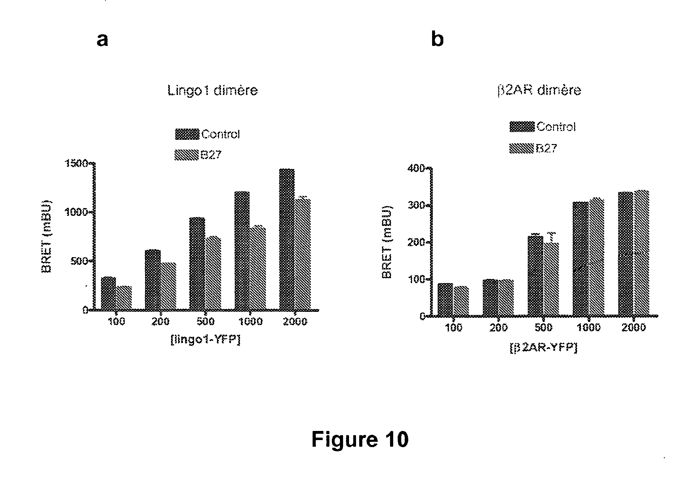

La figure 10 représente l'étude de la spécificité du B27 en test de contre-criblage (étude du signal provenant de la dimérisation du récepteur β2 adrénergique, β2AR).

### EXEMPLES

### Matériels et Méthodes

### 1. Cellules

Les lignées de cellules HEK-293 (fibroblastes d'embryons de rein humains, ATCC CRL-1573) et SH-SY5Y (neuroblastomes humains, ATCC CRL-2266) sont cultivées dans un milieu DMEM (Dulbecco's Modified Eagle Medium) supplémenté avec du sérum de veau foetal (10%, BioWest, France), de la pénicilline (100U/ml, Eurobio), de la streptomycine (100µg/ml, Eurobio, France) et de la L-Glutamine (2mM, Eurobio). Les cultures primaires de neurones sont réalisées à partir de cortex d'embryons de rat prélevés à E18 (18ème jour embryonnaire). Les neurones sont cultivés dans du NeurobasalTM (Gibco®, Invitrogen, composé de glycine, L-alanine, L-Arginine hydrochloride, L-Asparagine-H2O, L-Cysteine, L-Histidine hydrochloride-H2O, L-Isoleucine, L-Leucine, L-Lysine hydrochloride, L-Methionine, L-Phenylalanine, L-Proline, L-Serine, L-Threonine, L-Tryptophane, L-Tyrosine, L-Valine, Choline chloride, D-Calcium pantothenate, acide folique, Niacinamide, Pyridoxine hydrochloride, Riboflavine, Thiamine hydrochloride, Vitamine B12, i-Inositol, Calcium Chloride, Ferric Nitrate, Magnésium Chloride, Potassium Chloride, Sodium Bicarbonate, Sodium Chloride, Sodium Phosphate monobasique, Zinc sulfate, HEPES, Phenol Red, ) supplémenté avec du B27 (composé notamment de d-Biotine, Sérum albumine bovine, exempte d'acide gras, Catalase, L-Carnitine HCl, Corticostérone, Ethanolamine HCl, D-Galactose, Glutathione, Insuline, acide Linoléique, acide linolénique, Progestérone, Putrescine.2HCl, Sodium Sélénite, Superoxide Dismutase, T-3/ Albumine, DL Alpha-Tocophérole, DL Alpha Tocopherol Acétate, Transferrin, Vitamine A Acétate, Gibco®), de la pénicilline (100U/ml) et de la streptomycine (100µg/ml). Les cellules sont cultivées dans des incubateurs à 37°C sous atmosphère humide, à 5% de CO₂.

### 2. Construction des vecteurs d'expression: Cinq vecteurs d'expression ont été utilisés :

Lingo1-HA : la séquence codant pour la protéine de Lingo-1 humaine (620 acides aminés, numéro d'accession NP_116197) suivie d'une étiquette épitopique hémagglutinine (HA) a été amplifié par PCR (pour « Polymerise Chain Reaction » ou « Réaction en chaîne par polymérase »). Le fragment obtenu a été introduit dans le plasmide pcDNA₃ (Invitrogen). Le plasmide Lingo1-HA a la séquence SEQ ID NO: 19.

DN-Lingo1-HA : la séquence codant pour la partie extracellulaire de Lingo ainsi que son domaine transmembranaire (acides aminés 1- 577) suivie d'une étiquette épitopique hémagglutinine (HA) a été amplifiée par PCR. Le fragment obtenu a été introduit dans le plasmide pcDNA₃ (fourni par Invitrogen, de taille 5446 pb, contenant notamment un promoteur Cytomegalovirus, une amorce de séquençage T7, un gène de résistance à l'ampiciline et le gène de sélection à la néomycine.

Lingo1-RLuc : contient la séquence codant pour la protéine Lingo-1 humaine entière (620 acides aminés) étiquetée en 3' avec la séquence d'un marqueur de luminescence (Renilla Luciformis). Le plasmide Lingo1-RLuc a la séquence SEQ ID NO : 17.

Lingo1-eYFP : contient la séquence codant pour la protéine Lingo-1 humaine entière (620 acides aminés) étiquetée en 3' avec la séquence d'un marqueur de fluorescence jaune (eYFP : enhanced yellow fluorescent protein), dont les caractéristiques physiques, en particulier son intensité de fluorescence, ont été améliorées par rapport à la YFP. Le plasmide Lingo1-eYFP a la séquence SEQ ID NO : 18.

pRLuc-N1 : contient la séquence codante de la Renilla Luciferase (RLuc) en aval d'un multi site de clonage. Ce plasmide a été construit à partir du plasmide commercialisé pEYFP-N1 en enlevant la séquence de l'eYFP et en la remplaçant par la séquence de la RLuc. Le plasmide pRLuc-N1 a la séquence SEQ ID NO : 15.

Les protéines de fusion Lingo1-RLuc et Lingo1-eYFP ont été obtenues par des techniques classiques de sous-clonage (digestion de fragments d'ADN ou de plasmides par les enzymes de restriction Sal I, BamH I, Notl , Hind III, EcoR I, Nhe I ; ligation de fragments d'ADN dans un plasmide ; transformation dans des bactéries compétentes DH5α, Invitrogen) à partir d'un vecteur d'expression commercialisé (peYFP-N1, Clonetech) ou disponible au laboratoire (pRLuc-N1) possédant des multi-sites de clonage adjacents aux séquences codantes pour la Renilla Luciferase (Rluc) ou la protéine fluorescente eYFP.

Les protéines de fusion Lingo1-Rluc et Lingo1-eYFP ont été obtenues en fusionnant la RLuc et l'eYFP à l'extrémité C-terminale de la forme humaine de Lingo (620 acides aminés, accession number MP_116197). La séquence de Lingo-1 dépourvue de son codon stop a été amplifiée par PCR. Le fragment obtenu a été introduit dans le plasmide pEYFP-N1 (fourni par Clontech, de séquence SEQ ID NO : 16) de taille environ 4700 pb, contenant notamment un promoteur CMV, une amorce de séquençage EGFP-N de séquence 5'd[CGTCGCCGTCCAGCTCGACCAG]3', un gène de résistance à la Kanamycine, un gène de résistance à la néomycine pour la sélection (avec le G418 ou la généticine) des cellules de mammifères, pour obtenir la construction Lingo-1-eYFP ou dans le plasmide pRLuc-N1 pour obtenir la construction Lingo-1-Rluc.

### 3. Transfection des constructions

Pour les expériences de Western Blot et de co-immunoprécipitation (Figure 1), les cellules HEK-293 sont transfectées en utilisant un lipide de transfection : la lipofectamine^{™} LTX (Invitrogen^{™}).

La veille de la transfection, les cellules sont réparties dans leur milieu de culture sans antibiotiques sur des boîtes collagénées (collagène de type I, Serva).

Les cellules HEK-293 sont cotransfectées avec 5µg de différents plasmides. Deux jours après transfection, les cellules sont lysées dans un tampon contenant 0,5% de triton X100. Le jour de la transfection, l'ADN plasmidique (5 à 10µg) dilué dans du milieu Optimem (3ml, Cat n°31985-047 Gibco®, Invitrogen, composé notamment de L-Glutamine, 2400 mg/L Bicarbonate de Sodium, HEPES, Pyruvate de Sodium, Hypoxanthine, Thymidine, facteurs de croissance, mg/L Phenol Red) est incubé 5 minutes en présence du Plus ReagentTM (10µl, cat n° 11514015, InvitrogenTM). La lipofectamine LTX (20µl, cat n°15338-500, InvitrogenTM) est ensuite ajoutée au mélange et incubée pendant 30 minutes. Après avoir changé le milieu (7ml/ boîte), le complexe formé par l'ADN et la lipofectamine est ajouté goutte à goutte sur les cellules. Pour éviter un effet toxique de la lipofectamine, le milieu de transfection est aspiré au bout de 5 heures et il est remplacé par du milieu frais sans antibiotiques.

Pour les expériences de BRET, les cellules HEK-293 cultivées en plaques 6 puits, préalablement traitées au collagène sont transfectées de manière transitoire par la technique de phosphate de calcium. Brièvement, l'ADN plasmidique est mélangé dans un tampon phosphate (BBS 2X, composé notamment de 50 mM BES (cat n°B4554, Sigma), 280mM NaCl, 1.5mM Na₂HPO₄ Ajusté à pH 6,95) avec du chlorure de calcium (2,5M). Après 15 minutes d'incubation, le précipité obtenu est appliqué sur les cellules pendant 24 heures. Les cellules sont ensuite rincées et incubées à nouveau 24 heures dans un milieu de culture sans antibiotiques.

Pour les expériences de BRET, les cellules SH-SY5Y cultivées en plaques 6 puits, préalablement traitées au collagène, sont transfectées de manière transitoire en utilisant un lipide de transfection : la lipofectamine 2000 (cat n° 11668019, InvitrogenTM). Le jour de la transfection, l'ADN plasmidique dilué dans du milieu Optimem (500µl) est mélangé avec la lipofectamine 2000 (5µl) puis incubé pendant 20 minutes. Après avoir changé le milieu (2ml de milieu frais sans antibiotiques par puits), le complexe est appliqué sur les cellules pendant 24 heures. Les cellules sont ensuite rincées et incubées à nouveau 24 heures dans le milieu de culture sans antibiotiques.

### 4. Préparation des lysats, pontage chimique, Western blot

Dans le cas où l'on réalise un pontage chimique, celui-ci est effectué sur les cellules vivantes avant la lyse. Après avoir rincé les cellules avec du PBS, les cellules sont incubées avec le DSP (Lomant's Reagent, Dithiobis[succinimidyl propionate], cat n° 22585, Thermo Scientific) (160µg/ml, dilué dans un tampon HBSS) pendant 10 minutes à température ambiante. Après trois rinçages au PBS (« phosphate buffered saline » pour tampon phosphate salin), les cellules sont ensuite lysées. La lyse est effectuée sur la glace. Après trois rinçages au PBS froid, les cellules sont incubées dans le tampon de lyse pendant 10 minutes (Tris/HCl pH 7,5 50mM, Triton X100 de 0,5 ou 1% selon les expériences, NaCl 100mM, NaF 50mM, EDTA 5mM, aprotinine 10µg/ml, pyrophosphate de sodium 10mM) auquel on ajoute un cocktail d'inhibiteur de protéases et de phosphatases. Les cellules sont ensuite récupérées à l'aide d'un grattoir puis centrifugées (15 minutes à 22 000g à 4°C). Le surnageant est conservé à -20°C, déposé sur gel ou utilisé directement pour les expériences de co-immunoprécipitation. Pour ce faire, une quantité fixe du lysat cellulaire est incubée avec les billes d'agarose couplé à l'anticorps anti-HA (40µl de billes. EZview, Sigma), à 4°C, sur roue pendant une nuit. Après 5 lavages successifs avec le tampon de lyse, l'immunoprécipitat est récupéré en ajoutant 40µl de Laemmli 4X (200mM Tris pH 6,8, SDS 4%, glycerol 40%, β-mercaptoéthanol 0,5mM, bleu de bromophénol 0,02%). Les lysats cellulaires et les immunoprécipitats sont ensuite déposés sur gel d'acrylamide 8% en condition dénaturantes et réductrices. L'électrophorèse est réalisée dans un tampon de migration Tris/Glycine, puis les protéines sont transférées sur une membrane de PVDF Hybond-P (Amersham Biosciences). La membrane est saturée dans une solution de TBS-T (Tween²⁰ 0,1%)-Lait écrémé 5% pendant 1 heure. Les anticorps primaires sont incubés dans la même solution pendant 2 heures à température ambiante : anti-Lingo (anticorps dirigés contre les acides aminés 40 à 556 de la séquence de Lingo-1 ; numéro d'accession AAH11057, cat n° AF3086, R &D Systems) au 1/1000^{ème}, anti-HA (Roche, rat) au 1/5000^{ème}, anti-Living Colors® (Clontech) au 1/5000^{ème}. Plusieurs lavages dans du TBS-T (Tris buffered saline - Tween²⁰ 0,1%) sont réalisés avant d'incuber la membrane avec les anticorps secondaires correspondants couplés à la peroxydase à une dilution 1/33000^{ème} dans du TBS-T (Tween²⁰ 0,1%)-Lait écrémé 5% pendant 1 heure. De nouveau, après plusieurs lavages, la membrane est incubée avec le substrat (SuperSignal West Dura, Pierce) pendant 5 minutes avant exposition sur film (CL-Xposure, Amersham Biosciences).

### 5. Mesure du BRET, luminescence et fluorescence

Les cellules sont resuspendues dans un tampon physiologique (HBSS pour *Hank's Buffered Salt Solution,* (Cat n°14025100, Gibco, composé notamment de chlorure de Calcium, chlorure de Magnésium, sulfate de Magnésium, chlorure de potassium, phosphate de potassium monobasique, bicarbonate de sodium, chlorure de sodium, phosphate de sodium dibasique anhydre) 48 heures après transfection et distribuées en plaques 96 puits (100 000 à 200 000 cellules par puits) ou en plaques 384 puits (20 000 à 40 000 cellules par puits). Les lectures de fluorescence et de luminescence sont réalisées à température ambiante sur un lecteur de microplaques, le Mithras LB 940 (Berthold). Le rendement de transfection de la construction Lingo-1-eYFP, Lingo-2-eYFP, Lingo-3-eYFP, Lingo-4-eYFP, est évalué par la lecture de la fluorescence de l'eYFP émise à 530-540 nm après excitation à 480 nm. Le rendement de transfection de la construction Lingo-1-RLuc, Lingo-2-RLuc, Lingo-3-RLuc, Lingo-4-RLuc est évalué par la lecture de luminescence (1 seconde) obtenue immédiatement après l'ajout du substrat de la coelenterazine (5µM).

La mesure du signal BRET est réalisée immédiatement après l'ajout de coelenterazine. Celle-ci provoque l'apparition d'un signal de bioluminescence avec un pic d'émission à 480 nm, en accord avec les propriétés spectrales de la Rluc. Le transfert d'énergie qui s'opère dans la protéine de fusion entre la Rluc et l'eYFP provoque l'apparition d'un signal de fluorescence avec un maximum d'émission à 530-540 nm, caractéristique des propriétés spectrales de l'eYFP. On définit le "BRET ratio" comme le rapport de la valeur de lumière émise par l'eYFP (530-540nm) sur la valeur de la lumière émise par la RLuc (480nm) après soustraction du rapport obtenu lorsque la construction RLuc est exprimée seule. Deux jeux de filtres BRET ont été utilisées : le premier couple de filtres (dit anciens filtres) correspond à un filtre de 480 nm, bande passante de 20 nm, transmission de lumière de 60% et à un filtre de 530 nm, bande passante de 25 nm, transmission de lumière de 60%. Le deuxième couple de filtres (dit nouveaux filtres) correspond à un filtre de 480 nm, bande passante de 20 nm, transmission de lumière de 75% et à un filtre de 540 nm, bande passante de 40 nm, transmission de lumière de 75%. Les résultats sont exprimés en milliBRET (mBu) qui correspond à la valeur du « BRET ratio » (ratio BRET) multiplié par 1000.

La mesure du BRET peut être également réalisée sur des cellules adhérentes. Dans ce cas, le substrat est préincubé pendant 15 minutes avant la lecture de luminescence.

### 6. Méthode de criblage de ligands de Lingo-1

Les cellules HEK-293 cultivées en boite (diamètre 10cm) ont été transfectées avec Lingo-1-RLuc (250ng) et Lingo-1-YFP (1500ng) de façon à atteindre environ 50-60% du BRETmax. Les cellules ont été re-suspendues dans un tampon physiologique (HBSS) 48 heures après transfection et distribuées sous 80µl en plaques 96 puits (100 000 à 200 000 cellules par puits).

Préalablement à l'ajout des cellules, les différents ligands (en général utilisés à une concentration de 20µM finale) ont été distribués dans les plaques 96 puits (sous 10µl).

Après un temps d'incubation de 15 minutes, la coelenterazine h (Interchim) a été ajoutée (sous 10µl) de façon à obtenir une concentration finale de 5µM.

La lecture de BRET a été réalisée à température ambiante sur un lecteur de microplaques, le Mithras LB 940 (Berthold) en utilisant les nouveaux filtres BRET. Ces expériences sont aussi réalisées à partir d'une lignée stable exprimant Lingo-1-RLuc et Lingo-1-YFP.

### 7. Test de contre-criblage

La spécificité de l'effet des molécules identifiées par notre méthode a été évaluée sur le signal BRET provenant de la formation de dimères du récepteur β2 adrénergique (test de contre-criblage).

Pour ce faire, les cellules HEK-293 ont été transfectées avec le récepteur β2 adrénergique fusionné en C-terminal avec la Renilla Luciférase (β2AR-RLuc, 50ng) jouant le rôle de donneur et des concentrations croissantes de récepteur β2 adrénergique fusionné en C-terminal avec la protéine fluorescente jaune (β2AR-YFP, de 50 à 1000 ng), jouant le rôle d'accepteur. Les cellules ont été resuspendues dans un tampon physiologique (HBSS) 48 heures après transfection et distribuées en plaques 96 puits sous 80µl (100 000 à 200 000 cellules par puits).

Préalablement à l'ajout des cellules, les produits ont été distribués dans les plaques 96 puits (sous 10µl).

Après un temps d'incubation de 15 minutes, la coelenterazine h (Interchim) a été ajoutée (sous 10µl) de façon à obtenir une concentration finale de 5µM. La lecture de BRET a été réalisé à température ambiante sur un lecteur de microplaques, le Mithras LB 940 (Berthold) en utilisant les nouveaux filtres BRET.

### EXEMPLE 1 : LINGO1 FORME UN DIMÈRE IN VIVO

### 1/ Lingo-1 forme un dimère dans les cellules HEK-293

Les cellules HEK-293 ont tout d'abord été co-transfectées par deux plasmides permettant l'expression de la protéine Lingo-1 étiquetée par HA et Lingo-1 étiquetée par une protéine fluorescente, YFP. Des expériences de co-immunoprécipitation ont montré que lorsqu'on immunoprécipite la protéine Lingo-1 étiquetée HA avec des billes d'agarose couplés à un anticorps anti-HA (figure 1 a), la protéine Lingo-1 étiquetée avec YFP est co-immuniprécipitée (Figure 1 b). Ces résultats montrent pour la première fois que Lingo-1 a formé un dimère *in vivo*. Ces résultats montrent également pour la première fois que la forme entière et membranaire de Lingo-1 a été capable de former des dimères dans des cellules HEK-293.

### 2/ Lingo-1 forme un dimère dans les neurones

Après validation de l'anticorps anti-Lingo-1 (Figure 2), l'existence de dimères dans des neurones de cortex en culture a été testée (Figure 3a). Etant donné que certains détergents utilisés lors de la lyse peuvent induire la formation d'aggrégats protéiques et conduire à la formation artéfactuelle de dimères, l'effet d'un pontage chimique avant la lyse des cellules a été testé (Figure 3b).

Douze jours après leur mise en culture, des neurones de cortex ont émis des prolongements et formé un réseau (cf. figure 3, partie a). Les neurones ont été traités (+ DSP) ou non (-DSP) avec un agent pontant, le DSP qui agit au niveau des fonctions aminés, avant d'être lysés dans un tampon contenant 1% de triton X-100. Lingo-1 a été bien exprimé dans les neurones de cortex comme l'atteste la présence d'une bande immunoréactive à 90 kDa lorsque le western blot est incubé en présence de l'anticorps anti-Lingo-1 (cf. figure 3, partie b). Lorsque les neurones ont été traités avec le DSP, Lingo-1 est bien apparu sous forme de dimères à 180kDa (bande immunoréactive supplémentaire apparaît à 180 kDa), ceci au détriment du monomère à 90 kDa qui a disparu (cf. figure 3, partie b).

### EXEMPLE 2: DÉTECTION DE L'OLIGOMÉRISATION DE LA PROTÉINE LINGO-1 DANS DES CELLULES VIVANTES EN UTILISANT LA TECHNIQUE DU BRET

Les cellules HEK-293 ou SHSY-5Y ont été transfectées avec une concentration fixe de donneur (100ng de plasmide Lingo1-RLuc) et des concentrations croissantes d'accepteurs, Lingo1-YFP ou la protéine fluorescente YFP seule (de 50 ng à 4000 ng de plasmides). La mesure de BRET a été réalisée 48 heures après transfection sur les cellules vivantes en utilisant des jeux d'anciens filtres et de nouveaux filtres.

Le signal BRET observé avec les anciens filtres correspondant à l'oligomérisation de Lingo-1 est bien spécifique puisque lorsqu'on a coexprimé Lingo1-RLuc et Lingo1-YFP le signal a augmenté selon une courbe hyperbolique et atteint une asymptôte dans les deux types cellulaires (cf. figure 4a et 4b). De plus, lorsque Lingo1-Rluc a été co-exprimé avec la protéine YFP seule, le signal BRET a été beaucoup plus faible (<20 mBU) et a augmenté de façon linéaire (cf. figure 4a, insert) ; ce qui atteste de l'apparition d'interactions non spécifiques et aléatoires.

Ces résultats montrent que les protéines de fusion Lingo1-RLuc et Lingo1-YFP ont été capables d'interagir pour former des dimères (voire des oligomères) dans des cellules vivantes en culture. En effet, avec les anciens filtres un signal BRET très intense (de 100 à 800mBu) a été observé après co-expression de Lingo1-Rluc et Lingo1-YFP dans des cellules HEK-293 (cf. figure 4a). Lingo-1 étant exclusivement exprimé dans le système nerveux, il a été également recherché si Lingo-1 était capable de se dimériser dans un environnement plus physiologique, c'est-à-dire dans des cultures de neuroblastomes humains (cf. figure 4b). Là encore, avec les anciens filtres un signal BRET très intense (de 100 à 600mBu) a été observé. Ce signal est spécifique puisque lorsque des courbes de saturation ont été réalisées, le signal BRET a atteint un plateau ainsi bien dans les cellules HEK-293 (cf. figure 4a) que dans les cellules SH-SY5Y (cf. figure 4b). En conclusion, la dimérisation de Lingo-1 a induit un signal de BRET fort et spécifique.

En outre, les résultats obtenus avec une mesure de BRET réalisée dans les mêmes conditions avec le jeu de nouveaux filtres dans les cellules HEK-293 (cf figure 4c) et dans les cellules de neuroblastomes humain SHSY-5Y (cf figure 4d) coexprimant Lingo1-Rluc et Lingo1-YFP, ont permis d'optimiser le test en obtenant un signal BRET deux fois plus important que précédemment.

### EXEMPLE 3: L'INTERACTION ENTRE LINGO1-RLuc et LINGO1-YFP est SPÉCIFIQUE ET MODULABLE

Les cellules HEK-293 ont été transfectées avec les protéines de fusions Lingo1-RLuc et Lingo1-YFP (le ratio donneur/ accepteur reste constant) et des concentrations croissantes de la protéine entière de Lingo-1 (Lingo1-HA, acides aminées 1-620) ou son dominant négatif (DN-HA, acides aminés 1-577) étiqueté(e) HA. La mesure du BRET a été réalisée 48 heures après la transfection sur les cellules vivantes. Les constructions étiquetées HA ont diminué significativement le signal de BRET obtenu entre Lingo1-RLuc et Lingo1-YFP (p<0.001 vs control, ANOVA, NewMan Keuls). Les résultats sont montrés dans la figure 5.

Le signal BRET obtenu entre les protéines Lingo1-RLuc et Lingo1-YFP est donc spécifique et modulable. En effet, lorsqu'on surexprime à 800 ng la protéine entière Lingo-1 (620 acides aminés) ou le dominant négatif de Lingo-1 (577 acides aminés, sans la portion cytoplasmique de la protéine) étiqueté(e) HA, le signal BRET obtenu entre Lingo1-RLuc et Lingo1-YFP a diminué significativement (Figure 5a). En outre, lorsque l'on a ajouté des concentrations croissantes de protéine Lingo-1 étiquetée HA (Lingo1-HA à 0, 200, 400, 800, 1600 et 2600 ng), le signal BRET a diminué (Figure 5b); ce qui permet de réaliser des courbes d'inhibition.

Ainsi, les résultats montrent que les protéines Lingo-1 étiquetées HA empêchent, par compétition, l'interaction de Lingo1-RLuc avec Lingo1-YFP.

### EXEMPLE 4: DÉTECTION DE L'OLIGOMÉRISATION DES PROTEINES LINGO-1, LINGO-2, LINGO-3, LINGO-4, DANS DES CELLULES VIVANTES EN UTILISANT LA TECHNIQUE DU BRET

Les cellules HEK-293 ont été transfectées avec une concentration fixe de donneur (100ng de plasmide Lingo1-RLuc, Lingo2-RLuc ou Lingo3-RLuc ou Lingo4-RLuc) et des concentrations croissantes d'accepteurs, Lingo2-YFP ou Lingo3-YFP ou Lingo4-YFP (de 50 ng à 4000 ng de plasmides). La mesure de BRET a été réalisée 48 heures après la transfection sur les cellules vivantes en plaques 96 et 384 puits.

Le signal BRET observé en plaques 96 puits, correspondant à l'oligomérisation de Lingo-2 et lingo-3 et Lingo-4, est bien spécifique puisqu'il a augmenté selon une courbe hyperbolique et atteint une asymptôte pour chaque couple de protéines (cf. figure 6a).

En outre, les résultats montrent qu'un signal BRET obtenu avec Lingo1 a pu être détecté sans aucun problème lorsque les mesures ont été réalisées en plaques 384 puits (haut/moyen débit). D'ailleurs, le calcul du Z' (valeur p>0,85) montre toute la robustesse du test (Figure 6b).

La Demanderesse a donc montré qu'il est possible, en mesurant le transfert de luminescence (BRET) entre chaque monomère modifié, de repérer des variations de conformation du dimère *in vivo,* dans les cellules vivantes en temps réels. En outre, elle a montré que la mesure de BRET peut s'effectuer sur des cellules adhérentes ou en suspension en plaques 96 puits et qu'elle est adaptable au haut/moyen débit.

### EXEMPLE 5 : CONSTRUCTION DE LIGNEES STABLES

Afin d'assurer une reproductibilité des mesures de BRET de haut/moyen débit, des lignées stables de cellules HEK-23 exprimant Lingo1-RLuc ou Lingo1-YFP ont été développées.

Les cellules HEK-293 ont été transfectées comme précédemment décrit avec .des plasmides Lingo1-RLuc ou Lingo-1-YFP portant notamment la résistance à la néomycine, comme précédemment décrit. La sélection des cellules ayant intégré l'ADN plasmidique a été réalisée 48 heures après la transfection par l'ajout de l'antibiotique de sélection (G-418 ou généticine à 2mg/ml) dans le milieu de culture. Après deux à trois semaines de sélection, plusieurs dizaines de clones résistants à l'antibiotique ont été récupérés. Chaque clone a ensuite été amplifié de façon à obtenir suffisamment de cellules pour réaliser le test d'expression de Lingo-1. Ce test consiste en la lecture de fluorescence dans le cas de clones de cellules exprimant Lingo1-YFP ou en la lecture de la luminescence après ajout de la coelenterazine dans le cas de clones de cellules exprimant Lingo1-RLuc, comme précédemment décrit.

Les résultats montrent que parmi les clones testés (environ une centaine de clones testés) exprimant Lingo1-RLuc, plusieurs clones (par exemple C5, D1 et D2) expriment hLingo1-RLuc à un niveau satisfaisant (Figure 7a) et ont été congelés. En outre le clone C5, présentant un bon signal de luminescence avec une bonne viabilité cellulaire, a été déposé le 31 mars 2011, à la CNCM (Institut Pasteur, Paris) sous le numéro I-4463.

Les résultats montrent un clone Y1 exprimant hLingo1-YFP à un niveau satisfaisant, à savoir présentant un bon signal de fluorescence avec une bonne viabilité cellulaire (Figure 7b). En outre le clone Y1 a été déposé le 31 mars 2011, à la CNCM (Institut Pasteur, Paris) sous le numéro I-4462.

De même, des lignées stables de cellules HEK-23 co-exprimant Lingo1-RLuc et Lingo1-YFP sont développées. Les clones co-exprimant les deux protéines à un niveau satisfaisant sont congelés et déposés près de la CNCM.

### EXEMPLE 6: PROCEDE D'IDENTIFICATION DE LIGANDS DE LINGO1 UTILISANT LE SYSTEME DE L'INVENTION

### Validation du procédé d'identification de l'invention

Les cellules HEK-293 ont été transfectées avec une concentration fixe de Lingo1-RLuc (250ng) et Lingo1-YFP (1500ng) de façon à atteindre environ 50-60% du BRETmax. La mesure de BRET utilisant le jeu de nouveaux filtres a été réalisée 48 heures après la transfection sur les cellules vivantes en plaques 96 puits, en présence de concentrations croissantes d'un inhibiteur connu de Lingo-1 (DN Lingo1-HA à 0, 200, 400, 800, 1600 et 2600 ng) ou d'une protéine neuronale n'interagissant pas avec Lingo1 (Larp6-HA à 0, 200, 400, 800, 1600 et 2600 ng) étiqueté(e) HA.

Les résultats montrent que l'inhibiteur connu de Lingo1 étiqueté HA (DN-Lingo1-HA) a diminué significativement le signal BRET obtenu entre les protéines Lingo1-RLuc et Lingo1-YFP (Figure 8a). En outre, les résultats montrent que cet effet inhibiteur était spécifique dans la mesure où la protéine neuronale n'interagissant pas avec Lingo1, nommée Larp6 étiquetée HA, n'a pas modifié le signal BRET (Figure 8b).

### Identification et validation d'un nouvel inhibiteur de dimérisation de Lingo1 : B27

Les cellules HEK-293 ont été transfectées avec une concentration fixe de Lingo1-RLuc (250ng) et Lingo1-YFP (1500ng) de façon à atteindre environ 50-60% du BRETmax. La mesure de BRET utilisant le jeu de nouveaux filtres a été réalisée 48 heures après la transfection sur les cellules vivantes en plaques 96 puits, en présence de concentration fixe de ligands (BDNF, N2, Fsk, pDBU à 20µM final) ou d'une solution de B27 (B27 50x, Gibco, référence 17-504-044, 10µl de solution pure) susceptibles de modifier le signal BRET obtenu entre les protéines Lingo1-RLuc et Lingo1-YFP.

Les résultats montrent que le composé B27 a diminué significativement la dimérisation de Lingo1 dans les cellules HEK-293 (Figure 9a). L'effet du composé B27 a été reproduit à différents niveaux de BRET et avec différents lots de B27 : B27 sans antioxydant (B27AO), B27 sans insuline (B27ins) ou sans vitamine A (B27VitA) (Figure 9b).

Une étude cinétique de l'effet du B27 a également été réalisée et a laissé apparaitre que la baisse du B27 était progressive et atteignait un plateau (-15%) au bout de 10 minutes (Figure 9c).

### Test de contre criblage : spécificité du B27

La spécificité du B27 a été évaluée dans un test de contre criblage (étude du signal provenant de la dimérisation du récepteur β2-adrénergique, β2AR), cette dimérisation ayant été décrite précédemment par Angers et al. [24].

Les cellules HEK-293 ont été transfectées avec (i) un dimère du récepteur β2AR : le récepteur β2 adrénergique fusionné en C-terminal avec la Renilla Luciférase (β2AR-RLuc, 50ng) jouant le rôle de donneur et des concentrations croissantes de récepteur β2 adrénergique fusionné en C-terminal avec la protéine fluorescente jaune (β2AR-YFP, de 100 à 2000 ng) jouant le rôle d'accepteur, ou (ii) un dimère de Lingo1 : Lingo1-.RLuc jouant le rôle de donneur et des concentrations croissantes de Lingo1-YFP (de 100 à 2000ng) jouant le rôle d'accepteur. La mesure de BRET utilisant le jeu de nouveaux filtres a été réalisée 48 heures après la transfection sur les cellules vivantes en plaques 96 puits, en absence ou présence de concentration fixe de B27 (B27 50x, Gibco, référence 17-504-044, 10µl de solution pure).

Les résultats montrent que le signal BRET provenant de la dimérisation du récepteur β2-adrénergique n'a pas été modifié dans le test de contre-criblage (Figure 10b) contrairement au signal BRET provenant de la dimérisation de Lingo1 (Figure 10a).

La Demanderesse a ainsi montré que le procédé de criblage utilisant le système de l'invention permet l'identification de ligands spécifiques de Lingo1.

### RÉFÉRENCES

1. Carim-Todd et al., "LRRN6A/LERN1 (leucine-rich repeat neuronal protein 1), a novel gene with enriched expression in limbic system and neocortex". 2003, Eur. J; Neurosci. 18 (12):3167-82.
2. Mi et al., "LINGO-1 is a component of the Nogo-66 receptor/p75 signaling complex". 2004, Nat Neurosci. 7(3):221-8.
3. Ji et al., "LINGO-1 antagonist promotes functional recovery and axonal sprouting after spinal cord injury", 2006, Mol Cell Neurosci. 33(3):311-20.
4. Mi et al., "Lingo-1 negatively regulates myelination by oligodendrocytes", 2005, Nature Neuroscience 8: 745- 751.
5. Lee et al. "NGF regulates the expression of axonal LINGO-1 to inhibit oligodendrocyte differentiation and myelination". 2007, J Neurosci. 27(1):220-5.
6. Zhao et al. "An in vitro study on the involvement of LINGO-1 and Rho GTPases in Nogo-A regulated differentiation of oligodendrocyte precursor cells", 2007, Mol Cell Neurosci. 36(2):260-9.
7. Mi et al., "Lingo-1 antagonist promotes spinal cord remyelination and axonal integrity in MOG-induced experimental autoimmune encephalomyelitis", 2007, Nature medicine 13: 1228-1233.
8. Fu et al., "Blocking LINGO-1 function promotes retinal ganglion cell survival following ocular hypertension and optic nerve transection". Invest Ophthalmol Vis Sci. 2008, 49(3):975-85.
9. Fu et al., "Combined effect of brain-derived neurotrophic factor and Lingo-1 fusion protein on long-term survival of retinal ganglion cells in chronic glaucoma", 2009, Neuroscience 162: 375-382.
10. Zhao et al. "Inactivation of glycogen synthase kinase-3beta and up-regulation of LINGO-1 are involved in LINGO-1 antagonist regulated survival of cerebellar granular neurons", 2008, Cell Mol Neurobiol. 28(5):727-35.
11. Inoue et al., "Inhibition of the leucine-rich repeat protein Lingo-1 enhances survival, structure, and function of dopaminergic neurons in Parkinson's disease models", 2007, PNAS 104: 14430-14435.
12. Mosyak et al., "The structure of Lingo-1 ectodomain, a module implicated in CNS repair inhibition", 2006, J. Biol. Chem. 281: 36378-36390.
13. Xu et al, « A bioluminescence resonance energy transfer (BRET) system: application to interacting circadian clock proteins » (1999) Proc. Natl. Acad. Sci. USA. 96, 151-156.
14.Angers 2000, « Detection of beta 2-adrenergic receptor dimerization in living cells using bioluminescence resonance energy transfer (BRET)" (2000) Proc.Natl. Acad. Sci. USA 97 : 3684-3689.
15. Mi et al., "Lingo-1 and its role in CNS repair", 2008, The Int. J. Bioch. Cell. Biol. 40: 1971-1978.
16. Rudick et al., "Lingo-1 antagonists as therapy for multiple sclerosis: in vitro and in vivo evidence", 2008, Expert Opin. Biol. Ther.8:1561-1570.
17.Kamal et al., « Improved donor/acceptor BRET couples for monitoring beta-arrestin recruitment to G protein-coupled receptors ». Biotechnol J. 2009 Sep;4(9):1337-44.
18. Kocan M et al., « Démonstration of improvements to the bioluminescence resonance energy transfer (BRET) technology for the monitoring of G protein-coupled receptors in live cells", J Biomol Screen. 2008 Oct;13(9):888-98.
19. Michelini E et al., « Luminescent probes and visualization of bioluminescence », Methods Mol Biol. 2009;574:1-13.
20.Kroeger et al., « Constitutive and agonist-dependent homo-oligomerization of the thyrotropin-releasing hormone receptor » (2001) J Biol Chem, 276, 12736-12743.
21.Maurel D et al., "Cell-surface protein-protein interaction analysis with time-resolved FRET and snap-tag technologies: application to GPCR oligomerization" (2008), Nat Methods (6):561-7.
22.Hermand P et al, "Functional adhesiveness of the CX3CL1 chemokine requires its aggregation. Role of the transmembrane domain" (2008) J Biol Chem 283(44):30225-34;
23.Whitfield J et al. "High-throughput methods to detect dimerization of Bcl-2 family proteins" (2003), Anal Biochem, 322(2):170-8.

## Revendications

1. Système comprenant :
a) un premier produit de couplage comprenant un monomère d'une protéine choisie parmi Lingo-1, Lingo-2, Lingo-3 et Lingo-4, et une sonde susceptible d'émettre un signal détectable lors de changements conformationnels dudit monomère,
b) un deuxième produit de couplage comprenant un monomère d'une protéine choisie parmi Lingo-1, Lingo-2, Lingo-3 et Lingo-4, et une sonde susceptible d'émettre un signal détectable lors de changements conformationnels dudit monomère,
dans lequel lesdits premier produit de couplage et deuxième produit de couplage forment un dimère par interaction entre lesdits monomères, et dans lequel les changements d'interactions entre lesdits monomères provoquent un signal.

2. Système selon la revendication 1, dans lequel ladite sonde est une protéine fluorescente choisie parmi la GFP (« Green Fluorescent Protein » ou « Protéine fluorescente verte »), la YFP (« Yellow Fluorescent Protein » ou « Protéine fluorescente jaune »), Enhanced Yellow Fluorescent Protein (eYFP), eGFP, GFP2 , GFP10, RGFP (Renilla Green Fuorescent Protein), YPet.

3. Système selon la revendication 1, dans lequel ladite sonde est une une luciférase choisie parmi la Renilla luciferase, la RLuc8, la firefly luciferase, la Gaussia luciferase ou l'Aequorin.

4. Système selon la revendication 1, ledit système étant un système de transfert d'énergie par résonance bioluminescente.

5. Utilisation d'un système tel que défini dans l'une quelconque des revendications 1 à 4 pour identifier des ligands d'une protéine choisie parmi Lingo-1, Lingo-2, Lingo-3 et Lingo-4.

6. Procédé d'identification d'un ligand d'une protéine choisie parmi Lingo-1, Lingo-2, Lingo-3 et Lingo-4, comprenant les étapes suivantes :
a) incuber un système tel que défini selon l'une quelconque des revendications 1 à 4 et une molécule candidate, ledit système comprenant :
(i) un premier produit de couplage comprenant un monomère d'une protéine choisie parmi Lingo-1, Lingo-2, Lingo-3 et Lingo-4, et une sonde susceptible d'émettre un signal détectable lors de changements conformationnels dudit monomère,
(ii) un deuxième produit de couplage comprenant un monomère d'une protéine choisie parmi Lingo-1, Lingo-2, Lingo-3 et Lingo-4, et une sonde susceptible d'émettre un signal détectable lors de changements conformationnels dudit monomère,
b) détecter une modification du signal émis par au moins une desdites sondes,
ladite modification du signal étant révélateur de la liaison de ladite molécule candidate à au moins un desdits produits de couplage.

7. Procédé selon la revendication 6, comprenant un système de criblage à haut-débit.

## Patentansprüche

1. System, das Folgendes umfasst:
a) ein erstes Kopplungsprodukt, das ein Monomer eines Proteins, das aus Lingo-1, Lingo-2, Lingo-3 und Lingo-4 ausgewählt ist, und eine Sonde umfasst, die dazu imstande ist, ein nachweisbares Signal während Konformationsänderungen des besagten Monomers abzugeben,
b) ein zweites Kopplungsprodukt, das ein Monomer eines Proteins, das aus Lingo-1, Lingo-2, Lingo-3 und Lingo-4 ausgewählt ist, und eine Sonde umfasst, die dazu imstande ist, ein nachweisbares Signal während Konformationsänderungen des besagten Monomers abzugeben,
wobei das besagte erste Kopplungsprodukt und das besagte zweite Kopplungsprodukt durch Interaktion zwischen den besagten Monomeren ein Dimer bilden und wobei die Änderungen von Interaktionen zwischen den besagten Monomeren ein Signal hervorrufen.

2. System nach Anspruch 1, wobei die besagte Sonde ein fluoreszierendes Protein ist, das aus GFP ("grün fluoreszierendes Protein"), YFP ("gelb fluoreszierendes Protein"), verstärkt gelb fluoreszierendem Protein (eYFP), eGFP, GFP2, GFP10, RGFP (Renilla-grün fluoreszierendes Protein), YPet ausgewählt ist.

3. System nach Anspruch 1, wobei die besagte Sonde eine Luciferase ist, die aus Renilla-Luciferase, RLuc8, Firefly-Luciferase, Gaussia-Luciferase oder Äquorin ausgewählt ist.

4. System nach Anspruch 1, wobei das besagte System ein Biolumineszenzresonanz-Energietransfer-System ist.

5. Verwendung eines wie in einem der Ansprüche 1 bis 4 definierten Systems zum Identifizieren von Liganden eines Proteins, das aus Lingo-1, Lingo-2, Lingo-3 und Lingo-4 ausgewählt ist.

6. Verfahren zur Identifizierung eines Liganden eines Proteins, das aus Lingo-1, Lingo-2, Lingo-3 und Lingo-4 ausgewählt ist, wobei das Verfahren die folgenden Schritte umfasst:
a) Inkubieren eines wie in einem der Ansprüche 1 bis 4 definierten Systems und eines Kandidatenmoleküls, wobei das besagte System Folgendes umfasst:
i) ein erstes Kopplungsprodukt, das ein Monomer eines Proteins, das aus Lingo-1, Lingo-2, Lingo-3 und Lingo-4 ausgewählt ist, und eine Sonde umfasst, die dazu imstande ist, ein nachweisbares Signal während Konformationsänderungen des besagten Monomers abzugeben,
ii) ein zweites Kopplungsprodukt, das ein Monomer eines Proteins, das aus Lingo-1, Lingo-2, Lingo-3 und Lingo-4 ausgewählt ist, und eine Sonde umfasst, die dazu imstande ist, ein nachweisbares Signal während Konformationsänderungen des besagten Monomers abzugeben, b) Nachweisen einer Modifikation des von mindestens einer der besagten Sonden abgegebenen Signals,
wobei die besagte Modifikation des Signals ein Indikator für die Bindung des besagten Kandidatenmoleküls an mindestens eines der besagten Kopplungsprodukte ist

7. Verfahren nach Anspruch 6, das ein Screening-System mit hohem Durchsatz umfasst.

## Claims

1. A system comprising:
a) a first coupling product comprising a monomer of a protein chosen from Lingo-1, Lingo-2, Lingo-3 and Lingo-4, and a probe capable of emitting a detectable signal when said monomer undergoes conformational changes,
b) a second coupling product comprising a monomer of a protein chosen from Lingo-1, Lingo-2, Lingo-3 and Lingo-4, and a probe capable of emitting a detectable signal when said monomer undergoes conformational changes,
wherein said first coupling product and said second coupling product form a dimer by interaction between said monomers, and wherein the changes in interactions between said monomers cause a signal.

2. The system as claimed in claim 1, wherein said probe is a fluorescent protein chosen from GFP ("Green Fluorescent Protein"), YFP ("Yellow Fluorescent Protein"), Enhanced Yellow Fluorescent Protein (eYFP), eGFP, GFP2 , GFP10, RGFP (*Renilla* Green Fluorescent Protein), YPet.

3. The system as claimed in claim 1, wherein said probe is a luciferase chosen from *Renilla* luciferase, RLuc8, firefly luciferase, Gaussia luciferase or Aequorin.

4. The system as claimed in claim 1, said system being a bioluminescence resonance energy transfer system.

5. Use of a system as defined in any one of claims 1 to 4, for identifying ligands of a protein chosen from Lingo-1, Lingo-2, Lingo-3 and Lingo-4.

6. A method for identifying a ligand of a protein chosen from Lingo-1, Lingo-2, Lingo-3 and Lingo-4, comprising the following steps:
a) incubating a system as defined in any one of claims 1 to 4 and a candidate molecule, said system comprising:
(i) a first coupling product comprising a monomer of a protein chosen from Lingo-1, Lingo-2, Lingo-3 and Lingo-4, and a probe capable of emitting a detectable signal when said monomer undergoes conformational changes,
(ii) a second coupling product comprising a monomer of a protein chosen from Lingo-1, Lingo-2, Lingo-3 and Lingo-4, and a probe capable of emitting a detectable signal when said monomer undergoes conformational changes,
b) detecting a modification of the signal emitted by at least one of said probes,
said modification of the signal revealing the binding of said candidate molecule to at least one of said coupling products.

7. The method as claimed in claim 6, comprising a high-throughput screening system.
